(19) Europäisches Patentamt
European Patent Office
Office européen des brevets

(11) EP 2 062 945 B1

(12) **FASCICULE DE BREVET EUROPEEN**

(45) Date de publication et mention de la délivrance du brevet:
**21.12.2011 Bulletin 2011/51**

(51) Int Cl.:
***C09B 23/14** (2006.01)* ***A61K 8/49** (2006.01)*
***A61Q 5/10** (2006.01)*

(21) Numéro de dépôt: **08164635.8**

(22) Date de dépôt: **18.09.2008**

(54) **Colorant hemicyanine styryle thiol/disulfure, composition tinctoriale comprenant ce colorant, procédé d'éclaircissement des matières kératiniques à partir de ce colorant**

Hemicyaninstyryl-thiol/disulphid-Farbstoff, Farbstoffzusammensetzung enthaltend diesen Farbstoff, Verfahren zur Aufhellung keratinischen Materialien unter Verwendung dieses Farbstoffs

Hemicyanin styryl thiol/disulfide dye, dye composition comprising this dye, process for lightening keratin materials using this dye

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité: **21.09.2007 FR 0757778**

(43) Date de publication de la demande:
**27.05.2009 Bulletin 2009/22**

(73) Titulaire: **L'Oréal**
**75008 Paris (FR)**

(72) Inventeurs:
- **Greaves, Andrew**
**77700 Bailly Romainvilliers (FR)**
- **Daubresse, Nicolas**
**78170 La Celles St Cloud (FR)**

(74) Mandataire: **Fevrier, Murielle Françoise E.**
**L'Oréal**
**D.I.P.I.**
**25-29 Quai Aulagnier**
**92600 Asnières-sur-Seine Cedex (FR)**

(56) Documents cités:
**WO-A-2007/039527 BE-A- 669 934**
**GB-A- 2 143 541**

EP 2 062 945 B1

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

## Description

L'invention concerne la coloration de matières kératiniques à l'aide de colorants fluorescent hémicyanines styryles thiols et disulfures.

Il est connu de teindre les fibres kératiniques, notamment humaines, par une coloration directe. Le procédé classiquement utilisé en coloration directe consiste à appliquer sur les fibres kératiniques des colorants directs qui sont des molécules colorées et colorantes ayant une affinité pour les fibres, à les laisser diffuser, puis à rincer les fibres.

Les colorants directs qui sont classiquement utilisés sont par exemple des colorants du type nitrés benzéniques, des colorants anthraquinoniques, des nitropyridines, des colorants du type azoïque, xanthénique, acridinique, azinique ou triarylméthane.

Il est également connu d'utiliser des colorants directs hémicyanines styryles pour colorer des fibres kératiniques de façon puissante. Ces colorants à groupement hétéroaryle benzothiazolium, benzoimidazolium sont par exemple ceux décrits dans les demandes de brevet EP 1166753 et EP 1166757.

La coloration des fibres kératiniques à partir de ces colorants directs classiques ne permet pas d'éclaircir de façon notable les fibres kératiniques.

L'éclaircissement de la couleur de fibres kératiniques, foncées vers des nuances plus claires, en modifiant éventuellement la nuance de celles-ci, constitue une demande importante.

Classiquement, pour obtenir une coloration plus claire, on met en oeuvre un procédé de décoloration chimique. Ce procédé consiste à traiter les fibres kératiniques, telles que les cheveux, par un système oxydant fort, généralement constitué par du peroxyde d'hydrogène associé ou non à des persels, le plus souvent en milieu alcalin.

Ce système de décoloration présente l'inconvénient de dégrader les fibres kératiniques, et d'altérer leurs propriétés cosmétiques. Les fibres ont en effet tendance à devenir rêches, plus difficilement démêlables et plus fragiles. Enfin, l'éclaircissement ou la décoloration de fibres kératiniques par des agents oxydants est incompatible avec les traitements de modification de la forme desdites fibres particulièrement dans les traitements de défrisage.

Une autre technique d'éclaircissement consiste à appliquer sur les cheveux foncés des colorants directs fluorescents. Cette technique décrite notamment dans les documents WO 03/028685 et WO 2004/091473 permet de respecter la qualité de la fibre kératinique lors du traitement. Cependant ces colorants directs fluorescents ne possèdent pas une tenacité satisfaisante vis-à-vis des agents extérieurs.

Pour augmenter la ténacité des colorations directes, il est connu d'utiliser des colorants disulfures notamment colorants à chromophore aza-imidazoliums dans les demandes de brevet WO 2005/097051 ou EP 1647580, et des colorants à chromophores pyridinium/indolizinium-styryles dans les demandes de brevet WO 2006/134043 et WO 2006/136617. La demande de brevet WO 2007/039527 décrit des colorants styrylthiol pour colorer les cheveux portant un motif indole substitué en position 1,2- dudit groupe indole. Aucun de ces documents ne mentionne l'utilisation des ces colorants pour éclaircir les fibres kératiniques sans employer des agents oxydants.

Le but de la présente invention est de fournir de nouveaux systèmes de coloration de matières kératiniques en particulier les fibres kératiniques humaines, notamment les cheveux foncés, qui ne présentent pas les inconvénients des procédés de décoloration existants.

Particulièrement, un but l'invention est de fournir des systèmes de coloration directe permettant d'obtenir des effets éclaircissants notamment sur des fibres kératiniques naturellement ou artificiellement foncées, tenaces face à des shampooings successifs, qui ne dégradent pas les fibres kératiniques et qui n'altèrent pas leurs propriétés cosmétiques.

Un autre but de l'invention est de colorer de façon chromatique et rémanente vis-à-vis des agressions extérieures les matières kératiniques.

Un autre but de l'invention et de pouvoir étendre la gamme tinctoriale accessible sur les fibres kératiniques notamment sur cheveux clairs et sur les cheveux foncés et de pouvoir décaper les colorations obtenues par des traitements tout en respectant l'intégrité de la qualité des dites fibres

Ce but est atteint avec la présente invention qui a pour objet un procédé de coloration de matières kératiniques, en particulier les fibres kératiniques, notamment humaines telles que les cheveux, plus particulièrement les cheveux foncés, consistant à appliquer sur les matières kératiniques, une composition tinctoriale, comprenant dans un milieu cosmétique approprié, au moins un colorant fluorescent hémicyanine styryle disulfure ou thiol, choisi parmi les colorants de formules **(I)** et **(II) :**

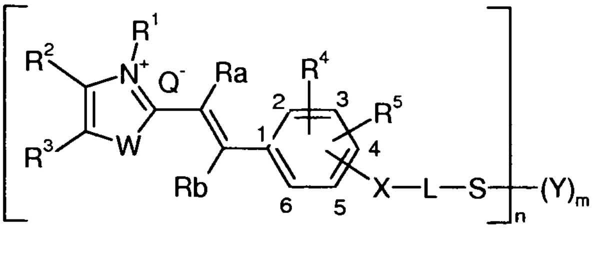

(I)

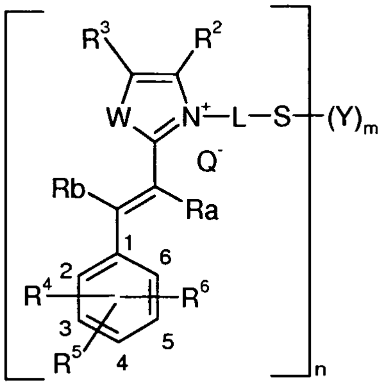

(II)

leurs sels d'acide organique ou minéral, isomères optiques, isomères géométriques, et les solvates tels que hydrates ; formules **(I)** ou **(II)** dans lesquelles :

➢ **n** représente 1 ou 2 ;
➢ **m** représente 0 ou 1 ;
➢ **Ra** et **Rb,** identiques ou différents, représentent un atome d'hydrogène ou un groupement alkyle en $C_1$-$C_4$ ; particulièrement Rb représente un atome d'hydrogène et plus particulièrement Ra et Rb représentent un atome d'hydrogène ;
➢ $\mathbf{R^1}$ représente un atome d'hydrogène ou un groupement ($C_1$-$C_6$)alkyle, éventuellement substitué par un groupement choisi parmi halogène, alcoxy en $C_1$-$C_4$, (di)(alkyl)amino en $C_1$-$C_4$ , phényle, tolyle et méthoxyphényle ;
➢ ou alors $\mathbf{R^1}$ et **Ra** forment ensemble une chaîne ($C_3$-$C_6$)alkylène telle que éthylène ou propylène ou une chaîne ($C_3$-$C_7$)alkénylène telle que -CH=CH- ;
➢ $\mathbf{R^2}$ et $\mathbf{R^3}$**,** identiques ou différents, représentent un atome d'hydrogène, d'halogène, un groupement ($C_1$-$C_6$)alkyle éventuellement substitué, ($C_1$-$C_6$)alkoxy, ($C_1$-$C_6$)alkylthio; ou alors $\mathbf{R^2}$ et $\mathbf{R^3}$ forment ensemble avec les atomes de carbone qui les portent un cycle benzo éventuellement substitué ;
➢ $\mathbf{R^4}$**,** $\mathbf{R^5}$ et $\mathbf{R^6}$**,** identiques ou différents, représentent :

- un atome d'hydrogène ;
- un radical alkyle en $C_1$-$C_6$, de préférence en $C_1$-$C_2$, éventuellement substitué par un ou plusieurs radicaux choisis parmi les radicaux hydroxy, alcoxy en $C_1$-$C_2$, (poly)-hydroxyalcoxy en $C_2$-$C_4$, acylamino, amino substitué par deux radicaux alkyle, identiques ou différents, en $C_1$-$C_4$ ;
- un atome d'halogène tel que chlore, fluor ou brome ;
- un groupement hydroxy ;
- un radical alcoxy en $C_1$-$C_2$ ;
- un radical alkylthio en $C_1$-$C_2$ ;
- un radical amino ;
- un radical héterocycloalkyle à 5 ou 6 chaînons pouvant être substitués par 1 à 3 groupements identiques ou différents choisis parmi hydroxy, amino, (di)alkyamino, hydroxyalkyle en $C_1$-$C_4$, tel que morpholino, (hydroxyéthyl)pipérazino, (di)(hydroxy)pyrrolidino (di)(hydroxy)pipéridino ;
- un radical amino substitué par un ou deux radicaux alkyle, identiques ou différents, en $C_1$-$C_6$ éventuellement porteurs d'au moins :

  i) un groupement hydroxy,
  ii) un groupement amino éventuellement substitué par un ou deux radicaux alkyle en $C_1$-$C_3$, lesdits radicaux alkyle pouvant former avec l'atome d'azote auquel ils sont rattachés, un hétérocycle comprenant de 5 à 7 chaînons, saturé ou insaturé éventuellement substitué comprenant éventuellement au moins un autre hétéroatome différent ou non de l'azote,

- -N(R)-C(O)-R' dans lequel le radical R est un atome d'hydrogène, un radical alkyle en $C_1$-$C_4$ éventuellement porteur d'au moins un groupement hydroxy et le radical R' est un radical alkyle en $C_1$-$C_2$ ;
- un radical carboxylique sous forme acide ou salifiée (de préférence avec un métal alcalin ou un ammonium, substitué ou non) ;

- un groupement polyhalogénoalkyle comprenant de 1 à 6 atomes de carbones et de 1 à 6 atome d'halogène, identiques ou différents, le groupement polyhalogénoalkyle est par exemple le trifluorométhyle
- $R_4$ et $R_5$ sont de préférence des atomes d'hydrogène et $R^6$ est de préférence positionné sur l'atome de carbone 4 du cycle phényle, $R^6$ représente particulièrement un groupement amino substitué ;

➢ ou alors deux radicaux contigus $R^4$ avec $R^5$ et/ou $R^5$ avec $R^6$ forment ensemble avec les atomes de carbone qui les portent un cycle benzo ou un hétérocycle fusionné éventuellement substitué au groupe phényle, notamment un hétérocycle choisi parmi morpholinyle, pipérazinyle et pipéridinyle, indolyle, ces derniers étant fusionnés sur le groupe phényle de la formule (I) ou (II) préférentiellement en b-[3,4] ou $R^4$ et $R^5$ forment particulièrement ensemble, avec le groupement phényle de la formule (I) ou (II) qui les porte un groupement carbazole ;

➢ ou alors $R^4$, $R^5$ et $R^6$ forment ensemble avec le groupement phényle de la formule (II) un motif tricyclique de type julolidine ;

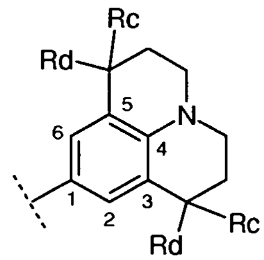

avec Rc et Rd représentant un atome d'hydrogène ou une groupement alkyle en $C_1$-$C_4$ ;

➢ L représente une chaîne hydrocarbonée bivalente en $C_1$-$C_{20}$, éventuellement substituée, éventuellement interrompue et/ou éventuellement terminée à l'une ou l'autre de ses extrémités i) par un ou plusieurs groupes divalents ou leur combinaisons choisis parmi : -N(R)-; $-N^+(R)(R°)-$, $An^-$; -O-, -S-, -CO-, $-SO_2-$ avec R, R°, identiques ou différents, choisi parmi un hydrogène, un radical alkyle en $C_1$-$C_4$, hydroxyalkyle, aminoalkyle et $An^-$ représentant un contre-ion anionique ou ii) par un hétérocycle cationique ou hétéroaryle cationique $Hét^+$, $An^-$, avec $An^-$ tel que défini précédemment et $Het^+$ représentant un hétérocycle comprenant de 5 à 10 chaînons, saturé ou non, ou un hétéroaryle comprenant de 5 à 10 chaînons tel que imidazolium, pyridinium, pipérazinium, pipéridinium, pyrrolidinium, benzoimidazolium; notamment L représente une chaîne ($C_1$-$C_6$)alkylène telle que méthylène, éthylène, propylène ou butylène ;

➢ **X** représente un atome d'oxygène, de soufre ou un groupement NR avec R représentant un atome d'hydrogène ou un groupement ($C_1$-$C_6$)alkyle ;

➢ **Y** représente : i) un atome d'hydrogène ; ii) un métal alcalin ; iii) un métal alcalinoterreux ; iv) un groupement ammonium : $N^+R^{\alpha}R^{\beta}R^{\gamma}R^{\delta}$, $An''^-$ ou un groupement phosphonium : $P^+R^{\alpha}R^{\beta}R^{\gamma}R^{\delta}$, $An''^-$ avec $R^{\alpha}$, $R^{\beta}$, $R^{\gamma}$ et $R^{\delta}$, identiques ou différents, représentant un atome d'hydrogène ou un groupement ($C_1$-$C_4$)alkyle et , $An''^-$ un contre-ion anionique ; ou v) un groupement protecteur de fonction thiol ;

➢ **W** représente un atome d'oxygène, de soufre ou un groupement NR' avec R' représente un atome d'hydrogène ou un groupe ($C_1$-$C_4$)alkyle ;

➢ **Q⁻** représente un contre-ion anionique ;

étant entendu que lorsque n vaut 2 alors m vaut zéro, et lorsque n vaut 1 alors m vaut 1.

Un autre objet de l'invention est une composition tinctoriale pour la coloration de fibres kératiniques avec effet éclaircissant comprenant, dans un milieu cosmétique au moins un colorant fluorescent hémicyanine styryle disulfure ou thiol de formule **(I)** ou **(II)** tels que définis précédemment, et éventuellement un agent réducteur.

L'invention a aussi pour objet de nouveaux colorants fluorescents hémicyanines styryles disulfures ou thiols de formule **(I)** ou **(II)** tels que définis précédemment.

Un autre objet de l'invention est un dispositif à plusieurs compartiments dans lequel un premier compartiment contient une composition tinctoriale comprenant au moins un colorant fluorescent de formule **(I)** ou **(II)** tel que défini précédemment et un deuxième compartiment contient un agent réducteur et éventuellement un troisième compartiment comprenant un un agent oxydant.

Le procédé de coloration selon l'invention permet de colorer de façon visible les matières kératiniques foncées, en particulier les fibres kératiniques humaines foncées, notamment les cheveux foncés. De plus le procédé de l'invention permet d'obtenir une coloration des cheveux, sans les dégrader, rémanente vis-à-vis de shampooings, des agressions courantes (soleil, transpiration), et de traitements capillaires. Le procédé de l'invention permet également d'obtenir un éclaircissement des matières kératiniques telles que les fibres kératiniques particulièrement les fibres kératiniques

foncées et plus particulièrement les cheveux foncés.

Par ailleurs les nouveaux colorants selon l'invention présentent une photostabilité, et une stabilité chimique très satisfaisantes. Ces colorants sont solubles dans les milieux cosmétiques appropriés aux teintures capillaires et tout particulièrement dans les mélanges eau/éthanol. Ils permettent d'étendre la gamme colorielle accessible (colorants jaunes, oranges, rouges, violets et bleus) tout en permettant un décapage des colorations obtenues sans altération de la fibre. Ce procédé permet également de colorer des fibres kératiniques décolorées de façon puissante et chromatique.

La gamme tinctoriale obtenue à partir des colorants de l'invention couvre également les nuances fondamentales les plus demandées en teinture capillaire.

Au sens de l'invention, on entend par matière kératinique foncée celle qui présente une luminescence L* chiffrée dans le système C.I.E.L*a*b*, inférieure ou égale à 45 et de préférence inférieure ou égale à 40, sachant par ailleurs que L*=0 équivaut au noir et L*=100 au blanc.

Au sens de l'invention, on entend par cheveux naturellement ou artificiellement foncés, des cheveux dont la hauteur de ton est inférieure ou égale à 6 (blond foncé) et de préférence inférieure ou égale à 4 (châtain).

L'éclaircissement des cheveux est évalué par la variation de «hauteur de ton» avant et après application du composé de formule (I) et (II). La notion « ton » repose sur la classification des nuances naturelles, un ton séparant chaque nuance de celle qui la suit ou la précède immédiatement. Cette définition et la classification des nuances naturelles est bien connue des professionnels de la coiffure et publiée dans l'ouvrage « Science des traitements capillaires » de Charles ZVIAK 1988, Ed. Masson, p. 215 et 278.

Les hauteurs de ton s'échelonnent de 1 (noir) à 10 (blond très clair), une unité correspondant à un ton ; plus le chiffre est élevé et plus la nuance est claire.

Un cheveu coloré artificiellement est un cheveu dont la couleur a été modifiée par un traitement de coloration par exemple une coloration avec des colorants directs ou des colorants d'oxydation.

Au sens de l'invention, on entend par « cheveux décolorés », des cheveux dont la hauteur de ton est supérieure à 6 et de préférence supérieure à 8.

Au sens de l'invention on entend par « décapage » de la coloration des fibres kératiniques un procédé qui fait intervenir l'application d'un stimulus chimique ou physique apte à ramener le cheveu vers sa couleur originelle. A titre d'exemple le décapage peut être obtenu par application d'une composition oxydante à base de peroxyde d'hydrogène, de persulfate de sodium, de potassium ou d'ammonium, à un pH modérément alcalin, *i.e.* compris entre 7 et 12, préférentiellement entre 8 et 10,5.

Un moyen pour mesurer l'effet éclaircissant est d'utiliser le phénomène de réflectance des cheveux.

De préférence, la composition doit, après application sur des cheveux foncés amener aux résultats ci-dessous.

- On s'intéresse aux performances de réflectance des cheveux lorsqu'on les irradie avec de la lumière visible dans la gamme de longueurs d'onde allant de 400 à 700 nanomètres.
- On compare alors les courbes de réflectance en fonction de la longueur d'onde, des cheveux traités avec la composition de l'invention et des cheveux non traités.
- La courbe correspondant aux cheveux traités doit montrer une réflectance dans la gamme des longueurs d'onde allant de 500 à 700 nanomètres supérieure à la courbe correspondant aux cheveux non traités.
- Cela signifie que, dans la gamme de longueur d'onde allant de 540 à 700 nanomètres, il existe au moins une plage où la courbe de réflectance correspondant aux cheveux traités est supérieure à la courbe de réflectance correspondant aux cheveux non traités. On entend par "supérieure", un écart d'au moins 0,05% de réflectance, et de préférence d'au moins 0,1%. Ceci n'empêche pas qu'il peut exister dans la gamme de longueur d'onde allant de 540 à 700 nanomètres, au moins une plage où la courbe de réflectance correspondant aux cheveux traités soit superposable, ou inférieure à la courbe de réflectance correspondant aux cheveux non traités.

De préférence, la longueur d'onde où l'écart est maximal entre la courbe de réflectance des cheveux traités et celle des cheveux non traités, se situe dans la gamme de longueur d'onde allant de 500 à 650 nanomètres, et de préférence dans la gamme de longueur d'onde allant de 550 à 620 nanomètres.

Au sens de la présente invention, et à moins qu'une indication différente ne soit donnée :

- les radicaux « aryle » ou « hétéroaryle » ou la partie aryle ou hétéroaryle d'un radical peuvent être substitués par au moins un substituant choisi parmi :

  - un radical alkyle en $C_1$-$C_{16}$, de préférence en $C_1$-$C_8$, éventuellement substitué par un ou plusieurs radicaux choisis parmi les radicaux hydroxy, alcoxy en $C_1$-$C_2$, (poly)-hydroxyalcoxy en $C_2$-$C_4$, acylamino, amino substitué par deux radicaux alkyle, identiques ou différents, en $C_1$-$C_4$, éventuellement porteurs d'au moins un groupement hydroxy ou, les deux radicaux pouvant former avec l'atome d'azote auquel ils sont rattachés, un hétérocycle comprenant de 5 à 7 chaînons, de préférence de 5 ou 6 chaînons, saturé ou insaturé éventuellement substitué

comprenant éventuellement un autre hétéroatome identique ou différent de l'azote ;

- un atome d'halogène tel que chlore, fluor ou brome ;
- un groupement hydroxy ;
- un radical alcoxy en $C_1$-$C_2$ ;
- un radical alkylthio en $C_1$-$C_2$ ;
- un radical (poly)-hydroxyalcoxy en $C_2$-$C_4$ ;
- un radical amino ;
- un radical héterocycloalkyle à 5 ou 6 chaînons ;
- un radical hétéroaryle à 5 ou 6 chaînons éventuellement cationique, préférentiellement imidazolium, et éventuellement substitué par un radical ($C_1$-$C_4$) alkyle, préférentiellement méthyle ;
- un radical amino substitué par un ou deux radicaux alkyle, identiques ou différents, en $C_1$-$C_6$ éventuellement porteurs d'au moins :

  i) un groupement hydroxy,
  ii) un groupement amino éventuellement substitué par un ou deux radicaux alkyle en $C_1$-$C_3$ éventuellement substitués, lesdits radicaux alkyle pouvant former avec l'atome d'azote auquel ils sont rattachés, un hétérocycle comprenant de 5 à 7 chaînons, saturé ou insaturé éventuellement substitué comprenant éventuellement au moins un autre hétéroatome différent ou non de l'azote,

- -N(R)-C(O)-R' dans lequel le radical R est un atome d'hydrogène, un radical alkyle en $C_1$-$C_4$ éventuellement porteur d'au moins un groupement hydroxy et le radical R' est un radical alkyle en $C_1$-$C_2$ ;
- $(R)_2N$-C(O)- dans lequel les radicaux R, identiques ou non, représentent un atome d'hydrogène, un radical alkyle en $C_1$-$C_4$ éventuellement porteur d'au moins un groupement hydroxy ;
- $R'S(O)_2$-N(R)- dans lequel le radical R représente un atome d'hydrogène, un radical alkyle en $C_1$-$C_4$ éventuellement porteur d'au moins un groupement hydroxy et le radical R' représente un radical alkyle en $C_1$-$C_4$, un radical phényle ;
- $(R)_2N$-$S(O)_2$- dans lequel les radicaux R, identiques ou non, représentent un atome d'hydrogène, un radical alkyle en $C_1$-$C_4$ éventuellement porteur d'au moins un groupement hydroxy,
- un radical carboxylique sous forme acide ou salifiée (de préférence avec un métal alcalin ou un ammonium, substitué ou non) ;
- un groupement cyano ;
- un groupement polyhalogénoalkyle comprenant de 1 à 6 atomes de carbones et de 1 à 6 atome d'halogène, identiques ou différents, le groupement polyhalogénoalkyle est par exemple le trifluorométhyle ;

- la partie cyclique ou hétérocyclique d'un radical non aromatique peut être substituée par au moins un substituant choisi parmi les groupements :

  - hydroxy,
  - alcoxy en $C_1$-$C_4$,
  - alkyle en $C_1$-$C_4$,
  - (poly)hydroxyalcoxy en $C_2$-$C_4$,
  - un radical alkylthio en $C_1$-$C_2$ ;
  - RC(O)-N(R')- dans lequel le radical R' est un atome d'hydrogène, un radical alkyle en $C_1$-$C_4$ éventuellement porteur d'au moins un groupement hydroxy et le radical R est un radical alkyle en $C_1$-$C_2$, amino substitué par deux groupements alkyle identiques ou différents en $C_1$-$C_4$, éventuellement porteurs d'au moins un groupement hydroxy ;
  - RC(O)-O- dans lequel le radical R est un radical alkyle en $C_1$-$C_4$, amino substitué par un ou deux groupements alkyle identiques ou différents en $C_1$-$C_4$ éventuellement porteurs d'au moins un groupement hydroxy, lesdits radicaux alkyle pouvant former avec l'atome d'azote auquel ils sont rattachés, un hétérocycle comprenant de 5 à 7 chaînons, saturé ou insaturé éventuellement substitué comprenant éventuellement au moins un autre hétéroatome différent ou non de l'azote ;
  - RO-C(O)- dans lequel le radical R est un radical alkyle en $C_1$-$C_4$ éventuellement porteurs d'au moins un groupement hydroxy ;

- un radical cyclique, hétérocyclique, ou une partie non aromatique d'un radical aryle ou hétéroaryle, peut également être substitué par un ou plusieurs groupements oxo ou thioxo ;
- un radical « aryle » représente un groupement mono ou polycyclique, condensé ou non, comprenant de 6 à 22 atomes de carbones, et dont au moins un cycle est aromatique ; préférentiellement le radical aryle est un phényle,

biphényle, naphtyle, indényle, anthracényle, ou tétrahydronaphtyle ;

- un radical « diarylalkyle » représente un groupement comportant sur le même atome de carbone d'un groupement alkyle deux groupement aryle, identiques ou différents tel que diphénylméthyle ou 1,1-diphényléthyle ;
- un « radical hétéroaryle » représente un groupement mono ou polycyclique, condensé ou non, éventuellement cationique, comprenant de 5 à 22 chaînons, de 1 à 6 hétéroatomes choisis parmi l'atome d'azote, d'oxygène, de soufre et de sélénium, et dont au moins un cycle est aromatique ; préférentiellement un radical hétéroaryle est choisis parmi acridinyle, benzimidazolyle, benzobistriazolyle, benzopyrazolyle, benzopyridazinyle, benzoquinolyle, benzothiazolyle, benzotriazolyle, benzoxazolyle, pyridinyle, tétrazolyle, dihydrothiazolyle, imidazopyridinyle, imidazolyle, indolyle, isoquinolyle, naphthoimidazolyle, naphthooxazolyle, naphthopyrazolyle, oxadiazolyle, oxazolyle, oxazolopyridyle, phénazinyle, phénooxazolyle, pyrazinyle, pyrazolyle, pyrilyle, pyrazoyltriazyle, pyridyle, pyridinoimidazolyle, pyrrolyle, quinolyle, tétrazolyle, thiadiazolyle, thiazolyle, thiazolopyridinyle, thiazoylimidazolyle, thiopyrylyle, triazolyle, xanthylyle et son sel d'ammonium ;
- un radical « dihétéroarylalkyle » représente un groupement comportant sur le même atome de carbone d'un groupement alkyle deux groupement hétéroaryle, identiques ou différents tel que difurylméthyle, 1,1-difuryléthyle, dipyrrolylméthyle, dithiénylméthyle ;
- un « radical cyclique » est un radical cycloalkyle non aromatique, mono ou polycyclique, condensé ou non, contenant de 5 à 22 atomes de carbone, pouvant comporter de 1 à plusieurs insaturations ; particulièrement le radical cyclique est un cyclohexyle ;
- un radical « cyclique stériquement encombré» est un radical cyclique, aromatique ou non, substitué ou non, encombré par effet ou contrainte stérique, comprenant de 6 à 14 chaînons, pouvant être pontés, à titre de radicaux stériquement encombrés on peut citer le bicyclo[1.1.0]butane, les mésytyles tels que le 1,3,5-triméthylpnényle, le 1,3,5-triterbutylphényle, le 1,3,5-isobutylphényle, le 1,3,5-trimétylsillylphényle et l'adamantyle ;
- un « radical hétérocyclique ou hétérocycle» est un radical non aromatique mono ou polycyclique, condensé ou non, contenant de 5 à 22 chaînons, comportant de 1 à 6 hétéroatomes choisis parmi l'atome d'azote, d'oxygène, de soufre et de sélénium ;
- un « radical alkyle » est un radical hydrocarboné en $C_1$-$C_{16}$, linéaire ou ramifié, de préférence en $C_1$-$C_8$ ;
- un radical « alkylène » est un radical divalent alkyle tel que défini ci-dessus ;
- un radical « alkénylène » est un radical hydrocarboné divalent comportant de une à trois doubles liaisons carbone-carbone, particulièrement les doubles liaisons sont conjuguées telle que -CH=CH-, -CH=CH-CH=CH-, -CH=CH-CH=CH-CH=CH- ;
- l'expression « éventuellement substitué » attribué au radical alkyle sous entend que ledit radical alkyle peut être substitué par un ou plusieurs radicaux choisis parmi les radicaux i) hydroxy, ii) alcoxy en $C_1$-$C_4$, iii) acylamino, iv) amino éventuellement substitué par un ou deux radicaux alkyle, identiques ou différents, en $C_1$-$C_4$, lesdits radicaux alkyles pouvant former avec l'atome d'azote qui les portent un hétérocyle comprenant de 5 à 7 chaînons, comprenant éventuellement un autre hétéroatome différent ou non de l'azote ; v) ou un groupement ammonium quaternaire $-N^+R'R''R'''$, $M^-$ pour lequel R', R'', R''', identiques ou différents représentent un atome d'hydrogène, ou un groupement alkyle en $C_1$-$C_4$, ou alors $-N^+R'R''R'''$ forme un hétéroaryle tel que imidazolium éventuellement substitué par un groupement $C_1$-$C_4$ alkyle, et $M^-$ représente le contre-ion de l'acide organique, minéral ou de l'halogénure correspondant,
- un « radical alcoxy » est un radical alkyle-oxy ou alkyl-O- pour lequel le radical alkyle est un radical hydrocarboné, linéaire ou ramifié, en $C_1$-$C_{16}$ préférentiellement en $C_1$-$C_8$ ;
- un « radical alkylthio » est un radical alkyl-S- pour lequel le radical alkyle est un radical hydrocarboné, linéaire ou ramifié, en $C_1$-$C_{16}$ préférentiellement en $C_1$-$C_8$, lorsque le groupement alkylthio est éventuellement substitué, cela sous entend que le groupement alkyle est éventuellement substitué tel que défini précédemment ;
- les bornes délimitant l'étendue d'une plage de valeurs sont comprises dans cette plage de valeurs ;
- un « sel d'acide organique ou minéral » est plus particulièrement choisi parmi un sel dérivé i) d'acide chlorhydrique HCl, ii) d'acide bromhydrique HBr, iii) d'acide sulfurique $H_2SO_4$, iv) d'acides alkylsulfoniques : Alk-$S(O)_2OH$ tels que d'acide méthylsulfonique et d'acide éthylsulfonique ; v) d'acides arylsulfoniques : Ar-$S(O)_2OH$ tel que d'acide benzène sulfonique et d'acide toluène sulfonique ; vi) d'acide citrique ; vii) d'acide succinique ; viii) d'acide tartrique ; ix) d'acide lactique, x) d'acides alkoxysulfiniques : Alk-O-S(O)OH tels que d'acide méthoxysulfinique et d'acide éthoxysulfinique ; xi) d'acides aryloxysulfiniques tels que d'acide toluèneoxysulfinique et d'acide phénoxysulfinique ; xii) d'acide phosphorique $H_3PO_4$; xiii) d'acide acétique $CH_3C(O)OH$ ; xiv) d'acide triflique $CF_3SO_3H$ et xv) d'acide tétrafluoroborique $HBF_4$ ;
- un « contre-ion anionique » est un anion ou un groupement anionique associé à la charge cationique du colorant ; plus particulièrement le contre-ion anionique est choisi parmi i) les halogénures tels que le chlorure, le bromure ; ii) les nitrates ; iii) les sulfonates parmi lesquels les $C_1$-$C_6$ alkylsulfonates : Alk-$S(O)_2O^-$ tels que le méthylsulfonate ou mésylate et l'éthylsulfonate ; iv) les arylsulfonates : Ar-$S(O)_2O^-$ tel que le benzènesulfonate et le toluènesulfonate ou tosylate ; v) le citrate ; vi) le succinate ; vii) le tartrate ; viii) le lactate ; ix) les alkylsulfates : Alk-O-S(O)$O^-$ tels que

le méthysulfate et l'éthylsulfate; x) les arylsulfates : Ar-O-S(O)$O^-$ tels que le benzènesulfate et le toluènesulfate ; xi) les alkoxysulfates: Alk-O-S$(O)_2O^-$ tel que le méthoxy sulfate et l'éthoxysulfate ; xii) les aryloxysulfates : Ar-O-S$(O)_2O^-$, xiii) le phosphate ; xiv) l'acétate ; xv) le triflate ; et xvi) les borates tels que le tétrafluoroborate.

- Les « solvates » représentent les hydrates ainsi que l'association avec des alcools linéaires ou ramifiés en C1-C4 linéaire ou ramifié tels que l'éthanol, l'isopropanol, le n-propanol.

Les colorants hémicyanines styryles disulfures ou thiols de formule **(I)** ou **(II)** tels que définis précédemment sont des colorants fluorescents, *i.e.* capables d'absorber dans le rayonnement UV ou visible à une longueur d'onde $\lambda_{abs}$ comprise entre 250 et 800 nm et capables de réémettre dans le domaine du visible à une longueur d'onde d'émission $\lambda_{ém}$ comprise entre 400 et 800 nm.

De préférence les composés fluorescents de formules **(I)** ou **(II)** de l'invention sont des colorants capables d'absorber dans le visible $\lambda_{abs}$ comprise entre 400 et 800 nm et de réémettre dans le visible $\lambda_{ém}$ comprise entre 400 et 800 nm. Plus préférentiellement les colorants de formules (I) ou (II) sont des colorants capables d'absorber à une $\lambda_{abs}$ comprise entre 420 nm et 550 nm et de réémettre dans le visible à une $\lambda_{ém}$ comprise entre 470 et 600 nm.

Les composés de l'invention de formule **(I)** ou **(II)** lorsque n et m valent 1, contiennent une fonction SY qui peut se trouver sous la forme covalente -S-Y ou ionique $-S^-$ $Y^+$ selon la nature de Y et du pH du milieu.

Un mode particulier concerne les colorants fluorescents hémicyanines styryles disulfures ou thiols de formule **(I)** ou **(II)** avec n et m qui valent 1, et Y représente un atome d'hydrogène, ou un métal alcalin. Avantageusement Y représente un atome d'hydrogène.

Conformément à un autre mode de réalisation particulier de l'invention, dans la formule **(I)** ou **(II)** précitée, Y est un groupement protecteur connu par l'homme du métier comme par exemple ceux décris dans les ouvrages « Protective Groups in Organic Synthesis », T. W. Greene, John Willey & Sons ed., NY, 1981, pp.193-217; « Protecting Groups », P. Kocienski, Thieme, 3ème ed., 2005, chap. 5. Etant entendu que Y en tant que groupement protecteur ne peut pas représenter avec l'atome de soufre sur lequel il est lié un colorant disulfure i.e. ne peut représenter un composé de formule (I) ou (II) dans lequel n=m=1. Y comme groupement protecteur ne peut pas représenter un groupement directement relié à l'atome de soufre de formule (I) et (II) via un autre atome de soufre non oxydé.

Particulièrement lorsque Y représente un groupement protecteur de la fonction thiol, Y est choisi parmi les radicaux suivants :

■ $(C_1-C_4)$alkylcarbonyle ;
■ $(C_1-C_4)$alkylthiocarbonyle ;
■ $(C_1-C_4)$alcoxycarbonyle ;
■ $(C_1-C_4)$alcoxythiocarbonyle ;
■ $(C_1-C_4)$alkylthio-thiocarbonyle ;
■ (di) $(C_1-C_4)$ (alkyl)aminocarbonyle ;
■ (di) $(C_1-C_4)$ (alkyl)aminothiocarbonyle;
■ arylcarbonyle comme phénylcarbonyle ;
■ aryloxycarbonyle ;
■ aryl$(C_1-C_4)$alkcoxycarbonyle ;
■ (di) $(C_1-C_4)$ (alkyl)aminocarbonyle comme diméthylaminocarbonyle ;
■ $(C_1-C_4)$(alkyl)arylaminocarbonyle ;
■ carboxy ;
■ $SO_3^-$ ; $M^+$ avec $M^+$ représentant un métal alcalin tel que le sodium ou le potassium, ou alors $Q^-$ ou $An'^-$ de la formule **(I)** ou **(II)** et $M^+$ sont absents ;
■ aryle éventuellement substitué tel que le phényle, dibenzosubéryle, ou 1,3,5-cycloheptatriényle ;
■ hétéroaryle éventuellement substitué ; dont notamment l'hétéroaryle cationiques ou non, comprenant de 1 à 4 hétéroatomes suivants :

i) monocycliques à 5, 6 ou 7 chaînons tels que furanyle ou furyle, pyrrolyle ou pyrryle, thiophényle ou thiényle, pyrazolyle, oxazolyle, oxazolium, isoxazolyle, isoxazolium, thiazolyle, thiazolium, isothiazolyle, isothiazolium, 1,2,4-triazolyle, 1,2,4-triazolium, 1,2,3-triazolyle, 1,2,3-triazolium, 1,2,4-oxazolyle, 1,2,4-oxazolium, 1,2,4-thiadiazolyle, 1,2,4-thiadiazolium, pyrylium, thiopyridyle, pyridinium, pyrimidinyle, pyrimidinium, pyrazinyle, pyrazinium, pyridazinyle, pyridazinium, triazinyle, triazinium, tétrazinyle, tétrazinium, azépine, azépinium, oxazépinyle, oxazépinium, thiépinyle, thiépinium, imidazolyle, imidazolium ;

ii) bicycliques à 8 à 11 chaînons tels que indolyle, indolinium, benzoimidazolyle, benzoimidazolium, benzoxazolyle, benzoxazolium, dihydrobenzoxazolinyle, benzothiazolyle, benzothiazolium, pyridoimidazolyle, pyridoimidazolium, thiénocycloheptadiényle, ces groupes mono ou bicycliques étant éventuellement substitués par un ou plusieurs groupements tels que $(C_1-C_4)$alkyle comme méthyle, ou polyhalogéno$(C_1-C_4)$alkyle comme

trifluorométhyle ;
iii) ou tricyclique ABC suivant :

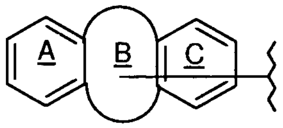

dans lequel les deux cycles A, C comportent éventuellement un hétéroatome, et le cycle B est un cycle à 5, 6 ou 7 chaînons particulièrement à 6 chaînons et contient au moins un hétéroatome comme pypéridyle, pyranyle ;

■ hétérocycloalkyle éventuellement substitué, éventuellement cationique, le groupement hétérocycloalkyle représente notamment un groupement monocyclique saturé ou partiellement saturé à 5, 6 ou 7 chaînons comprenant de 1 à 4 hétéroatomes choisis parmi l'oxygène, le soufre et l'azote, tel que di/tétrahydrofuranyle, di/tétrahydrothiophényle, di/tétrahydropyrrolyle, di/tétrahydropyranyle, di/tétra/hexa-hydrothiopyranyle, dihydropyridyle, pipérazinyle, pipéridinyle, tétraméthylpipéridinyle, morpholinyle, di/tétra/hexahydroazépinyle, di/tétrahydropyrimidinyle ces groupes étant éventuellement substitués par un ou plusieurs groupes comme $(C_1\text{-}C_4)$ alkyle, oxo ou thioxo ; ou l'hétérocycle représente le groupement suivant :

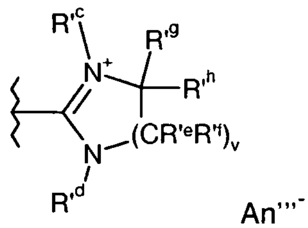

dans lequel $R'^c$, $R'^d$, $R'^e$, $R'^f$, $R'^g$ et $R'^h$, identiques ou différents représentent un atome d'hydrogène ou un groupement $(C_1\text{-}C_4)$ alkyle, ou alors deux groupement $R'^g$ avec $R'^h$, et/ou $R'^e$ avec $R'^f$ forment un groupement oxo ou thioxo, ou alors $R'^g$ avec $R'^e$ forment ensemble un cycloalkyle ; et v représente un entier compris inclusivement entre 1 et 3 ; préférentiellement $R'^c$ à $R'^h$ représentent un atome d'hydrogène ; et $An'''^-$ représente un contre-ion ;

■ isothiouronium ;
■ $-C(NR'^cR'^c)=N^+R'^eR'^f$; $An'''^-$ avec $R'^c$, $R'^d$, $R'^e$ et $R'^f$, identiques ou différents représentent un atome d'hydrogène ou un groupement $(C_1\text{-}C_4)$alkyle; préférentiellement $R'^c$ à $R'^f$ représentent un atome d'hydrogène ; et $An'''^-$représente un contre-ion ;
■ isothiourée ;
■ $-C(NR'^cR'^d)=NR'^e$; avec $R'^c$, $R'^d$ et $R'^e$ sont tels que définis précédemment ;
■ (di)aryl$(C_1\text{-}C_4)$alkyle éventuellement substitué tel que le 9-anthracénylméthyle, phénylméthyle ou diphénylméthyle éventuellement substitué par un plusieurs groupements notamment choisis parmi $(C_1\text{-}C_4)$ alkyle, $(C_1\text{-}C_4)$ alcoxy comme le méthoxy, hydroxy, alkylcarbonyle, (di)$(C_1\text{-}C_4)$(alkyl)amino comme le diméthylamino ;
■ (di)hétéroaryl$(C_1\text{-}C_4)$akyle éventuellement substitué, le groupement hétéroaryle est notamment, cationique ou non, monocyclique, comprenant 5 ou 6 chaînons et de 1 à 4 hétéroatomes choisis parmi l'azote, l'oxygène et le soufre, tels que les groupes pyrrolyle, furanyle, thiophényle, pyridyle, pyridyle N-oxyde tels que le 4-pyridyle ou 2-pyridyl-N-oxyde, pyrylium, pyridinium, triazinyle, éventuellement substitué par un ou plusieurs groupement tel que alkyle particulièrement méthyle, avantageusement le (di)hétéroaryl$(C_1\text{-}C_4)$akyle est (di)hétéroarylméthyle ou (di) hétéroaryléthyle ;
■ $CR^1R^2R^3$ avec $R^1$, $R^2$ et $R^3$ identiques ou différents, représentant un atome d'halogène ou un groupement choisi parmi :

- $(C_1\text{-}C_4)$alkyle ;
- $(C_1\text{-}C_4)$alcoxy ;
- aryle éventuellement substitué tel que phényle éventuellement substitué par un ou plusieurs groupements comme $(C_1\text{-}C_4)$alkyle, $(C_1\text{-}C_4)$alcoxy, hydroxy ;
- hétéroaryle éventuellement substitué tel que thiophényle, furanyle, pyrrolyle, pyranyle, pyridyle, éventuellement

substitué par un groupement $(C_1-C_4)$alkyle ;

- $P(Z^1)R'^1R'^2R'^3$ avec $R'^1$, et $R'^2$ identiques ou différents représentent un groupement hydroxy, $(C_1-C_4)$alcoxy ou alkyle, $R'^3$ représente un groupement hydroxy ou $(C_1-C_4)$alcoxy, et $Z^1$ représente un atome d'oxygène ou de soufre ;

■ cyclique stériquement encombré ; et

■ alcoxyalkyle éventuellement substitué tels que le méthoxyméthyle (MOM), éthoxyéthyle (EOM) et l'isobutoxyméthyle.

Selon un mode de réalisation particulier les colorants fluorescents thiols protégés de formule **(I)** ou **(II)** pour lesquels m et n valent 1, comportent un groupement Y i) hétéroaryle monocyclique à 5 ou 6 chaînons aromatiques, cationiques comprenant de 1 à 4 hétéroatomes choisis parmi l'oxygène, le soufre et l'azote, tels que oxazolium, isoxazolium, thiazolium, isothiazolium, 1,2,4-triazolium, 1,2,3-triazolium, 1,2,4-oxazolium, 1,2,4-thiadiazolium, pyrylium, pyridinium, pyrimidinium, pyrazinyle, pyrazinium, pyridazinium, triazinium, tétrazinium, oxazépinium, thiépinyle, thiépinium, imidazolium ; ii) hétéroaryle bicyclique à 8 à 11 chaînons cationique tels que indolinium, benzoimidazolium, benzoxazolium, benzothiazolium, ces groupes hétéroaryle mono ou bicycliques étant éventuellement substitués par un ou plusieurs groupements tels que alkyle comme méthyle, ou polyhalogéno$(C_1-C_4)$alkyle comme trifluorométhyle ; iii) ou hétérocyclique suivant :

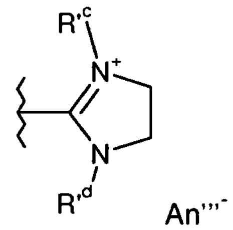

dans lequel $R'^c$ et $R'^d$, identiques ou différents, représentent un atome d'hydrogène ou un groupement $(C_1-C_4)$alkyle ; préférentiellement $R'^c$ à $R'^d$ représentent un groupement $(C_1-C_4)$alkyle tel que méthyle ; et $An'''^-$ représente un contre-ion.

Particulièrement Y représente un groupement choisi parmi oxazolium, isoxazolium, thiazolium, isothiazolium, 1,2,4-triazolium, 1,2,3-triazolium, 1,2,4-oxazolium, 1,2,4-thiadiazolium, pyrylium, pyridinium, pyrimidinium, pyrazinium, pyridazinium, triazinium et imidazolium, benzoimidazolium, benzoxazolium, benzothiazolium, ces groupements étant éventuellement substitués par un ou plusieurs groupes $(C_1-C_4)$ alkyle notamment méthyle.

En particulier Y représente un métal alcalin ou un groupement protecteur tel que:

➢ $(C_1-C_4)$alkylcarbonyle comme méthylcarbonyle ou éthylcarbonyle ;
➢ arylcarbonyle comme phénylcarbonyle ;
➢ $(C_1-C_4)$alkoxycarbonyle ;
➢ aryloxycarbonyle ;
➢ aryl$(C_1-C_4)$alkoxycarbonyle ;
➢ (di) $(C_1-C_4)$ (alkyl)aminocarbonyle comme diméthylaminocarbonyle;
➢ $(C_1-C_4)$(alkyl)arylaminocarbonyle ;
➢ aryle éventuellement substitué tel que le phényle ;
➢ hétéroaryle monocyclique à 5 ou 6 chaînons tels que imidazolyle ou pyridyle ;
➢ hétéroaryle monocyclique cationique à 5, 6 chaînons tels que pyrylium, pyridinium, pyrimidinium, pyrazinium, pyridazinium, triazinium, imidazolium ; ces groupements étant éventuellement substitués par un ou plusieurs, identiques ou différents, groupes $(C_1-C_4)$alkyle tel que méthyle ;
➢ hétéroaryle bicyclique cationique à 8 à 11 chaînons tels que benzoimidazolium, ou le benzoxazolium ; ces groupements étant éventuellement substitué par un ou plusieurs, identiques ou différents, groupes $(C_1-C_4)$alkyle tel que méthyle ;
➢ hétérocycle cationique de formule suivante :

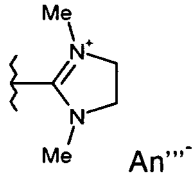

- ➢ isothiouronium ;
- ➢ $-C(NH_2)=N^+H_2$; An'''-;
- ➢ isothiourée ;
- ➢ $-C(NH_2)=NH$ ;
- ➢ $SO_3^-$, $M^+$ avec $M^+$ représentant un métal alcalin tel que le sodium ou le potassium, ou alors $Q^-$ ou An'- de la formule **(I)** ou **(II)** et $M^+$ sont absents.

Selon un mode particulier de l'invention les colorants fluorescent de formule **(I)** ou **(II)** sont des colorants disulfures avec n = 2 et m = 0.

Particulièrement les colorants fluorescents disulfures de formule **(I)** de l'invention sont choisis parmi ceux de formule **($I_a$)**, **($II_a$)**, **($I_b$)** et **($II_b$)** :

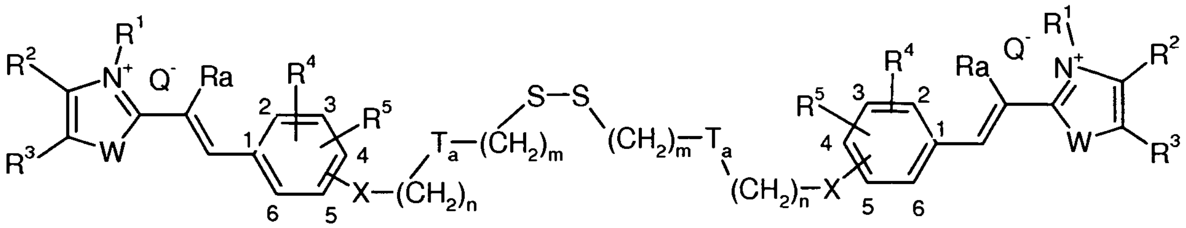

**($I_a$)**

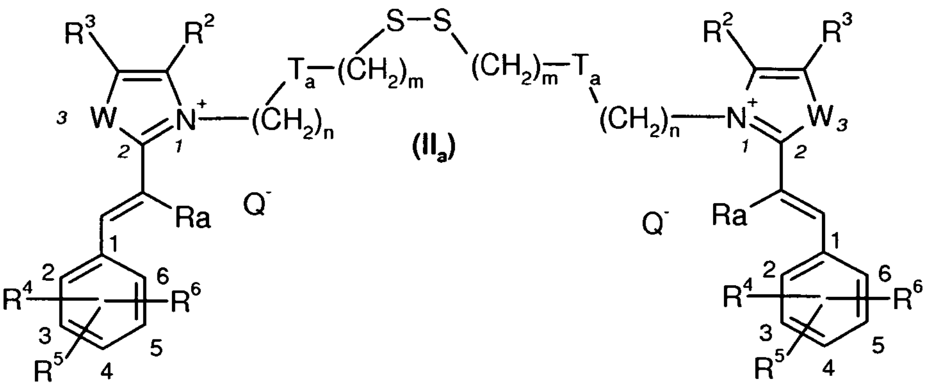

**($II_a$)**

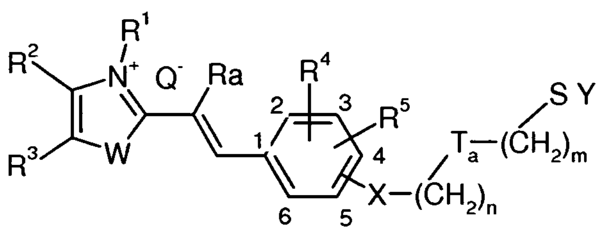

**($I_b$)**

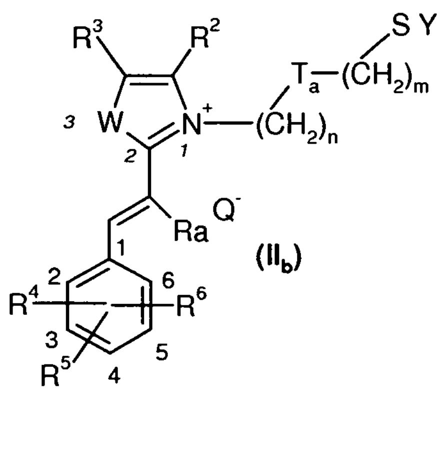

**($II_b$)**

formules **($I_a$)**, **($II_a$)**, **($I_b$)** et **($II_b$)** dans lesquelles :

- ➢ **Ra, $R^1$** à **$R^6$, B, Y** et **$Q^-$** sont tels que définis précédemment ;
- ➢ n et m, identiques ou différents, représentent un entier compris inclusivement entre 1 et 6 avec la somme de n+m qui est comprise inclusivement entre 2 et 4, particulièrement 2 ou 3 pour ($II_a$) ;
- ➢ **X** représente un radical choisi parmi : -G-, -G'-C(G)- et -C(G)-G'-, avec G et G', identiques ou différents, représentant un atome d'oxygène, de soufre, ou NR, avec R représentant un atome d'hydrogène ou un groupement ($C_1$-$C_6$)alkyle, avantageusement X représente un groupement -NR-, -NR-CO-, -C(O)-NR-, -$S(O)_2$-, -$S(O)_2$-NR- ; ou -NR-$S(O)_2$-, ou -NR- ; notamment X de la formule ($I_a$) est relié en position 4 sur le groupement phényle substitué ou non par les groupements $R^4$ et $R^5$ ;
- ➢ **$T_a$** représente une liaison covalente σ, un groupement -N($R'_a$)-; -$N^+$($R'_a$)($R'_b$)-, $An^-$; - C(O)-N($R'_a$)- ou -N($R'_a$)-C (O)-, ou un hétéroaryle cationique divalent comprenant de 5 à 7 chainons, tel que imidazolium, avec $R'_a$, $R'_b$, identiques ou différents, représentant un atome d'hydrogène, un radical ($C_1$-$C_4$)alkyle, $An^-$ représentant un contre-ion anionique ; particulièrement $T_a$ représente -C(O)-N($R'_a$)- et -N($R'_a$)-C(O)-.

Particulièrement les colorants de l'invention possèdent des radicaux X représentant un groupement NR en position 4 sur le phényle, $R^4$ et $R^5$ représentant un atome d'hydrogène ou alors formant ensemble un groupement benzo ou morpholinyle éventuellement substitué par un groupement alkyle, $R^2$ et $R^3$ représentant un atome d'hydrogène, d'halogène, ou alors formant ensemble un groupement benzo éventuellement substitué par de 1 à 3 atomes d'halogène, L représentant avantageusement un groupement éthylène, propylène ou butylène, plus particulièrement éthylène.

A titre d'exemple on peut citer les colorants de formule (I) ou (II) suivants

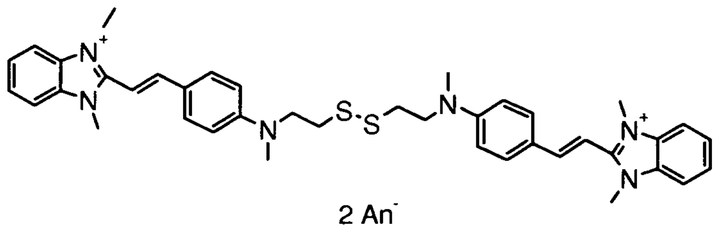

2 $An^-$

**1**

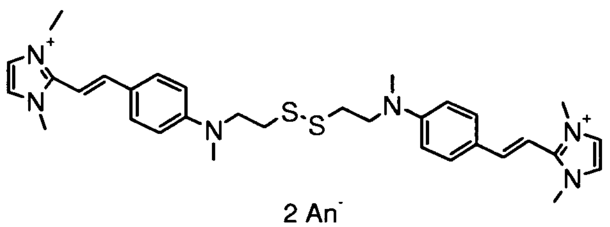

2 $An^-$

**2**

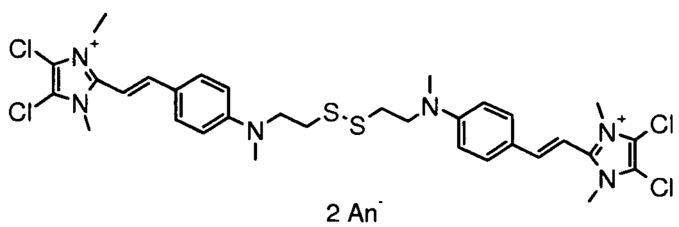

2 $An^-$

**3**

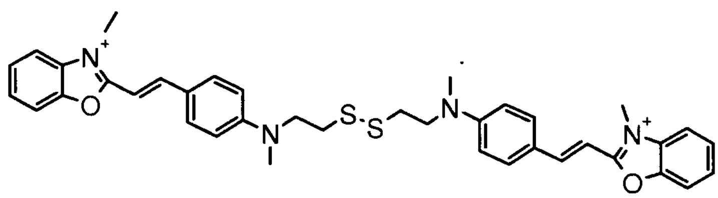

2 An⁻

**4**

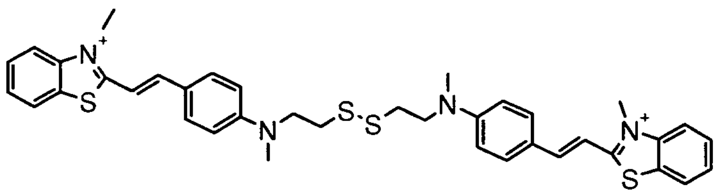

2 An⁻

**5**

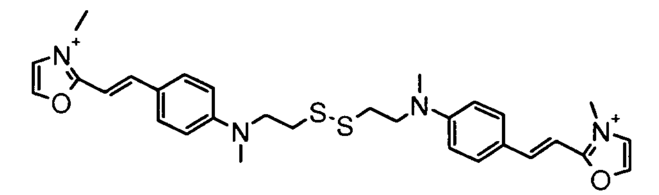

2 An⁻

**6**

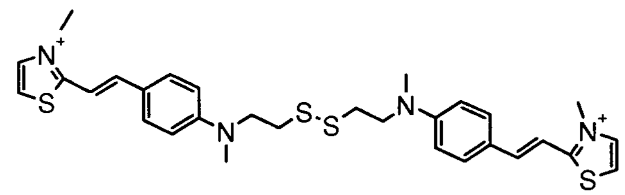

2 An⁻

**7**

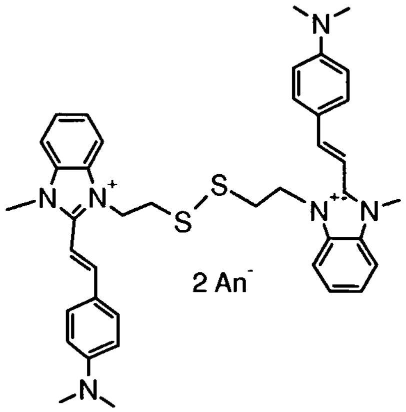

**8**

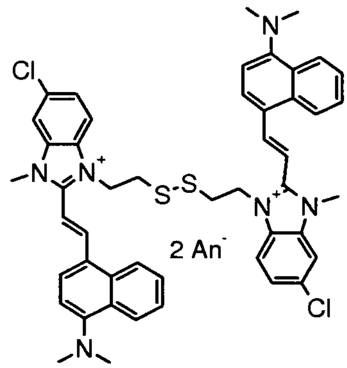

**9**

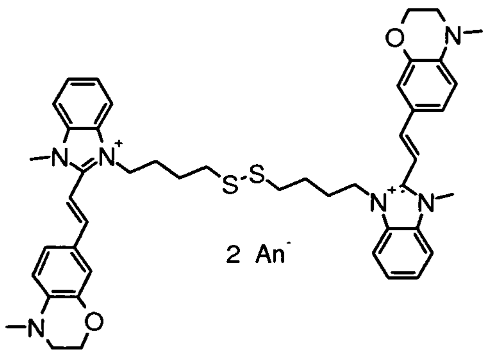

**10**

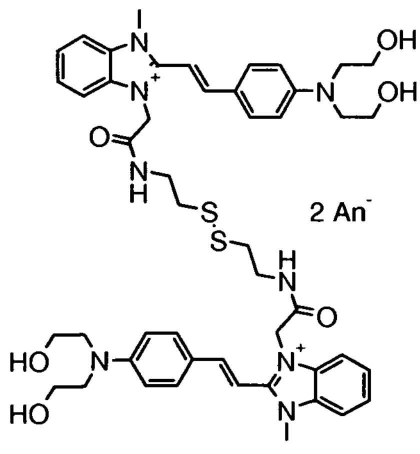

11

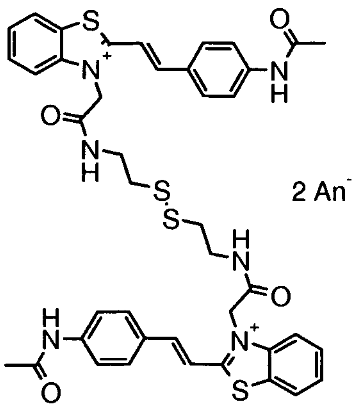

12

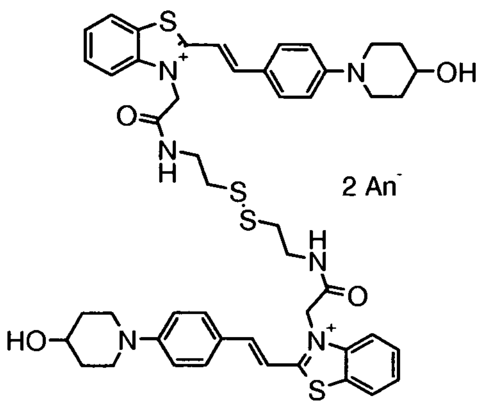

13

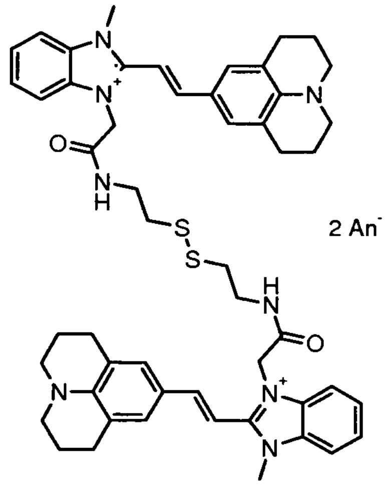

2 $An^-$

**14**

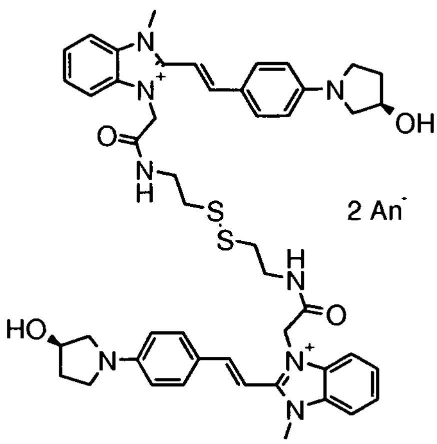

2 $An^-$

**15**

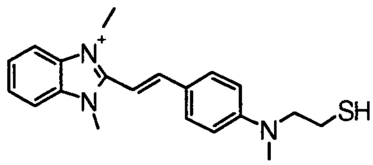

An-

**16**

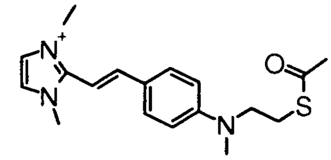

$An^-$

**17**

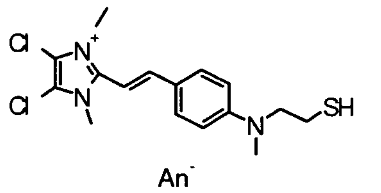

**18**

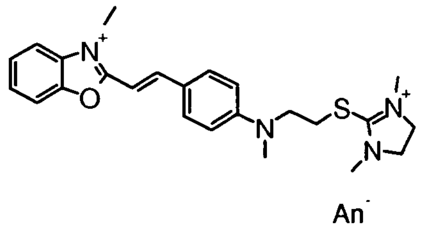

**19**

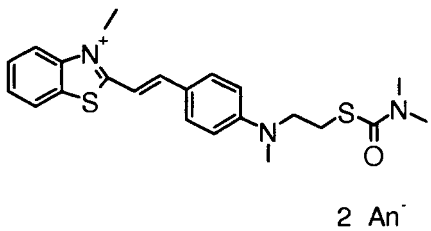

**20**

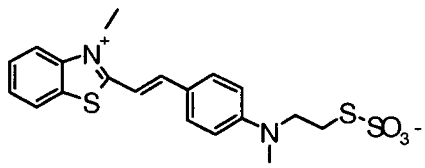

**21**

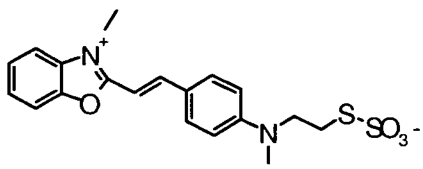

**22**

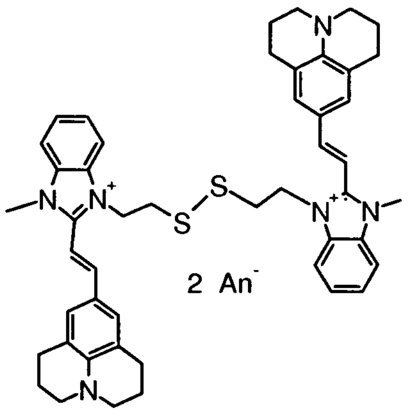

23

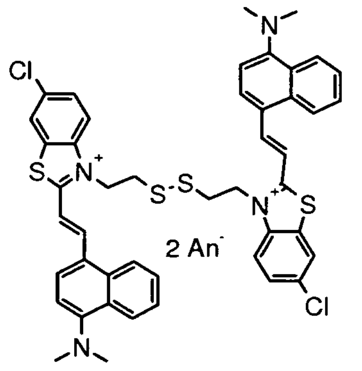

24

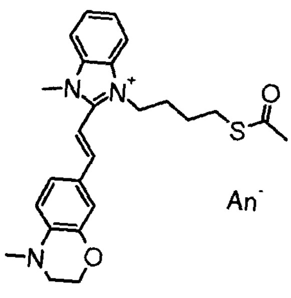

25

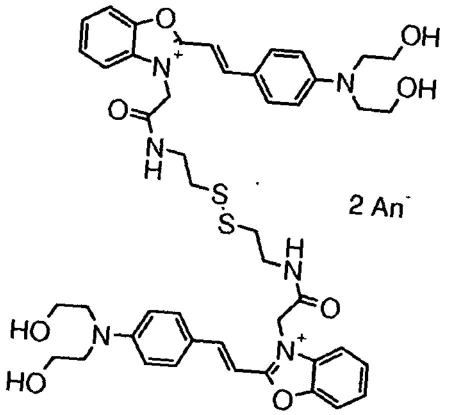

2 $An^-$

**26**

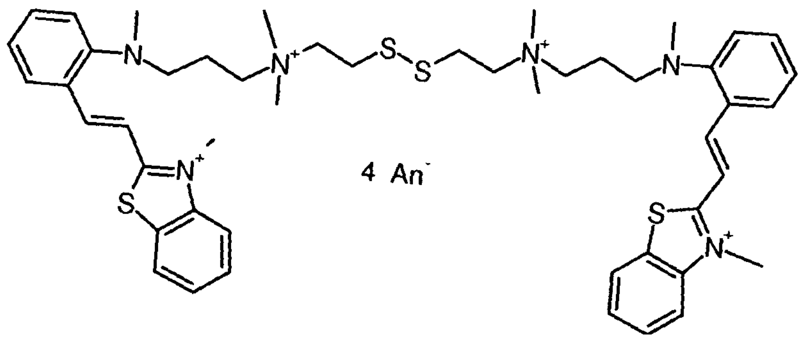

4 $An^-$

**27**

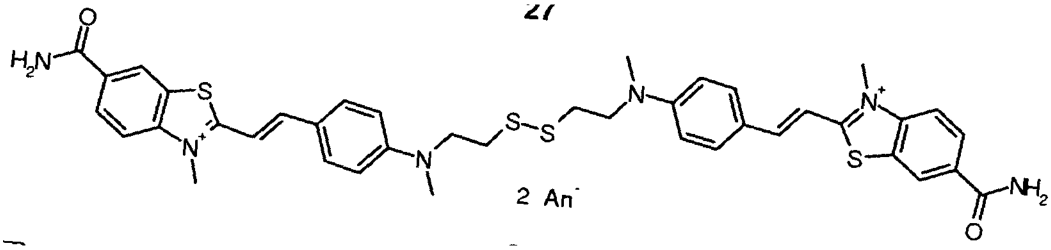

2 $An^-$

**28**

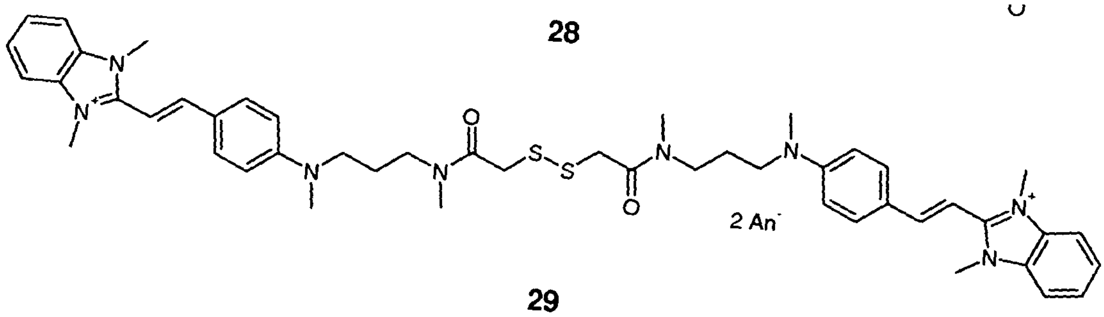

2 $An^-$

**29**

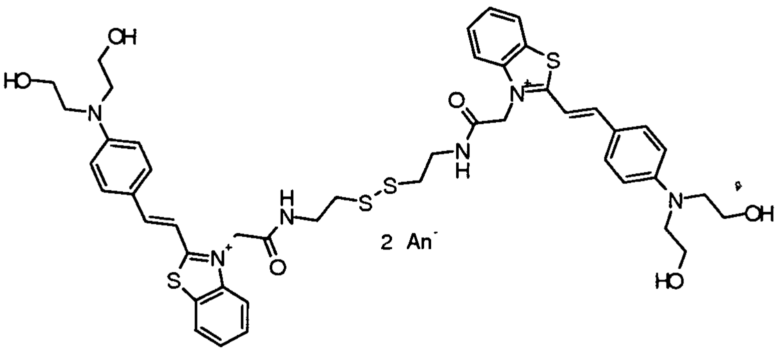

2 An⁻

**30**

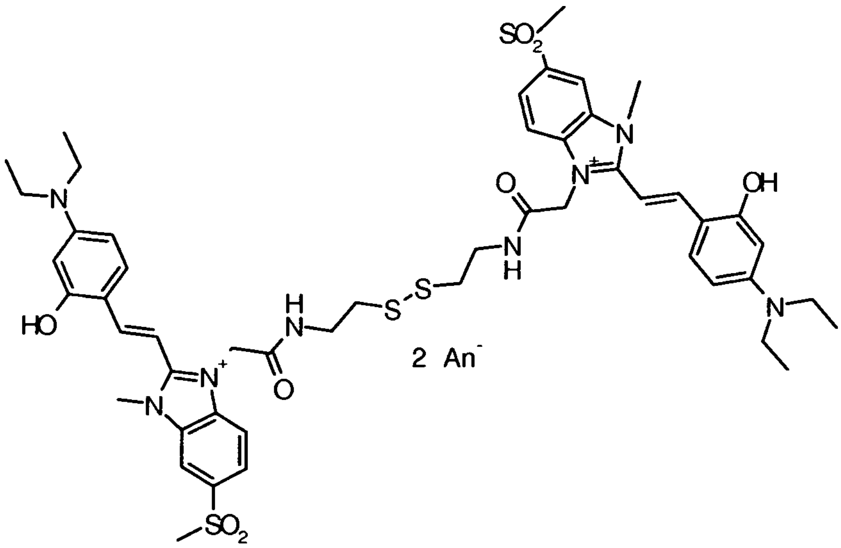

2 An⁻

**31**

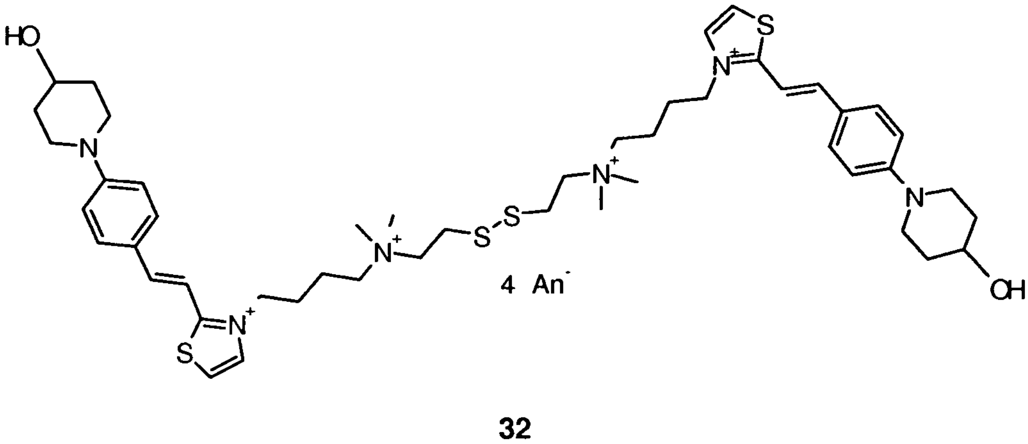

4 An⁻

**32**

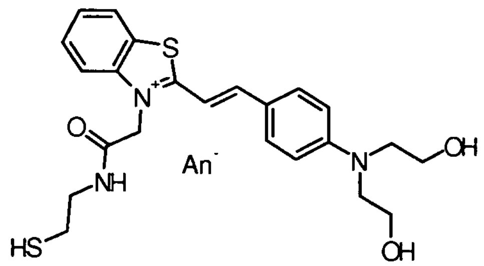

**33**

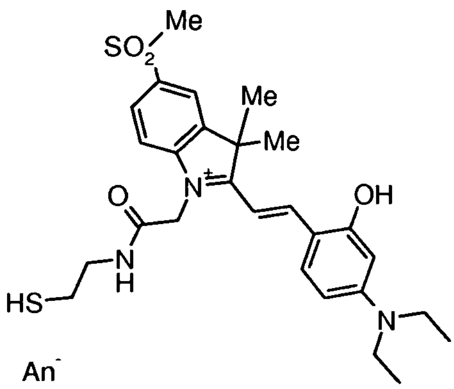

**34**

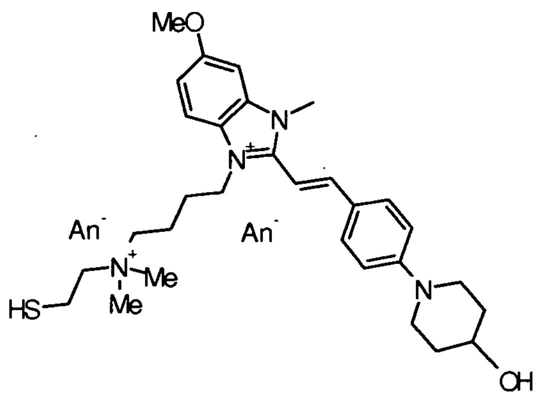

**35**

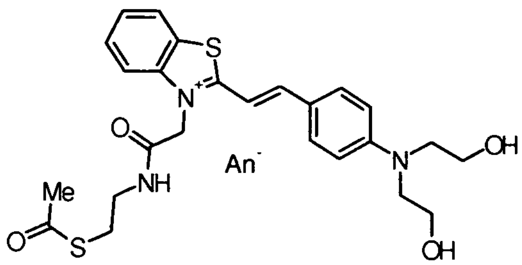

**36**

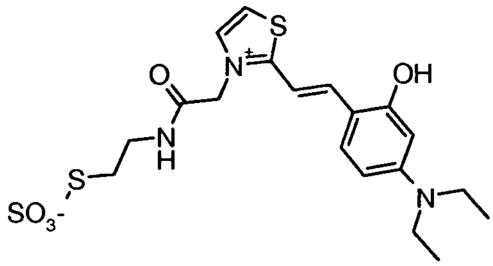

**37**

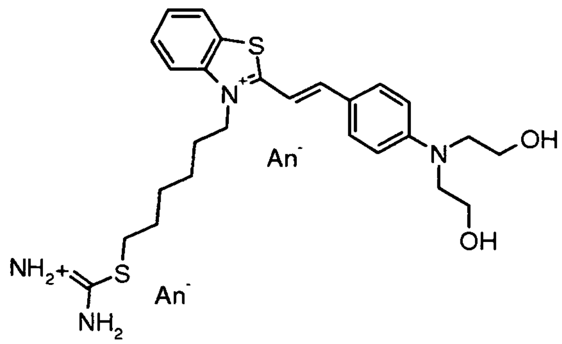

**38**

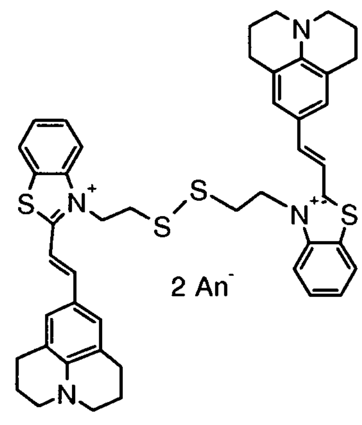

**39**

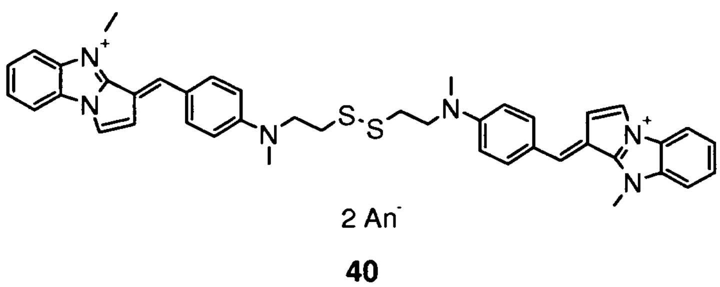

**40**

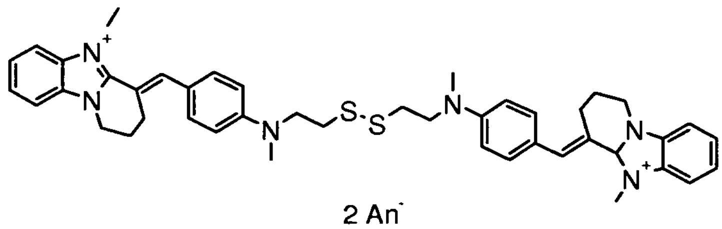

2 $An^-$

**41**

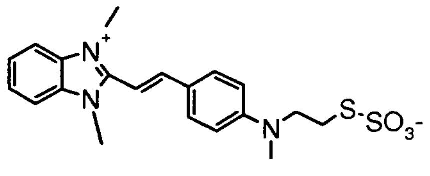

**42**

avec $An^-$, identiques ou différents, représentant un contre-ion anionique.

Pour tous les exemples des modes de réalisation de préparation qui suivent des nouveaux colorants fluorescents hémicyanines styryles thiols et disulfures de formule **(I)** et **(II),** l'homme du métier sait protéger au préalable les fonctions réactives telles que les fonctions cétones du chromophore puis les déprotéger pour les besoins de la réaction de synthèse, par les méthodes classiques connues de protection/déprotection comme celles décrites dans les ouvrages cités ci-dessus de T.W Greene John Willey & Sons ed., NY, 1981, ou P. Kocienski « Protecting Groups », P. Kocienski, Thieme, 3ème ed., 2005.

Les colorants thiols protégés de formule **(I-Y)** ou **(II-Y)** pour lesquels m et n valent 1, peuvent être synthétisés en deux étapes. La première étape consistant à préparer le colorant thiol non protégé **(I-H)** ou **(II-H)** selon les méthodes connues de l'homme de l'art comme par exemple « Thiols and organic Sulfides », « Thiocyanates and Isothiocyanates, organic », Ullmann's Encyclopedia, Wiley-VCH, Weinheim, 2005. Et la deuxième étape consistant à protéger la fonction thiol selon les méthodes classiques connues par l'homme du métier pour conduire aux colorants thiols protégés de formule **(I-Y)** ou **(II-Y).** A titre d'exemple pour protéger la fonction thiol -SH du colorant thiol on peut utiliser les méthodes des ouvrages « Protective Groups in Organic Synthesis », T. W. Greene, John Willey & Sons ed., NY, 1981, pp.193-217 ; « Protecting Groups », P. Kocienski, Thieme, 3ème ed., 2005, chap. 5. Nous pouvons illustrer cette méthode par la méthode consistant i) à générer des colorants hémicyanines styryles thiols de formule **(I-H)** ou **(II-H)** par réduction d'un colorant hémicyanine styryle à deux chromophores, portant une fonction disulfure -S-S- tels que **(I-S)** ou **(II-S)** et ii) à protéger selon les méthodes classiques ladite fonction thiol de **(I-H)** ou **(II-H)** avec le réactif 7 Y'R pour accéder aux colorants hémicyanine styryles thiols protégés de formule **(I-Y)** ou **(II-Y).** Le composé thiol **(I-H)** ou **(II-H)** peut également être métallé avec un métal alcalin ou alcalino terreux Met* pour conduire au colorant fluorescent thiolate de formule **(I-Mét)** ou **(II-Mét).**

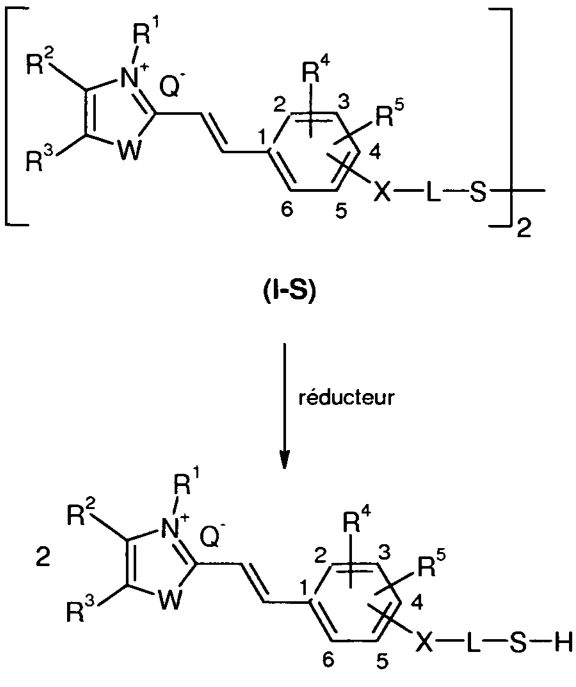


(I-S)

(I-H)

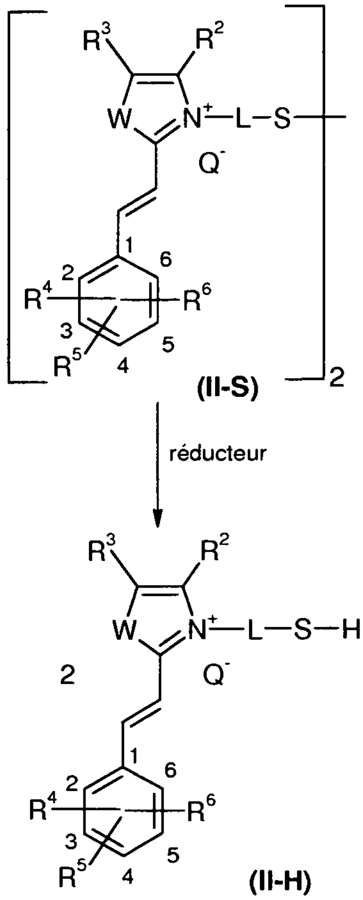


(II-S)

(II-H)

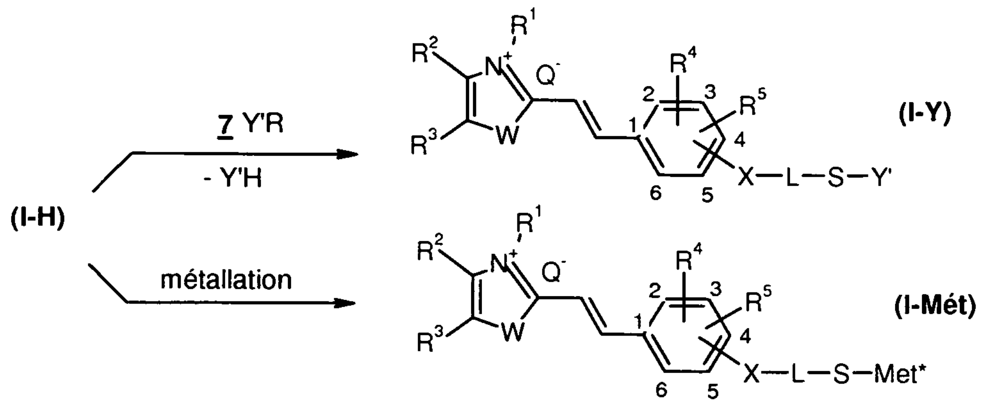


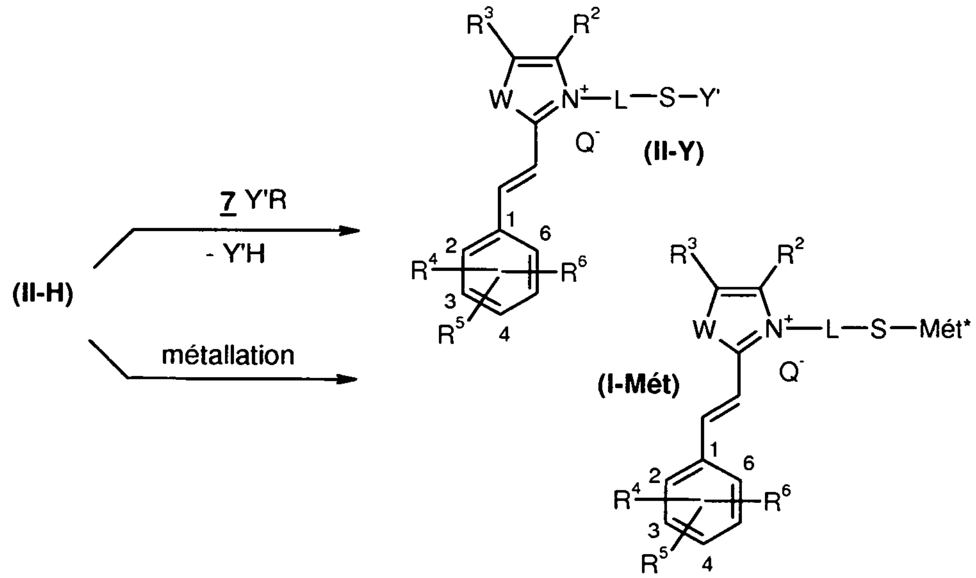


avec Y' représentant un groupement protecteur de fonction thiol ; Met* représentant un métal alcalin ou un métal alcalino térreux, particulièrement le sodium ou le potassium, étant entendu que lorsque le métal est un métal alkalino-terreux 2 chromophores à fonction thiolate-$S^-$ peuvent être associés à 1 $Métal^{2+}$; et avec $R^1$, à $R^6$, W, X, $Q^-$ et L sont tels que définis précédemment ; Y' représente un groupement protecteur de fonction thiol ; et R représente un groupement partant nucléofuge, comme par exemple mésylate, tosylate, triflate ou halogénure.

Selon une autre possibilité, on peut faire réagir un composé thiol protégé **(b)** par un groupement protecteur Y' tel que défini précédemment préparé selon une des procédures décrites dans les ouvrages cités précédemment, ledit composé thiol protégé comprenant au moins une fonction nucléophile avec une quantité suffisante, préférentiellement équimolaire, d'un chromophore **(a)** ou **($a_1$)** hémicyanine styryle, et qui comprend une fonction électrophile pour former une liaison covalente dans un groupement de liaison Σ ,voir ci-dessous la préparation de colorants de formule **(I'-Y)** et **(I'-Y):**

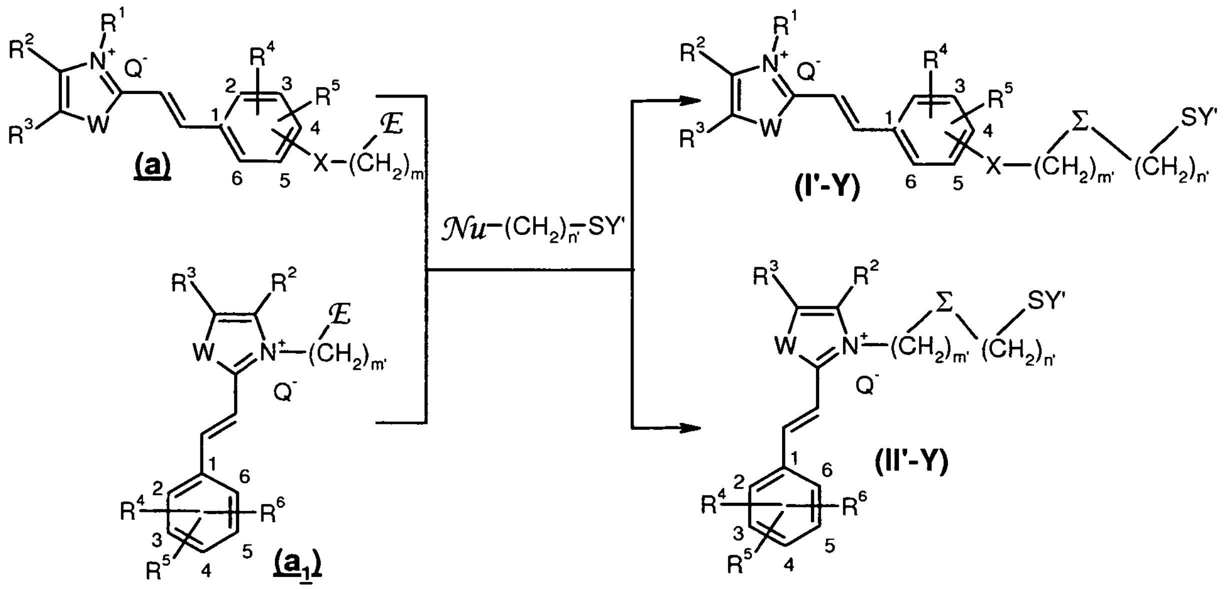


(a)

(a1)

(I'-Y)

(II'-Y)

avec $R^1$ à $R^6$, W, X, $Q^-$, sont tels que définis précédemment ; m' et n' sont des entiers compris entre 1 et 6 avec m'+n' vaut un entier compris entre 2 et 6, *Nu* représentant un groupement nucléophile ; *E* représentant un groupement électrophile ; et Σ représentant la liaison générée après attaque du nucléophile sur l'électrophile.

A titre d'exemple, les liaisons covalente Σ pouvant être générées sont répertoriées dans le tableau ci-dessous à partir de condensation d'électrophiles avec des nucléophiles :

| Electrophiles *E* | Nucléophiles *Nu* | Liaisons covalente Σ |
|---|---|---|
| Esters activés* | Amines | Carboxamides |
| Azotures d'acyles** | Amines | Carboxamides |
| Haloqénures d'acyles | Amines | Carboxamides |
| Haloqénures d'acyles | Alcools | Esters |
| Cyanures d'acyles | Alcools | Esters |
| Cyanures d'acyles | Amines | Carboxamides |
| Halogénures d'alkyles | Amines | Alkylamines |
| Halogénures d'alkyles | Acides carboxyliques | Esters |
| Halogénures d'alkyles | Thiols | Thioesters |
| Halogénures d'alkyles | Alcools | Ethers |
| Acides sulfoniques et leurs sels | Thiols | Thioéthers |
| Acides sulfoniques et leurs sels | Acides carboxyliques | Esters |
| Acides sulfoniques et leurs sels | Alcools | Ethers |
| Anhydrides | Alcools | Esters |
| Anhydrides | Amines | Carboxamides |
| Halogénures d'aryles | Thiols | Thioéthers |
| Halogénures d'aryles | Amines | Arylamines |
| Aziridines | Thiols | Thioéthers |
| Acides carboxyliques | Amines | Carboxamides |
| Acides carboxyliques | Alcools | Esters |
| Carbodiimides | Acides carboxyliques | N-acylurées ou anhydrides |
| Diazoalcanes | Acides carboxyliques | Esters |
| Epoxides | Thiols | Thioéthers |
| Haloacétamides | Thiols | Thioéthers |
| Esters imidiques | Amines | Amidines |
| Isocyanates | Amines | Urées |
| Isocyanates | Alcools | Uréthanes |

(suite)

| Electrophiles *E* | Nucléophiles *Nu* | Liaisons covalente Σ |
|---|---|---|
| Isothiocyanates | Amines | Thiourées |
| Maléimides | Thiols | Thioéthers |
| Esters sulfoniques | Amines | Alkylamines |
| Esters sulfoniques | Thiols | Thioéthers |
| Esters sulfoniques | Acides carboxyliques | Esters |
| Esters sulfoniques | Alcools | Ethers |
| Halogénures de sulfonyle | Amines | Sulfonamides |

**les esters activées de formule générale -CO-Part avec Part représentant un groupement partant tel que oxysuccinimidyle, oxybenzotriazolyle, aryloxy éventuellement substitué ;*

*** les azotures d'acyles peuvent se réarranger pour donner les isocyanates.*

Une variante à ce procédé est d'utiliser un chromophore hémicyanine styryle possédant une fonction acrylate électrophile (-OCO-C=C-) sur laquelle on effectue une réaction d'addition qui génèrera une liaison Σ.

On pourra également utiliser un réactif thiol (α) : Y'-SH comprenant un groupement Y' tel que défini précédemment dont la fonction nucléophile SH peut réagir sur l'atome de carbone du radical L en alpha de l'atome d'halogène porté par un chromophore hémicyanine styryle de **(a')** ou **(a'$_1$)**, pour conduire au colorant thiol protégé de formule **(I-Y)** ou **(II-Y)** tels que définis précédemment :

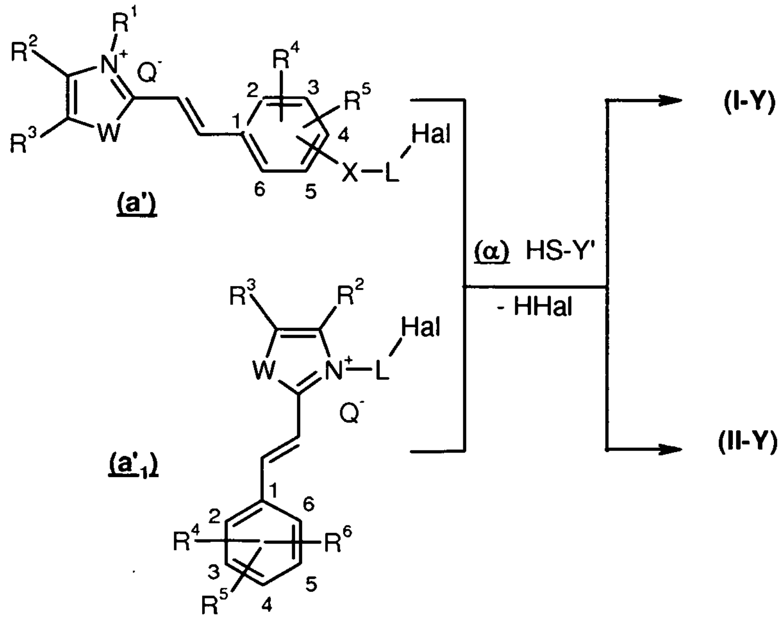


avec $R^1$ à $R^6$, W, X, L, $Q^-$, **(I-Y)** et **(II-Y)** sont tels que définis précédemment, et Hal représentant un atome d'halogène nucléofuge tel que le brome, l'iode ou le chlore.

Plus particulièrement on pourra substituer un groupement partant nucléofuge par un groupement dérivé de thiourées (S=C(NRR)NRR) ou de la thiourée pour générer les isothiouroniums. Par exemple à partir de chromophores **(a')** ou **(a'$_1$)** tels que définis précédemment si le groupement thiourée est une imidazoline thione (β) (ou sa forme tautomère thiodéhydroimidazolium), pour conduire au colorant S-protégé par un groupement imidazolinium **(I"-Y)** ou **(II"-Y):**

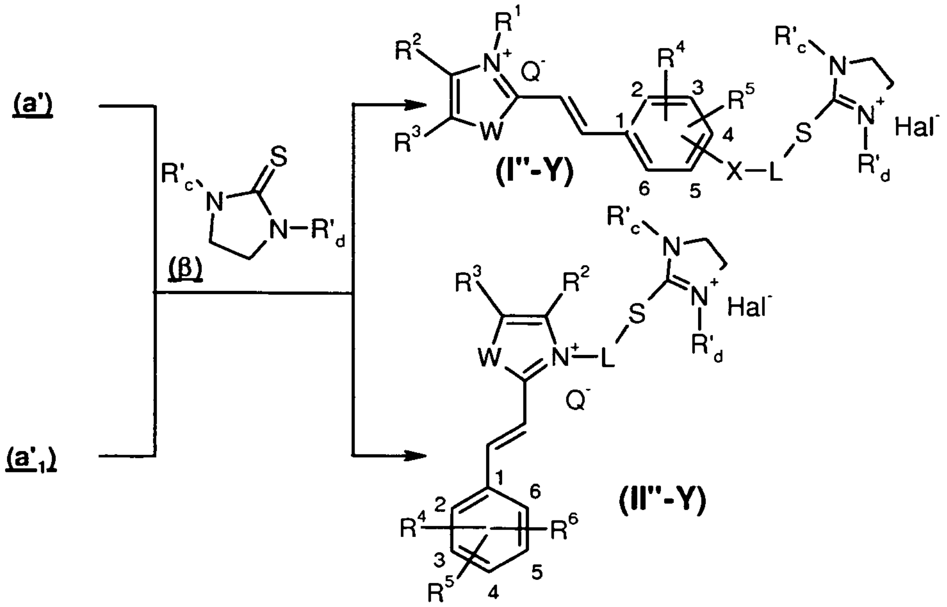


avec **(a')**, **(a'$_1$)**, R'$_c$, R'$_d$, $R^1$ à $R^6$, W, X, L, $Q^-$, Hal et $An^-$ sont tels que définis précédemment.

Une autre variante peut permettre d'accéder au composé **(I"-Y')** ou **(II"-Y')** à partir de la d'un dérivé de thiourée cyclique de type thioimidazole **(b')**, suivi de l'alkylation du dit imidazole à l'aide de R'$_d$-Gp avec Gp groupe partant tel que chlorure, bromure, tosylate, mésylate :

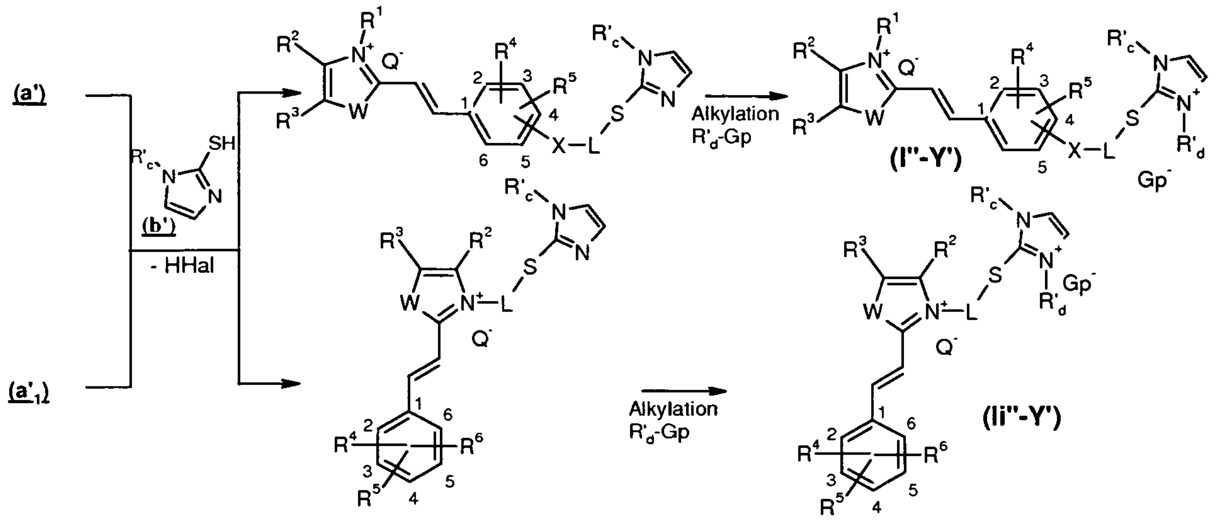


avec R'$_c$, R'$_d$, $R^1$ à $R^6$, X, L, **(a')**, **(a'$_1$)**, $Q^-$, Hal, Gp, sont tels que définis précédemment.

Une variante est d'utiliser à la place de l'halogénure comprenant le chromophore fluorescent **(a')** ou **(a'$_1$)** un chromophore comprenant un autre type de nucléofuge tel que le tosylate, mésylate.

Conformément à une autre possibilité, certains colorants thiols protégés **(I'-Y)** ou **(II'-Y)** peuvent être obtenus en faisant réagir un composé thiol protégé, avec un composé portant deux fonctions acide carboxylique activées selon les méthodes classiques (par exemple réaction avec un carbodiimide ou avec le chlorure de thionyle). Le produit résultant **(d)** est ensuite mis à réagir avec un chromophore **(c)** ou **(c$_1$)** hémicyanine styryle, et porteur d'une fonction nucléophile, par exemple de type amine primaire ou secondaire, ou de type alcool aliphatique.

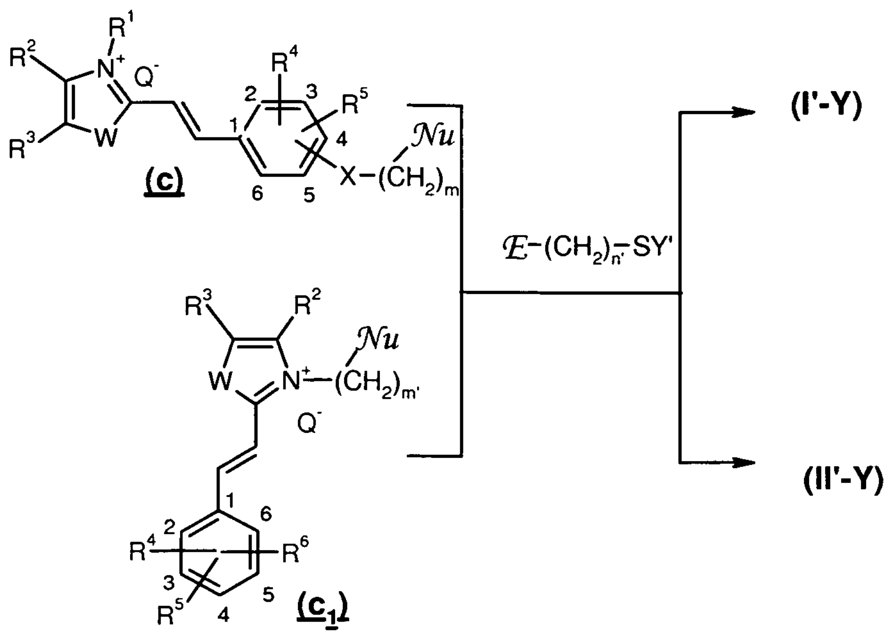


avec $R^1$ à $R^6$, m', n', W, X, $Q^-$, *E*, *Nu*, **(I'-Y)** et **(II'-Y)** tels que définis précédemment.

Une autre variante est d'utiliser un dérivé de thiolactone à partir de chromophores nucléophiles particuliers <u>**(c')**</u> et <u>**(c'$_1$)**</u> pour conduire aux dérivés **(I'-H)** ou **(II'-H)** comprenant un linker L interrompu par une fonction amide tel que représenté par le schéma ci-dessous:

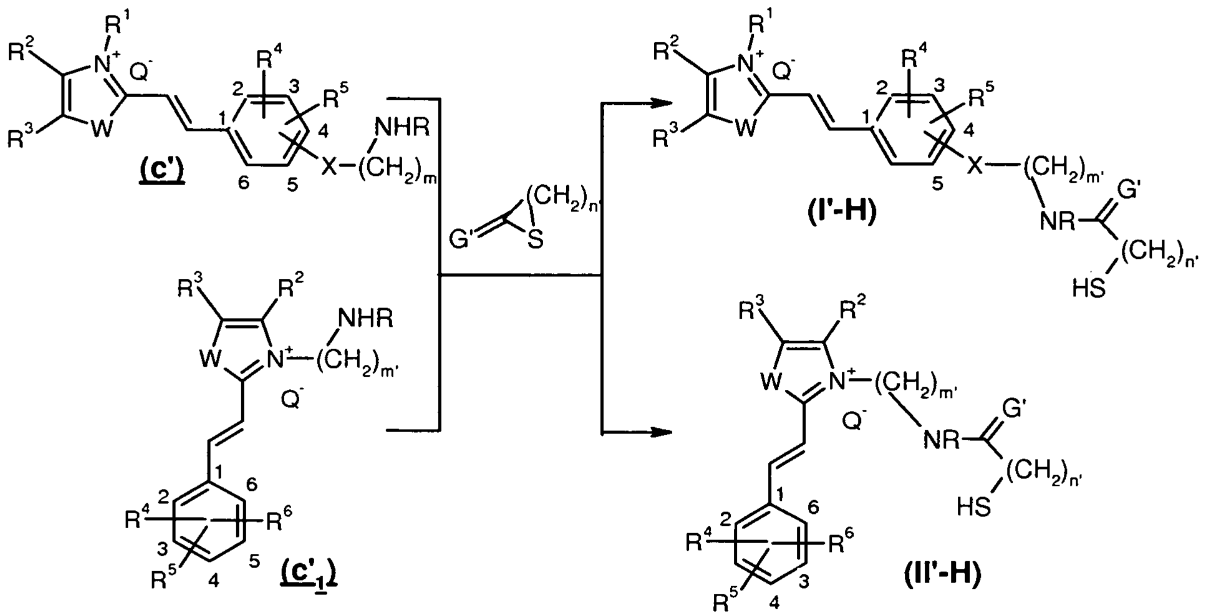


Avec $R^1$ à $R^6$, R, W, X, $Q^-$, n' et m', tels que définis précédemment, G' représentant un atome d'oxygène, de soufre ou un groupement NR' avec R' représentant un atome d'hydrogène ou un radical alkyle, et R représentant un atome d'hydrogène, un radical alkyle en $C_1$-$C_4$, hydroxyalkyle en $C_1$-$C_4$ ou un aryl($C_1$-$C_4$)alkyle. Le dérivé thiolactone est préférentiellement choisi avec n'=3 et G' représente un atome d'oxygène.

Les dérivés **(I'-H)** ou **(II'-H)** à fonction SH libre peuvent ensuite être protégés ou métallés comme vu précédemment.

Conformément à une autre possibilité, les colorants thiols protégés de formule **(I'''-Y)** et **(II'''-Y)** peuvent être obtenus par réaction d'un composé <u>**(d')**</u> comprenant un groupement thiol protégé par un groupement Y' et un groupement groupement partant *Gp* nucléofuge, comme par exemple mésylate, tosylate, triflate ou halogénure avec un chromophore <u>**(c')**</u> ou <u>**(c")**</u> hémicyanine styryle.

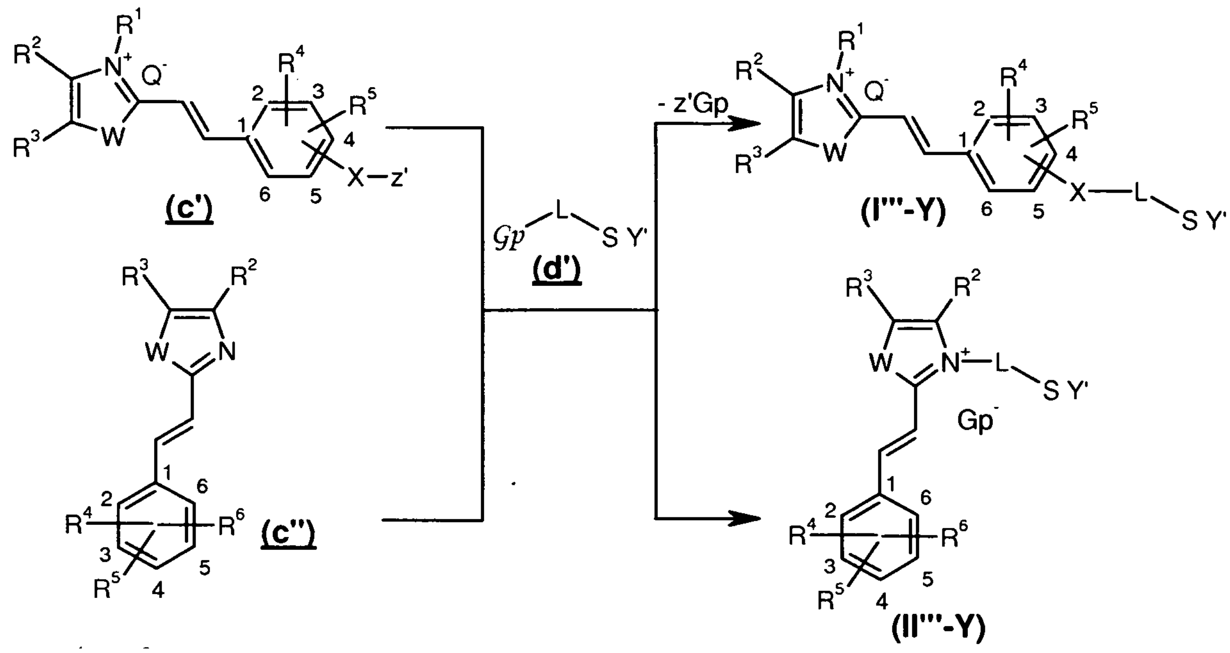


Avec $R^1$ à $R^6$, p', W, X, L et $Q^-$ sont tels que définis précédemment, et z' représente un atome d'hydrogène ou un groupe activant la nucléophilie de X.

Dans la formule c', Z', peut également représenter un groupement amine tertiaire ou un groupement hétérocycle azoté, associé à X, apte à substituer le groupement partant Gp. Auquel cas le composé (I'''-Y) comportera comme contre-ion additionnel à $Q^-$ le groupement Gp-.

A titre d'exemple de composés contenant un groupement thiol protégé **(I'''-Y)** ou **(II'''-Y),** contient un groupement partant nucléofuge R, comme par exemple mésylate, tosylate, ou triflate qui peut subir l'attaque nucléophile de l'amine du chromophore hémicyanine styryle <u>**(c')**</u> ou <u>**(c'')**</u>, comme suit:

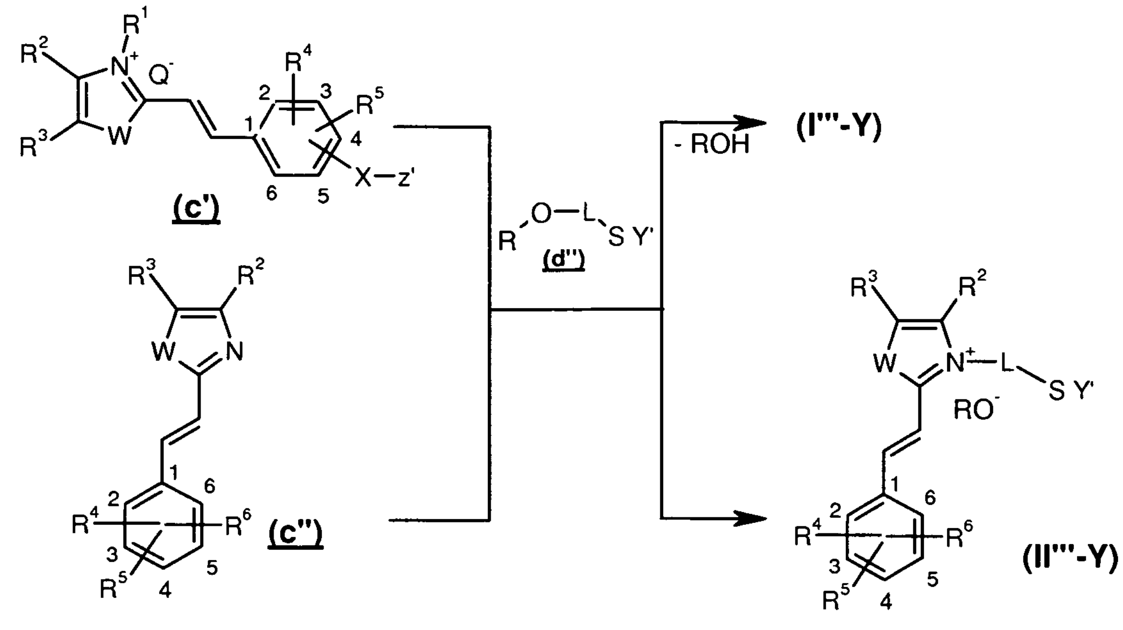


On pourra se référer à l'ouvrage Advanced Organic Chemistry, « Reactions, Mechanisms and Structures », J. March, 4ème Ed, John Willey & Sons, 1992 ou T. W. Greene « Protective Groups in Organic Synthesis », pour avoir plus de détails sur les conditions opératoires mises en oeuvre pour les procédés mentionnés ci-dessus.

Les colorants hémicyanines styryles thiols formés peuvent être transformés en colorants fluorescents thiols protégés -S Y' par la protection du thiol -SH en utilisant les groupements protecteurs classiques. Les colorants fluorescents thiols sont métallés en utilisant également les méthodes classiques connues par l'homme du métier telles que

celles décrites dans Advanced Organic Chemistry, « Reactions, Mechanisms and Structures », J. March, 4ème Ed, John Willey & Sons, NY, 1992.

Les colorants hémicyanines styryles thiols protégés peuvent êtres déprotégés par voies classiques telles que celles décrites dans les ouvrages « Protective Groups in Organic Synthesis », T. W. Greene, John Willey & Sons ed., NY, 1981 ; « Protecting Groups », P. Kocienski, Thieme, 3ème ed., 2005.

Les réactifs de départs sont commerciaux ou accessibles par les méthodes classiques connues par l'homme du métier.

Les colorants disulfures fluorescents de formule (I) et (II) peuvent être synthétisés par oxydation des colorants thiols fluorescents.

Nous pouvons illustrer cette méthode par la méthode consistant à générer des colorants hémicyanines styryles disulfures de formule **(I-S)** ou **(II-S)** par oxydation d'un colorant hémicyanine styryle thiol tels que **(I-H)** ou **(II-H).** L'oxydation peut se faire avec un agent oxydant éventuellement associé à un agent alcalin. On pourra utiliser n'importe quel type d'agent oxydant classique dans le domaine. Ainsi, il peut être choisi parmi le peroxyde d'hydrogène, le peroxyde d'urée, les bromates de métaux alcalins, les persels tels que les perborates et les persulfates, ainsi que les enzymes parmi lesquelles on peut citer les peroxydases, les oxydo-réductases à 2 électrons tels que les uricases et les oxygénases à 4 électrons comme les laccases. L'utilisation du peroxyde d'hydrogène est particulièrement préférée. L'agent alcalin étant par exemple choisi parmi l'ammoniaque, les amines et particulièrement l'éthanolamine, les sels de carbonates ou d'hydrogénocarbonate.

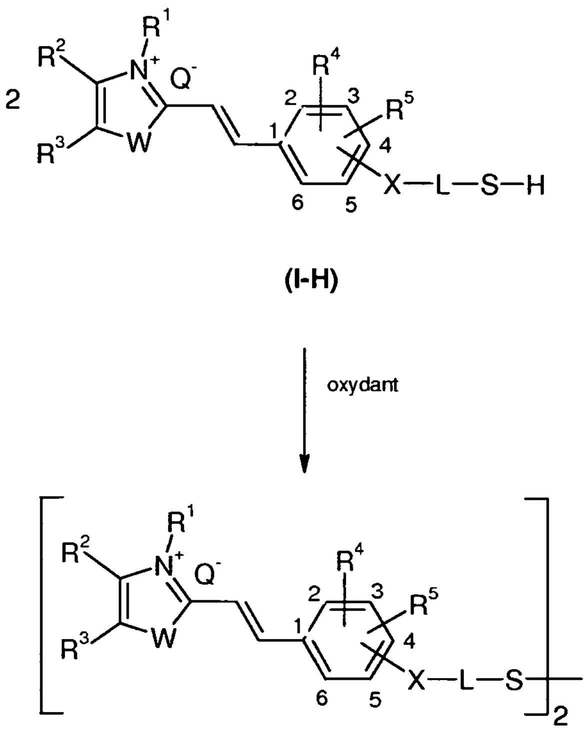


(I-H)

(I-S)

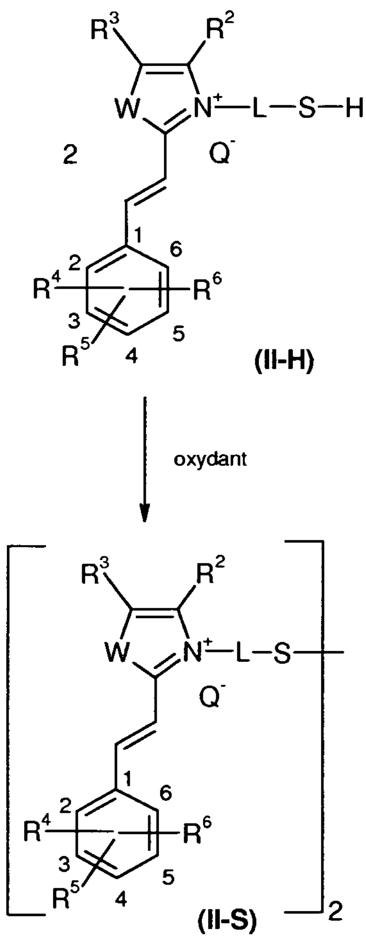


(II-H)

(II-S)

Selon une autre possibilité, on peut faire réagir un composé disulfure **(b1),** ledit composé disulfure comprenant deux fonctions nucléophiles avec une quantité suffisante, préférentiellement deux équivalents, d'un chromophore **(a)** ou **$(a_1)$** hémicyanine styryle, et qui comprend une fonction électrophile pour former un groupement de liaison Σ; étant entendu que $(CH_2)_m$-Σ-$(CH_2)_n$ représentent un sous-ensemble de L suivant la définition donnée pour les formules I et II.voir ci-dessous la préparation de colorants de formule **(I-S)** et **(II-S):**

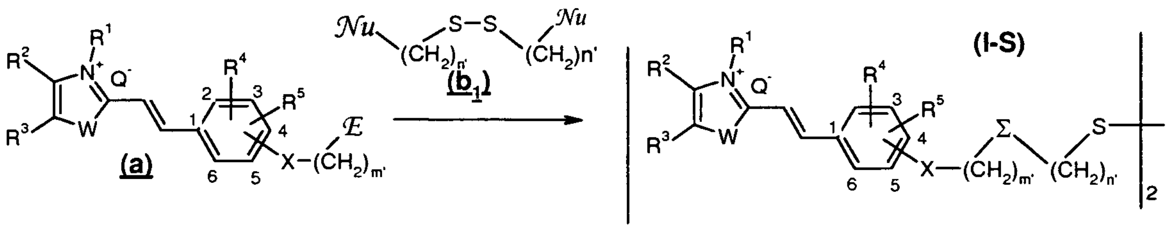


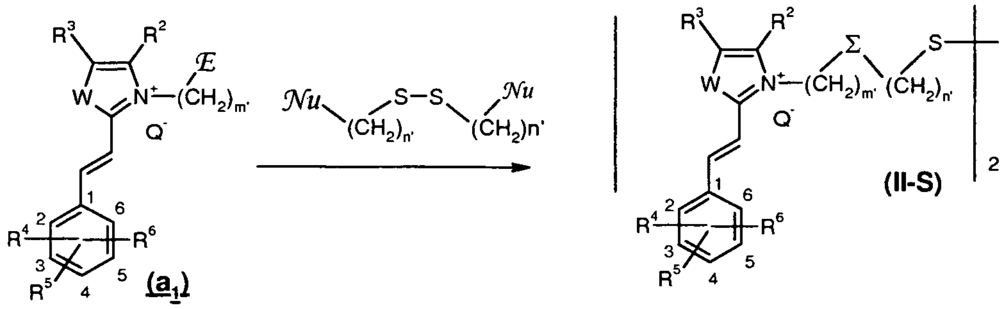


avec $R^1$ à $R^6$, m', n', W, X, $Q^-$, Σ, *E* et *Nu* sont tels que définis précédemment ;

Une variante à ce procédé est d'utiliser un chromophore hémicyanine styryle possédant une fonction acrylate électrophile (-OCO-C=C-) sur laquelle on effectue une réaction d'addition qui génèrera un groupement de liaison Σ.

Selon une autre possibilité, on peut faire réagir un composé disulfure **(b2),** ledit composé disulfure comprenant deux fonctions électrophile avec une quantité suffisante, préférentiellement deux équivalents, d'un chromophore **(c2)** ou **(c3)** hémicyanine styryle, et qui comprend une fonction nucléophiles pour former un groupement de liaison Σ; voir ci-dessous la préparation de colorants de formule **(I-S)** et **(II-S):**

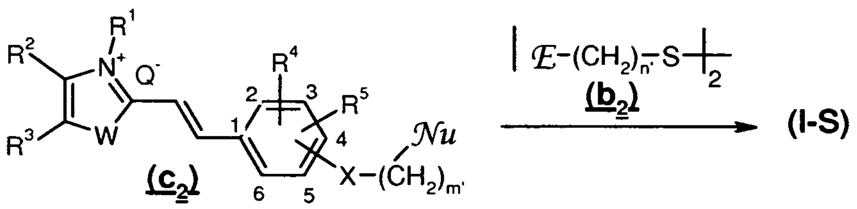


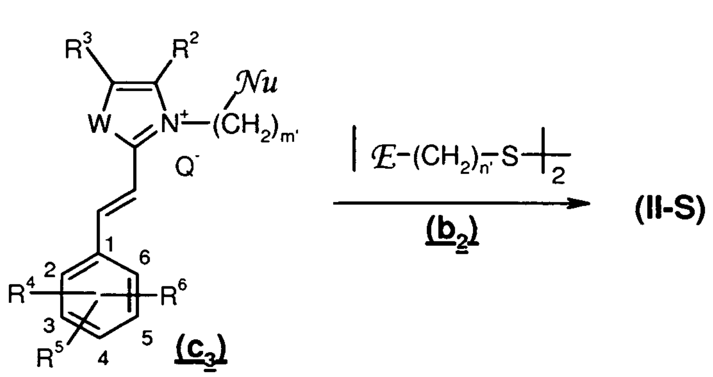


avec $R^1$ à $R^6$, m', n', W, X, $Q^-$, *E*, *Nu*, **(I'-Y)** et **(II'-Y)** tels que définis précédemment.

Conformément à une autre possibilité, les colorants disulfures de formule **(I-S)** et **(II-S)** peuvent être obtenus par réaction d'un composé **(d")** comprenant un groupement disulfure et deux groupements groupement partant *Gp* nucléofuges, comme par exemple mésylate, tosylate, triflate ou halogénure avec un chromophore **(c4)** ou **(c5)** hémicyanine styryle.

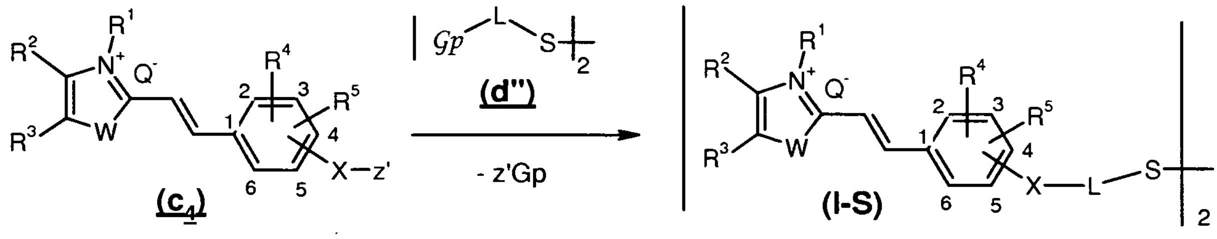


(c4) (d") (I-S)

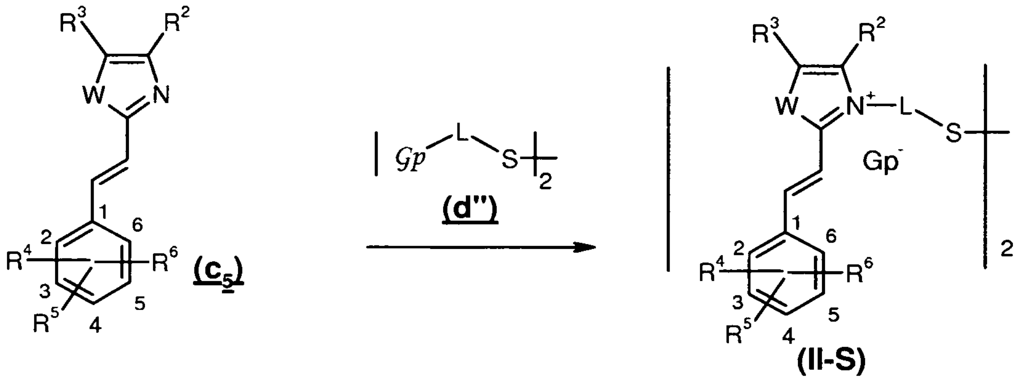

(c5) (d") (II-S)

Avec $R^1$ à $R^6$, p', W, X, L, z' et $Q^-$ sont tels que définis précédemment.

Conformément à une autre possibilité, les colorants disulfures de formule **(I)** ou **(II)** selon l'invention peuvent être obtenus par réaction d'un composé comprenant un groupement disulfure et un groupement électrophile **(f)** avec un composé heterocyclique comprenant un groupement nucléophile. A titre d'exemple, on pourra condenser un aldéhyde ou un thioaldéhyde lorsque G' représente un atome d'oxygène ou un soufre avec un « méthylène activé » tel que le dérivé hétéroaryle cationique **(e)** ou **(e')** pour générer une liaison éthylène >C=C<. Cette réaction est communément appelée condensation de « Knoevenagel » :

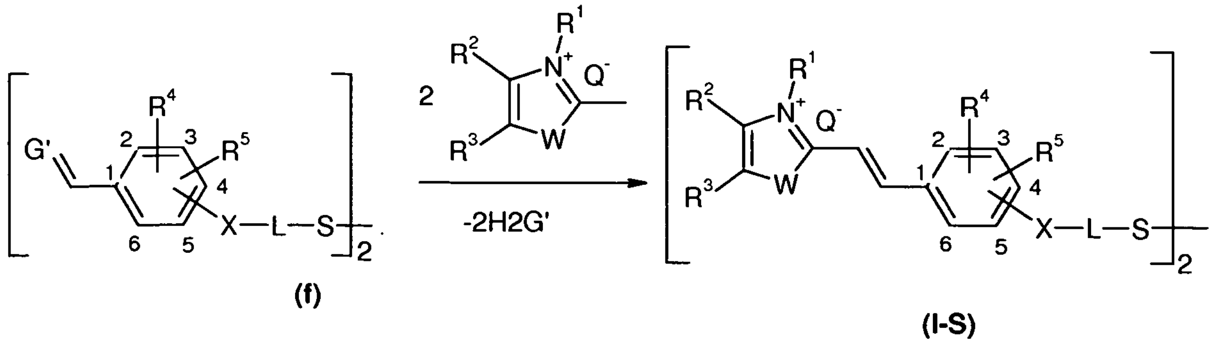


(f) (I-S)

Avec $R^1$ à $R^6$, W, X, L et $Q^-$ sont tels que définis précédemment. G' représente un atome d'oxygène ou un soufre.

On pourra se référer à l'ouvrage Advanced Organic Chemistry, « Reactions, Mechanisms and Structures », J. March, 4ème Ed, John Willey & Sons, 1992 pour avoir plus de détails sur les conditions opératoires mises en oeuvre pour les procédés mentionnés ci-dessus.

Les réactifs de départs sont commerciaux ou accessibles par les méthodes classiques connues par l'homme du métier.

A titre d'exemple on peut citer la synthétise du colorant (I), et de façon subséquente les réactifs **(I')** ou **(I")**, à partir de 2 equivalents de dérivé **(g)** et un équivalent de réactif disulfure **2** comprenant deux aryle ou hétéroaryle B à fonction électrophile tel que aldéhyde, thioaldéhyde ou un réactif disulfure à méthylène activé **3** comprenant avec deux equivalents de réactif électrophile dérivé d'aryle ou hétéroaryle (g'), pour conduire par double condensation de Knoevenagel aux dérivés styryles disulfures **(I)**.

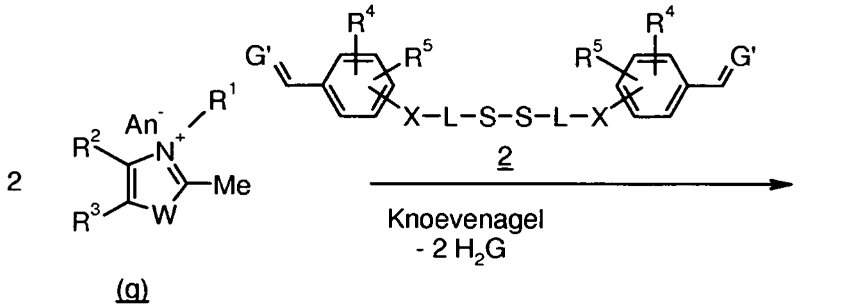


(g) (I)

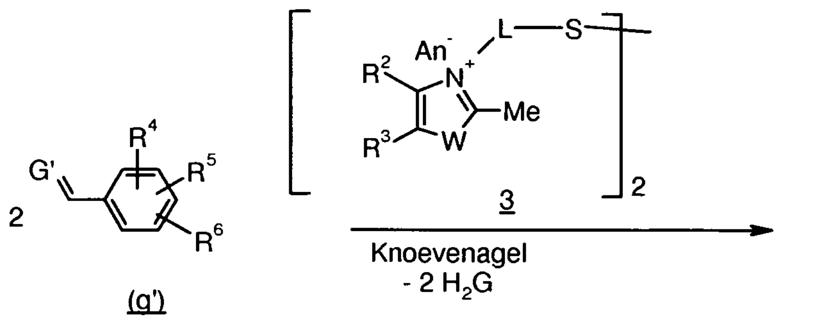


(g') (II)

avec $R^1$ à $R^6$, $An^-$, L, X, G' et **(I)** **(II)** tels que définis précédemment.

La composition de l'invention contient au moins un colorant fluorescent hémicyanine styryle disulfure, thiol ou thiol protégé de formule **(I)** ou **(II).** Outre la présence d'au moins un colorant fluorescent de formule **(I)** ou **(II),** la composition de l'invention peut également contenir au moins un agent réducteur.

Cet agent réducteur peut être choisi parmi les thiols par exemple la cystéine, l'homocystéine, l'acide thiolactique, les sels de ces thiols, les phosphines, le bisulfite, les sulfites, l'acide thioglycolique, ainsi que ses esters, notamment le monothioglycolate de glycérol, et le thioglycérol. Cet agent réducteur peut aussi être choisi parmi les borohydrures et leurs dérivés, comme par exemple les sels du borohydrure, du cyanoborohydrure, du triacétoxyborohydrure, du triméthoxyborohydrure : sels de sodium, lithium, potassium, calcium, ammoniums quaternaires (tétraméthylammonium, tétraéthylammonium, tétra n-butylammonium, benzyltriéthylammonium); le catéchol-borane.

La composition tinctoriale utile dans l'invention contient en général une quantité de colorant de formule **(I)** ou **(II)** comprise entre 0,001 et 50% par rapport au poids total de la composition. De préférence, cette quantité est comprise entre 0,005 et 20% en poids et encore plus préférentiellement entre 0,01 et 5% en poids par rapport au poids total de la composition.

La composition tinctoriale peut en outre contenir des colorants directs additionnels. Ces colorants directs sont par exemple choisis parmi les colorants directs nitrés benzéniques neutres, acides ou cationiques, les colorants directs azoïques neutres, acides ou cationiques, les colorants tétraazapentaméthiniques, les colorants quinoniques et en particulier anthraquinoniques neutres, acides ou cationiques, les colorants directs aziniques, les colorants directs triarylméthaniques, les colorants directs indoaminiques et les colorants directs naturels.

Parmi les colorants directs naturels, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

La composition tinctoriale peut contenir une ou plusieurs bases d'oxydation et/ou un ou plusieurs coupleurs conventionnellement utilisés pour la teinture de fibres kératiniques.

Parmi les bases d'oxydation, on peut citer les para-phénylènediamines, les bis-phénylalkylènediamines, les para-aminophénols, les bis-para-aminophénols, les ortho-aminophénols, les bases hétérocycliques et leurs sels d'addition

Parmi ces coupleurs, on peut notamment citer les méta-phénylènediamines, les méta-aminophénols, les méta-diphénols, les coupleurs naphtaléniques, les coupleurs hétérocycliques et leur sels d'addition.

Le ou les coupleurs sont chacun généralement présents en quantité comprise entre 0,001 et 10% en poids du poids total de la composition tinctoriale, de préférence entre 0,005 et 6%.

La ou les bases d'oxydation présentes dans la composition tinctoriale sont en général présentes chacune en quantité comprise entre 0,001 à 10% en poids du poids total de la composition tinctoriale, de préférence entre 0,005 et

6% en poids.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que les hydroxydes de métal alcalin comme la soude, la potasse, l'ammoniaque, les amines ou les alcanolamines.

Le milieu approprié pour la teinture, appelé aussi support de teinture, est un milieu cosmétique contenant généralement de l'eau ou un mélange d'eau et d'au moins un solvant organique. A titre de solvant organique, on peut par exemple citer les alcanols inférieurs en $C_1$-$C_4$, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants lorsqu'ils sont présents sont, de préférence présents dans des proportions de préférence comprises entre 1 et 40% en poids environ par rapport au poids total de la composition tinctoriale, et encore plus préférentiellement entre 5 et 30% en poids environ.

La composition tinctoriale peut également renfermer divers adjuvants utilisés classiquement dans les compositions pour la teinture des cheveux, tels que des agents tensioactifs anioniques, cationiques, non ioniques, amphotères, zwittérioniques ou leurs mélanges, des polymères anioniques, cationiques, non ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non tels que les silicones aminés, des agents filmogènes, des céramides, des agents conservateurs, des agents opacifiants, des polymères conducteurs.

Les adjuvants ci dessus sont en général présents en quantité comprise pour chacun d'eux entre 0,01 et 20% en poids par rapport au poids de la composition.

Bien entendu, l'homme de l'art veillera à choisir ce ou ces éventuels composés complémentaires de manière telle que les propriétés avantageuses attachées intrinsèquement à la composition de teinture conforme à l'invention ne soient pas, ou substantiellement pas, altérées par la ou les adjonctions envisagées.

Le pH de la composition tinctoriale est généralement compris entre 3 et 14 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (γ) suivante :

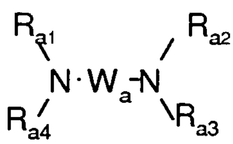

(γ)

dans laquelle $W_a$ est un reste propylène éventuellement substitué par un groupement hydroxy ou un radical alkyle en $C_1$-$C_4$ ; $R_{a1}$, $R_{a2}$, $R_{a3}$ et $R_{a4}$, identiques ou différents, représentent un atome d'hydrogène, un radical alkyle en $C_1$-$C_4$ ou hydroxyalkyle en $C_1$-$C_4$.

La composition tinctoriale peut se présenter sous des formes diverses, telles que sous forme de liquide, de crème, de gel, ou sous toute autre forme appropriée pour réaliser une teinture des fibres kératiniques, et notamment des cheveux.

Le procédé de coloration de matières kératiniques notamment foncés selon l'invention, consiste à appliquer sur les matières kératiniques, une composition tinctoriale, comprenant dans un milieu cosmétique approprié, au moins un colorant fluorescent hémicyanine styryle disulfure ou thiol, choisi parmi les colorants de formules **(I)** et **(II).**

Selon un mode de réalisation particulier dans le procédé de l'invention l'agent réducteur peut être appliqué en pré-traitement avant l'application de la composition contenant au moins un colorant fluorescent hémicyanine styryle de formule **(I)** ou **(II).**

Ce prétraitement peut être de courte durée, notamment de 1 seconde à 30 minutes, de préférence de 1 minute

à 15 minutes avec un agent réducteur tel que cité précédemment.

Selon un autre procédé, la composition comprenant au moins un colorant fluorescent hémicyanine styryle de formule **(I)** ou **(II)** et contient également au moins un agent réducteur tel que défini précédemment. Cette composition est alors appliquée aux cheveux.

Lorsque le colorant fluorescent hémicyanine styryle thiol de formule **(I)** ou **(II)** pour lesquels m et n valent 1 comprend un groupement Y protecteur de la fonction thiol, le procédé de l'invention peut être précédé d'une étape de déprotection visant à restituer *in-situ* la fonction SH.

A titre d'exemple il est possible de déprotéger la fonction S-Y des colorants de l'invention avec Y groupement protecteur en ajustant le pH comme suit :

| **Y : groupement protecteur** | **déprotection** |
|---|---|
| alkylcarbonyle, | pH>9 |
| arylcarbonyle, | pH>9 |
| alkoxycarbonyle, | pH>9 |
| aryloxycarbonyle, | pH>9 |
| arylalkoxycarbonyle | pH>9 |
| (di)(alkyl)aminocarbonyle, | pH>9 |
| (alkyl)arylaminocarbonyle | pH>9 |
| aryle éventuellement substitué tel que le phényle, | pH>9 |
| hétéroaryle monocyclique à 5, 6 ou 7 chaînons tel que l'oxazolium ; | pH>9 |
| hétéroaryle bicyclique à 8 à 11 chaînons tels que benzoimidazolium, ou benzoxazolium | pH>9 |

L'étape de déprotection peut également être réalisée au cours d'une étape de prétraitement des cheveux comme par exemple, le prétraitement réducteur des cheveux.

Selon une variante, l'agent réducteur est ajouté à la composition tinctoriale contenant au moins un colorant fluorescent hémicyanine styryle de formule **(I)** ou **(II)** au moment de l'emploi.

Selon un autre procédé, la composition comprenant au moins un colorant fluorescent hémicyanine styryle de formule **(I)** ou **(II)** contient également au moins un agent réducteur tel que défini précédemment. Cette composition est alors appliquée aux cheveux.

Selon une autre variante, l'agent réducteur est appliqué en post-traitement, après l'application de la composition contenant au moins un colorant fluorescent hémicyanine styryle de formule **(I)** ou **(II).** La durée du post traitement avec l'agent réducteur peut être courte, par exemple de 1 seconde à 30 minutes de préférence de 1 minute à 15 minutes, avec un agent réducteur tel que décrit précédemment. Selon un mode de réalisation particulier l'agent réducteur est un agent de type thiol ou borohydrure tel que décrit précédemment.

Un mode de réalisation particulier de l'invention concerne un procédé dans lequel le colorant fluorescent hémicyanine styryle de formule **(I)** ou **(II)** peut être appliqué directement aux cheveux sans réducteurs, exempt de pré ou post-traitement réducteurs.

Un traitement avec un agent oxydant peut éventuellement être associé. On pourra utiliser n'importe quel type d'agent oxydant classique dans le domaine. Ainsi, il peut être choisi parmi le peroxyde d'hydrogène, le peroxyde d'urée, les bromates de métaux alcalins, les persels tels que les perborates et les persulfates, ainsi que les enzymes parmi lesquelles on peut citer les peroxydases, les oxydo-réductases à 2 électrons tels que les uricases et les oxygénases à 4 électrons comme les laccases. L'utilisation du peroxyde d'hydrogène est particulièrement préférée. La durée d'un tel traitement est comprise entre 1 seconde et 40 minutes, de préférence entre 15 secondes et 15 minutes.

L'application de la composition tinctoriale selon l'invention est généralement effectuée à température ambiante. Elle peut cependant être réalisée à des températures variant de 20 à 180 °C.

L'invention a aussi pour objet un dispositif à plusieurs compartiments ou "kit" de teinture dans lequel un premier compartiment renferme une composition tinctoriale comprenant au moins un colorant fluorescent hémicyanine styryle de formule **(I)** ou **(II)** et un deuxième compartiment renferme un agent réducteur capable de réduire les fonctions disulfures des matières kératiniques et/ou du colorant fluorescent hémicyanine styryle disulfure de formule **(I)** ou **(II).**

L'un de ces compartiments peut en outre contenir un ou plusieurs autres colorants de type colorant direct ou colorant d'oxydation.

Elle concerne aussi un dispositif à plusieurs compartiments dans lequel un premier compartiment contient une

composition tinctoriale comprenant au moins un colorant fluorescent hémicyanine styryle de formule **(I)** ou **(II)**; un deuxième compartiment contient un agent réducteur capable de réduire la liaison disulfure des matières kératiniques et/ou du colorant fluorescent hémicyanine styryle disulfure de formule **(I)** ou **(II)**; un troisième compartiment contient un agent oxydant.

Alternativement, le dispositif de teinture contient un premier compartiment renfermant une composition tinctoriale qui comprend au moins un colorant fluorescent hémicyanine styryle thiol protégé de formule **(I)** ou **(II)** avec m et n valant 1, un deuxième compartiment renfermant un agent capable de déprotéger le thiol protégé pour libérer le thiol, et éventuellement un troisième compartiment comprenant un agent oxydant.

Chacun des dispositifs mentionnés ci-dessus peut être équipé d'un moyen permettant de délivrer sur les cheveux le mélange souhaité, par exemple tel que les dispositifs décrits dans le brevet FR2 586 913.

Les exemples qui suivent servent à illustrer l'invention sans toutefois présenter un caractère limitatif. Les colorants des exemples ci-après ont été entièrement caractérisés par les méthodes spectroscopiques et spectrométriques classiques.

## EXEMPLES

### EXEMPLES DE SYNTHESE

**<u>Exemple 1 :</u> Synthèse du di iodure de 2,2'-{disulfanediylbis[éthane-2,1-diyl(méthylimino)-4,1-phénylène éthène-2,1-diyl]}bis(3-éthyl-1,3-benzothiazol-3-ium) [1]**

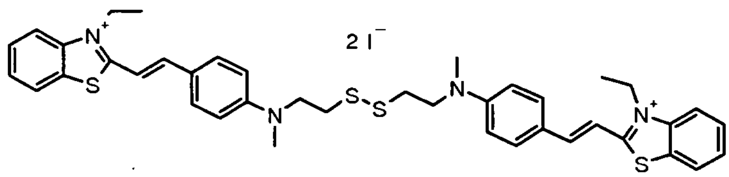

**Schéma de synthèse :**

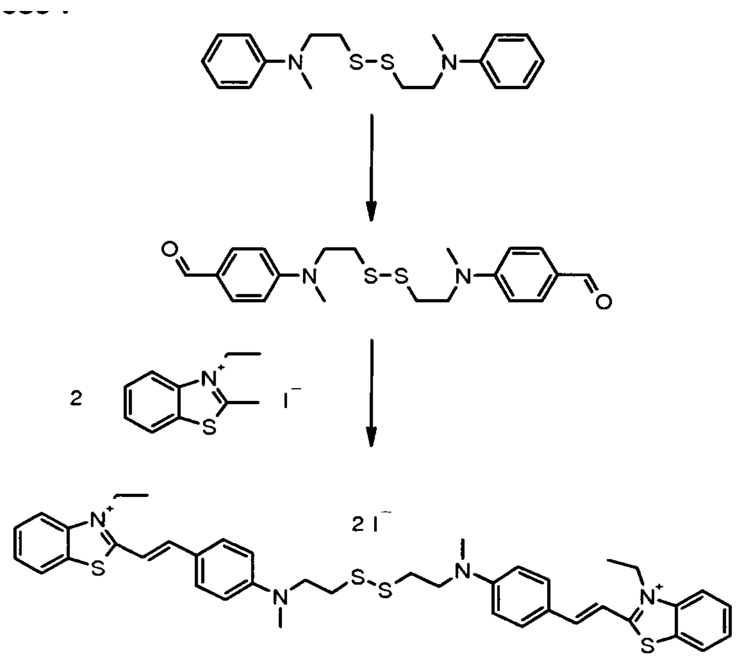

**[1]**

**Mode opératoire :**

**Etape 1 : 4,4'-{disulfanediylbis[éthane-2,1-diyl(méthylimino)]}dibenzaldéhyde** 82,3 g d'oxychlorure de phosphore sont ajoutés à 500 mL de DMF à 0°C. Après 30 min d'agitation à 0°C une solution de 47g de *N,N'*-(disulfanediyldiéthane-2,1-diyl)bis(N-méthylaniline) sont ajoutés goutte à goutte. Le mélange est agité 90 min. à 0°C puis 75 min. à 10°C et 105 min. à 40°C. Il est ensuite versé sur 2,5 L d'eau glacée, 700 mL de soude 5N sont ajoutés. Le précipité jaune obtenu est filtré sur célite, solubilisé dans 200 mL de dichlorométhane et la solution obtenue est lavée par 200 mL de solution aqueuse saturée en chlorure de sodium. Après séchage sur sulfate de magnésium et évaporation du dichlorométhane, le résidu jaune (80g) est purifié par chromatographie sur gel de silice. Après séchage, une poudre jaune clair est recueillie. Les analyses montrent que le produit est conforme à la structure attendue. **Etape 2 : diiodure de 2,2'-{disulfanediylbis[éthane-2,1-diyl(méthylimino)-4,1-phénylèneéthène-2,1-diyl]}bis(3-éthyl-1,3-benzothiazol-3-ium) [1]** 1.9 g d'iodure de N-éthyl benzothiazolium en solution dans 2 mL de dichlorométhane sont ajoutés à 1.2 g de 4,4'-{disulfanediylbis[éthane-2,1-diyl(méthylimino)]}dibenzaldehyde en suspension dans 9 mL d'éthanol à 79°C. Le mélange est maintenu à reflux, sous agitation pendant 72h. Après refroidissement, la suspension violette est filtrée. Après séchage, 1.5 g de poudre noire sont recueillie. Les analyses indiquent que le produit est conforme au composé [1] et pur. RMN $^1$H (400 MHz, MeOH-$d_4$) - 1.42 (t, 6 H), 3.04 (t, 4 H), 3.13 (s, 6 H), 3.82 (t, 4 H), 4.84 (q, 4 H), 6.90 (d, 4 H), 7.64 (d, 2 H), 7.68 (dd, 2 H), 7.78 (dd, 2 H), 7.95 (d, 4 H), 8.08 (d, 2 H), 8.15 (d, 2 H), 8.30 (d, 2 H).

**<u>Exemple 2 :</u> Synthèse du diméthylsulfate de 2-(2-{4-[[2-({2-[{4-[-2-(1,3-diméthyl-1H-benzimidazol-3-ium-2-yl)vinyl]phényl}(méthyl)amino]éthyl}disulfanyl)-éthyl](méthyl)amino]phényl}vinyl)-1,3-diméthyl-1H-3,1-benzimidazol-3-ium) [2]**

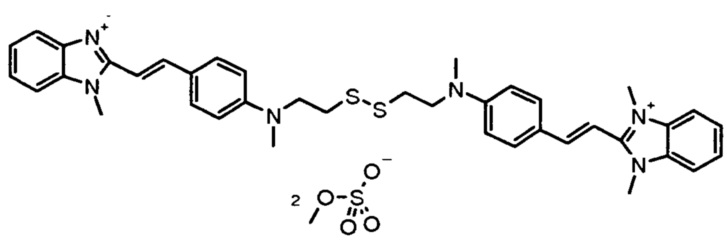

**Schéma de synthèse :**

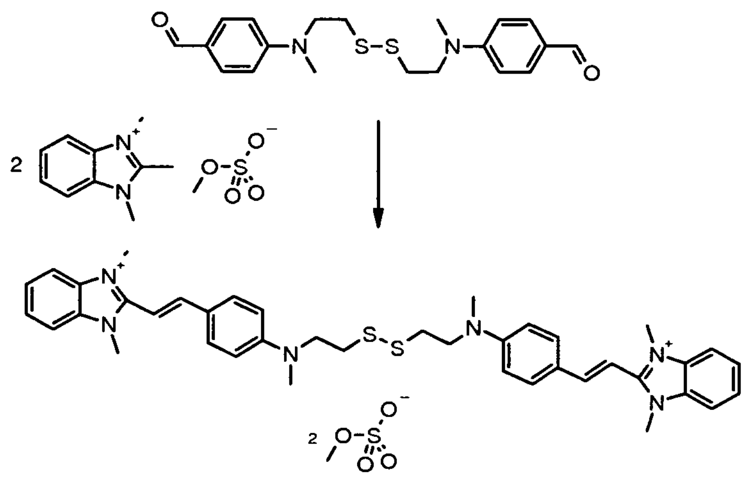

**[2]**

**Mode opératoire :**

**Synthèse du diméthylsulfate 2-(-2-{4-[[2-({2-[{4-[2-(1,3-diméthyl-1H-benzimidazol-3-ium-2-yl)vinyl]phényl}(méthyl)amino]éthyl}disulfanyl)éthyl](méthyl)-amino]phényl}vinyl)-1,3-diméthyl-1H-3,1-benzimidazol-3-ium) [2]** 0.5 g de méthylsulfate de 1,2,3-triméthyl-1H-benzimidazol-3-ium en solution dans 2 mL de dichlorométhane

sont ajoutés à 0.36 g de 4,4'-{disulfanediylbis[éthane-2,1-diyl(méthylimino)]}dibenzaldehyde en suspension dans 8 mL d'éthanol à 79°C et 0.15 mL de pyrrolidine. Le mélange est maintenu à 80°, sous agitation pendant 7h. Après refroidissement, la suspension est versée sur 25 mL d'acétate d'éthyle froid. Le précipité obtenu est filtré, lavé à l'acétate d'éthyle et séché..0.3 g de poudre orange sont recueillis. Les analyses indiquent que le produit est conforme au composé [2].

**<u>Exemple 3 :</u> Synthèse du dibromure de 3,3'-(disulfanediyldiéthane-2,1-diyl)bis(6-chloro-2-{-2-[4-(diméthylamino)-1-naphthyl]vinyl}-1-méthyl-1H-benzimidazol-3-ium) [3]**

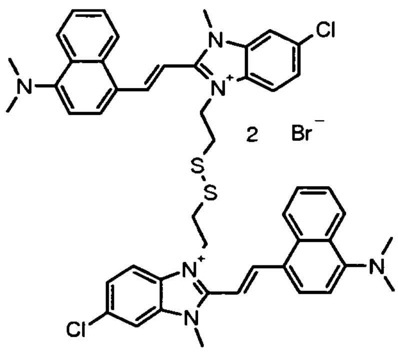

**[3]**

**Schéma de synthèse :**

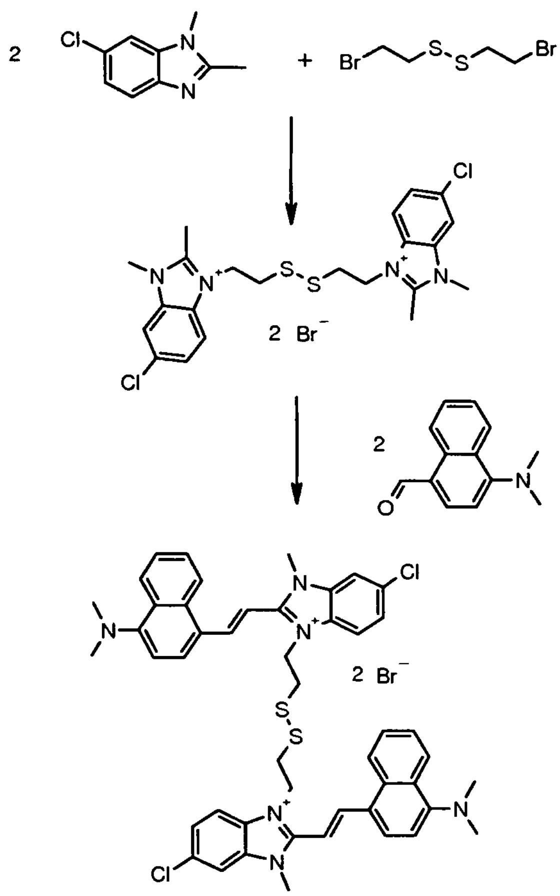

**[3]**

**Mode opératoire :**

**Etape 1 : synthèse du dibromure de 3,3'-(disulfanediyldiethane-2,1-diyl)bis(6-chloro-1,2-diméthyl-1H-benzimidazol-3-ium)** 142 mg de chlloro-1,2-diméthylbenzimidazole, 100 mg de bis(2-bromoéthyl) disulfide et 54 mg d'iodure de sodium et 0.4 mL de propionitrile sont agités à 100°C pendant 17 h. Après refroidissement, filtration et lavages par 5 fois 5 mL d'acétonitrile, 125 mg de poudre grise sont recueillis. Les analyses montrent que le produit est conforme à la structure attendue.

**Etape 2 : synthèse du dibromure de 3,3'-(disulfanediyldiéthane-2,1-diyl)bis(6-chloro-2-{-2-[4-(diméthylamino)-1-naphthyl]vinyl}-1-méthyl-1H-benzimidazol-3-ium)** 600 mg de dibromure de 3,3'-(disulfanediyldiethane-2,1-diyl)bis(6-chloro-1,2-dimethyl-1H-benzimidazol-3-ium, 370 mg de 4-diméthylaminonaphthaldéhyde, 6 mL de méthanol et 42 μL de pipéridine sont agités à température ambiante pendant 24 h. Le mélange obtenu est versé goutte à goutte dans 200 mL d'éther éthylique. Après filtration et séchage, 400 mg de solide orange sont recueillis. Les analyses montrent que le produit est conforme à la structure attendue [3].

**<u>Exemple 4 :</u> Synthèse du dibromure de 3,3'-(disulfanediyldiéthane-2,1-diyl)bis(2-{-2-[4-(diméthylamino)phényl]vinyl}-1-méthyl-1H-benzimidazol-3-ium) [4]**

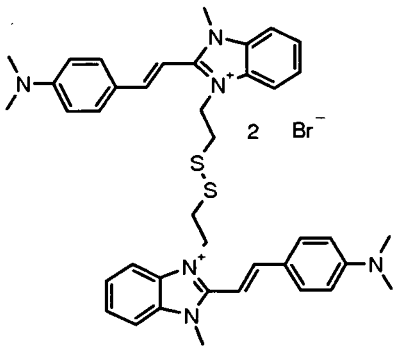

**Schéma de synthèse :**

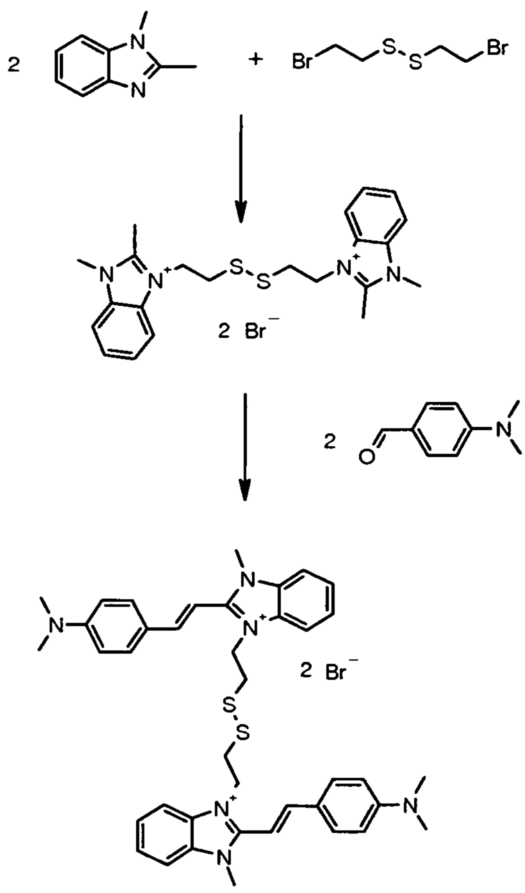

**[4]**

**Mode opératoire :**

**Etape 1 : synthèse du dibromure de 3,3'-(disulfanediyldiéthane-2,1-diyl)bis(1,2-diméthyl-1 H-benzimidazol-3-ium)** 2.4 g de 1,2-diméthylbenzimidazole, 2.1 g de bis(2-bromoéthyl) disulfide et 6 mL de propionitrile sont agités à 100°C dans un réacteur étanche de 20 mL pendant 8 h. Après refroidissement, filtration, lavages par 3 fois 10 mL et séchage sous vide, 2.2g de solide beige sont recueillis. Les analyses montrent que le produit est conforme à la structure attendue.

**Etape 2 : Synthèse du dibromure de 3,3'-(disulfanediyldiéthane-2,1-diyl)bis(2-{-2-[4-(diméthylamino) phényl]vinyl}-1-méthyl-1H-benzimidazol-3-ium)** 1.3 g de dibromure de 3,3'-(disulfanediyldiéthane-2,1-diyl)bis(1,2-diméthyl-1H-benzimidazol-3-ium, 1 g de 4-diméthylaminobenealdéhyde, 26 mL de méthanol et 500 µL de pipéridine sont agités à 60°C pendant 1h h 30. 350 µL de pipéridine sont ajoutés et l'agitation est maintenue pendant 2h 30. Après refroidissement, le précipité orange obtenu est filtré, lavé par 3 fois 5 ml de méthanol à 4°C. Le solide recueilli est malaxé dans de l'éthanol chaud, sous ultra sons. Le mélange est refroidi, filtré et séché. 450 mg de poudre rouge orangée sont obtenues. Les analyses montrent que le produit est pur et conforme à la structure attendue [4].

**<u>Exemple 5 :</u> Synthèse du di iodure de 2,2'-{disulfanediylbis[éthane-2,1-diyl(méthylimino)-4,1-phénylène éthène-2,1-diyl]}bis(3-éthyl-1,3-benzoxazol-3-ium) [5]**

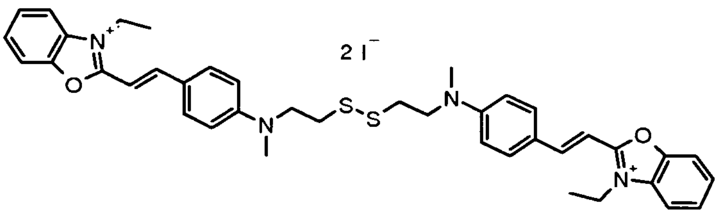

**[5]**

**Schéma de synthèse :**

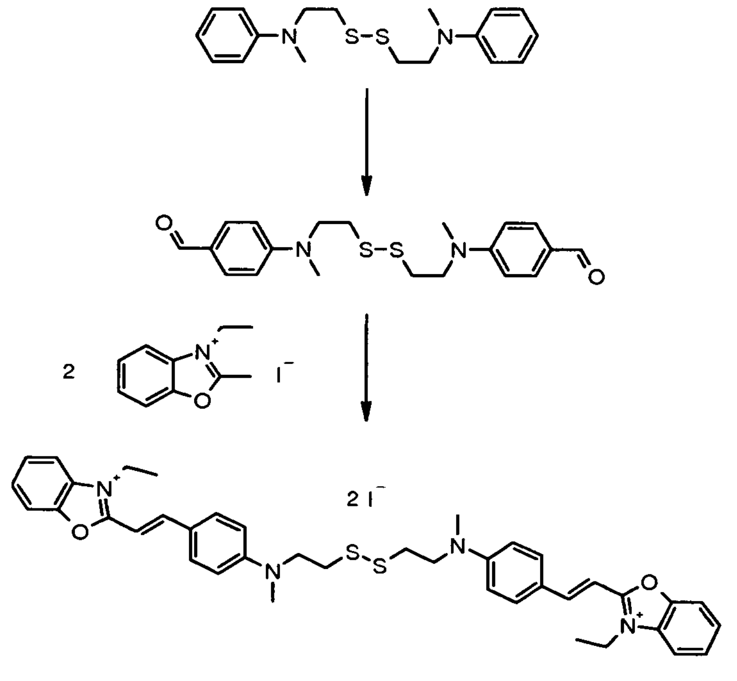

**[5]**

**Mode opératoire :**

6.56 g d'iodure de 3-éthyl-2-méthyl-1,3-benzoxazol-3-ium en solution dans 12 mL de mélange isopropanol / N-méthylpyrrolidinone 60: 40.sont ajoutés à 4.16 g de 4,4'-{disulfanediylbis[éthane-2,1-diyl(méthylimino)]}dibenzaldéhyde dispersés dans 8 mL de mélange isopropanol / N-méthylpyrrolidinone 60 : 40. Le mélange est agité à 80°C pendant 27 h. Après refroidissement, la solution est versée sur 200 mL d'acétate d'éthyle. Le précipité est filtré, lavé trois fois avec 150 mL d'acétate d'éthyle et séché. 6.83g de poudre rouge sont recueillies. Les analyses montrent que le produit est conforme à la structure attendue **[5].**

<u>Exemple 6 :</u> **Synthèse du diméthylsulfate de 2,2'-{disulfanediylbis[éthane-2,1-diyl(méthylimino)-4,1-phénylèneéthène-2,1-diyl]}bis(3-méthyl-1,3-thiazol-3-ium) [6]**

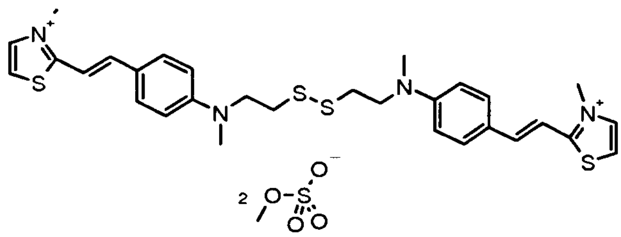

**[6]**

**Schéma de synthèse :**

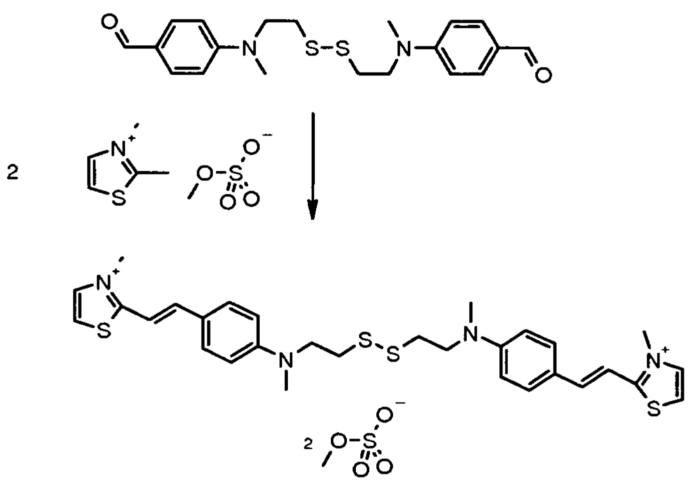

**[6]**

**Mode opératoire :**

**Synthèse du diméthylsulfate 2,2'-{disulfanediylbis[éthane-2,1-diyl(méthylimino)-4,1-phénylèneéthène-2,1-diyl]}bis(3-méthyl-1,3-thiazol-3-ium) [6]** 1.66 g de méthylsulfate de 2,3-diméthyl-thiazolium en solution dans 2 mL de mélange isopropanol / N-méthylpyrrolidinone 60: 40 sont ajoutés à 1.43 g de 4,4'-{disulfanediylbis[éthane-2,1-diyl(méthylimino)]}dibenzaldehyde en suspension dans 5 mL de mélange isopropanol / N-méthylpyrrolidinone 60 : 40 et 0.56 mL de pyrrolidine. Le mélange est maintenu à 50°C, sous agitation pendant 7h, sous argon à l'abri de la lumière. Après refroidissement, la suspension est versée sur 25 mL d'acétate d'éthyle froid. Le précipité obtenu est filtré, lavé à l'acétate d'éthyle et séché.

**Example 7 - Synthèse du mésylate de 2-[2-(4-{[2-(acétylsulfanyl)-éthyl](méthyl)amino}-phényl)éthényl]-1,3-diméthyl-1*H*-benzimidazol-3-ium [7]**

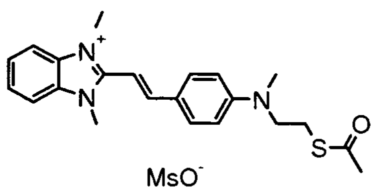

**[7]**

**Schéma de synthèse:**

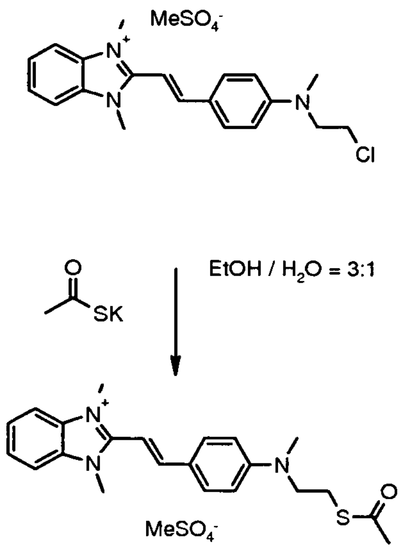


**[7]**

**Mode opératoire :**

Un mélange de mésylate de 2-[2-{4-[(2-chloroethyl)(methyl)-amino]phenyl}-éthényl]-1,3-diméthyl-1H-benzimidazol-3-ium (1.6 g, 3.5 mmol), de thioacétate de potassium (0.6 g, 5.3 mmol) dans 12 ml d'éthanol et de 4 ml d'eau est chauffé à reflux pendant 12 h. Après refroidissement, les solvants sont évaporés sous vide. Le résidu est chromatographié sur alumine, élué avec un mélange de dichlorométhane : méthanol (500:3) pour arriver à 0.8 g de produit brut. Par la suite le brut est purifié par recristallisation dans un mélange de 2-propanol et acétate d'éthyle pour conduire à 0.7 g de poudre orange. Les analyses sont en accord avec la structure attendu du composé **[7]** et indique un ratio E/Z en 69/31.

**Example 8 : synthèse de thiosulfate de S-{2-[{4-[2-(1,3-diméthyl-1*H*-benzimidazol-3-ium-2-yl)éthényl]phényl}(méthyl)amino]-éthyl} [8]**

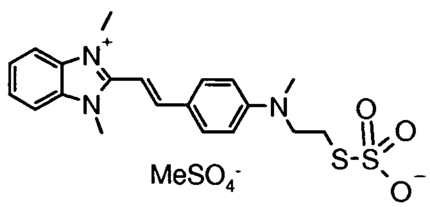

**[8]**

**Schéma de synthèse:**

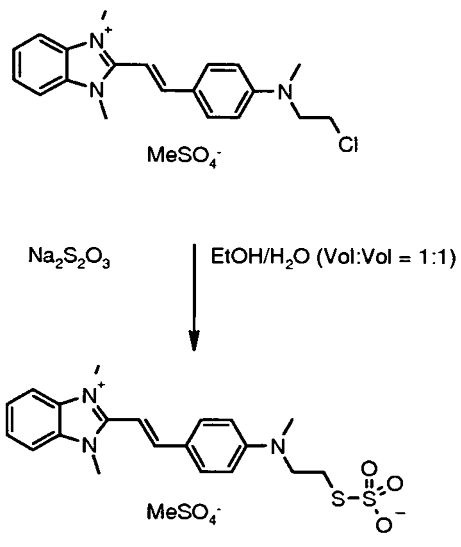


**[8]**

**Mode opératoire :**

A une solution sous agitation de mésylate de 2-[2-{4-[(2-chloroéthyl)(méthyl)-amino]phényl}-éthényl]-1,3-dimethyl-1 H-benzimidazol-3-ium (1.0 g, 2.2 mmol) dans 10 ml d'éthanol et 10 ml d'eau, est ajouté 1.4 g (8.9 mmol) de thiosulfate de sodium. Le mélange a été chauffé sous reflux pendant 48h. Le solide résultant est collecté par filtration et lavé 3 x avec de l'eau et 2 x dans l'éthanol. .Le résidu est ensuite séché pour conduire à 0.9 g de poudre orange-jaune. Les analyses sont en accord avec la structure du composé [8].

**<u>Example 9 :</u> synthèse de 1,1'-{disulfanediylbis[éthane-2,1-diylimino(2-oxoéthane-2,1-diyl)]}bis{2-[2-{4-[bis(2-hydroxyéthyl)amino]phényl}éthényl]-3-méthyl-1*H*-benzimidazol-3-ium} chloride [9]**

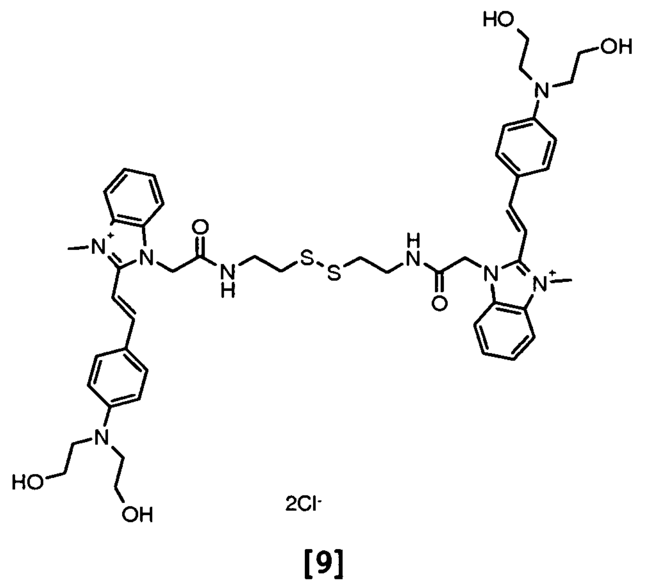

**[9]**

**Mode opératoire :**

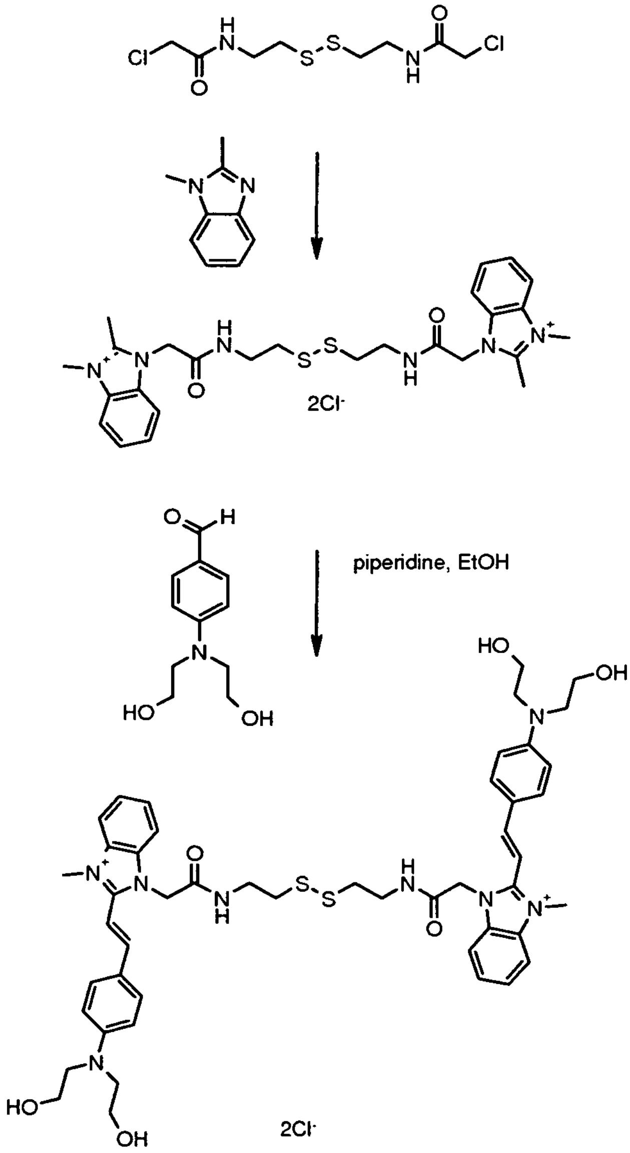


**[9]**

**Etape 1 : synthèse de chlorure 1,1'-{disulfanediylbis[éthane-2,1-diylimino(2-oxo-éthane-2,1-diyl)]}bis (2,3-diméthyl-1*H*-benzimidazol- 3- ium)** Un mélange de 1,2-diméthyl-1*H*-benzimidazole (2.93 g, 20 mmol) et *N,N'*-(disulfanediyldiéthane-2,1-diyl)bis(2-chloroacétamide) (1.53 g, 5 mmol) dans 20 ml d'acétonitrile est chauffé à reflux pendant 24 h. Après refroidissement, le solide blanc résultant est collecté et lavé avec de l'acétate d'éthyle et dichloromethane pour arriver à 1.94 g de poudre blanche. Les analyses sont en accord avec la structure attendue.

**Etape 2 : synthèse de chlorure de 1,1'-{disulfanediylbis[éthane-2,1-diylimino(2-oxoéthane-2,1-diyl)]} bis{2-[2-{4-[bis(2-hydroxyéthyl)amino]phényl}éthényl]-3-méthyl-1H-benzimidazol-3-ium} [9]** A une solution sous agitation de 4-[bis(2-hydroxyéthyl)-amino]benzaldéhyde (1.7 g, 8.1 mmol) et de chlorure de 1,1'-{disulfanediylbis[ethane-2,1-diylimino(2-oxoéthane-2,1-diyl)]}bis(2,3-diméthyl-1*H*-benzimidazol-3-ium) (1.2 g, 2 mmol) dans 35 ml d'éthanol, il est ajouté 0.5 ml of piperidine comme catalyseur. Le mélange réactionnel est chauffé à reflux pour 7 h. Après refroidissment le solide orange resultant est collecté par filtration puis lavé au dichlorométhane et méthanol. Le résidu séché est obtenu 1.6 g sous forme de solide orange. Les analyses sont en accord avec la structure du compos [9].

**<u>Example 10</u>: synthèse de chlorure de 1,1'-{idisulfanediylbis[éthane-2,1-diylimino(2-oxoéthane-2,1-diyl)]}bis{3-méthyl-2-[2-(2,3,6,7-tétrahydro-1*H*,5*H*-pyrido[3,2,1-ij]quinolin-9-yl)éthényl]-1*H*-benzimidazol-3-ium} [10]**

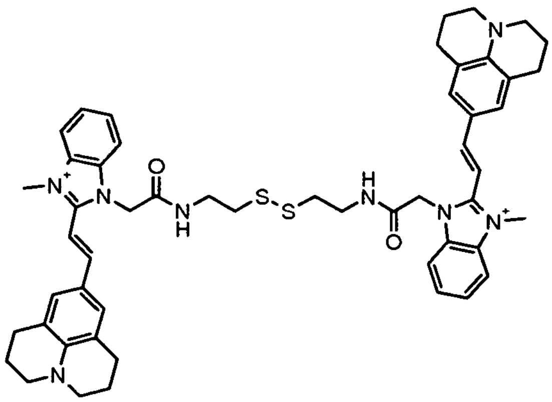

2Cl-

**[10]**

**Mode opératoire :**

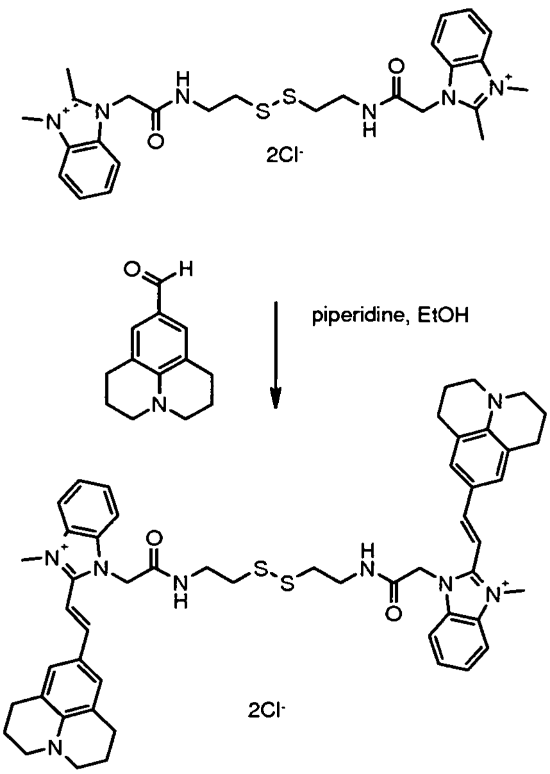


**[10]**

A une solution de 2,3,6,7-tétrahydro-1*H*,5*H*-pyrido[3,2,1-ij]quinoline-9-carbaldéhyde (julolidine carboxaldéhyde, 12.01 10 mmol) et de chlorure 1,1'-{disulfane-diylbis[éthane-2,1-diylimino(2-oxoéthane-2,1-diyl)]}bis(2,3-diméthyl-1*H*-benzimidazol-3-ium) (1.49 g, 2.5 mmol) dans 15 ml d'acétonitrile éthanol, Il est ajouté 0.2 ml de piperidine comme catalyseur. Le mélange réactionnel est chauffé à reflux 24 h. Après refroidissement le solide resultant est collecté par filtration et ensuite lavé avec 3 x de l'acétonitrile pour conduire à 1.45 de solide rouge. Les analyses (1 H NMR, 13C NMR and MS) sont en accord avec la structure **[10].**

## EXEMPLES DE COLORATION

**Exemple 1 : Procédé de coloration - composés [1], [2] et [4]**

Préparation d'une composition A

| | |
|---|---|
| Colorant disulfure de [1] [2] ou [4] | $5.10^{-4}$ mol% |
| Alcool Benzylique | 4 g |
| Polyéthylèneglycol 60E | 6 g |
| Hydroxyethylcellulose | 0,7 g |
| Alkylpolyglucoside en solution aqueuse à 65%MA | 4.5 g 4.5 g |
| Eau déminéralisée | qsp 100g |

Préparation d'une composition B

| | |
|---|---|
| Acide thioglycolique | 1M |
| Hydroxyde de Sodium | qsp pH 8,5 |
| Eau déminéralisée | qsp 100g |

Au moment de l'emploi, on mélange les compositions A (22.5 mL) et B (2.5 mL), puis on applique le mélange obtenu sur deux mèches de 1 g de cheveux blancs (BN), deux mèches de 1 g cheveux permanentés (BP) et une mèche de 1 g de cheveux foncés (hauteur de ton 4) pendant 20 minutes à température ambiante (les mèches sont retournées et réimprégnées après 10 minutes).

Après rinçage à l'eau courante, un shampooing et un nouveau rinçage suivi de séchage, on observe un éclaircissement des cheveux foncés ainsi traités : la mèche de hauteur de ton 4 est devenue visuellement plus claire que des mèches témoins non traitées. Les mèches de cheveux blancs sont colorées avec des nuances intenses.

*Observations visuelles*

Lors du rinçage et des shampooings des exemples [1], [2] et [4] il n'y a pas de dégorgement visible, la mousse des shampooings et les eaux de rinçage ne sont pratiquement pas colorées.

La couleur observée est conservée et l'effet d'éclaircissement reste visible sur les cheveux shampooinés.

*Résultats de Réflectance :*

Les performances d'éclaircissement des compositions conformes à l'invention et leur ont été exprimées en fonction de la réflectance des cheveux. Ces réflectances sont comparées à la réflectance d'une mèche de cheveux non traitées de hauteur de ton HT4 .

La réflectance est mesurée au moyen d'un appareil spectrophotocolorimètre KONIKA-MINOLTA ®, CM 3600d et après irradiation des cheveux avec une lumière visible dans la gamme de longueurs d'onde allant de 400 à 700 nanomètres.

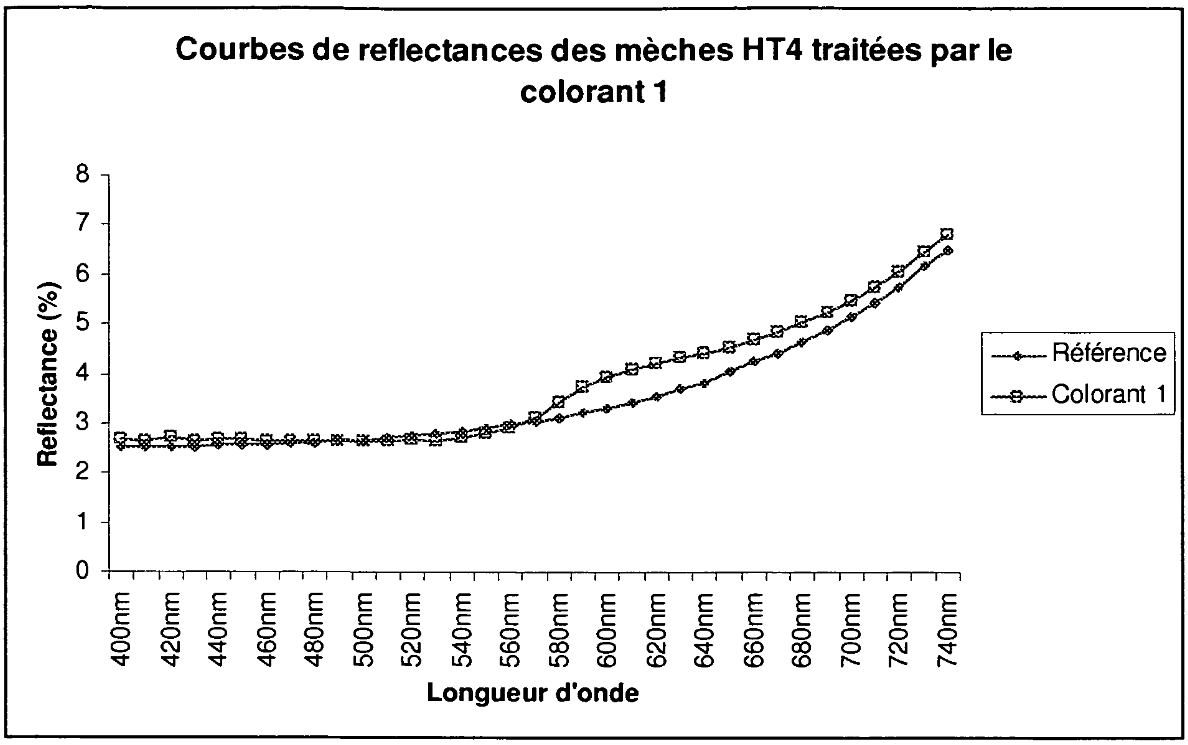


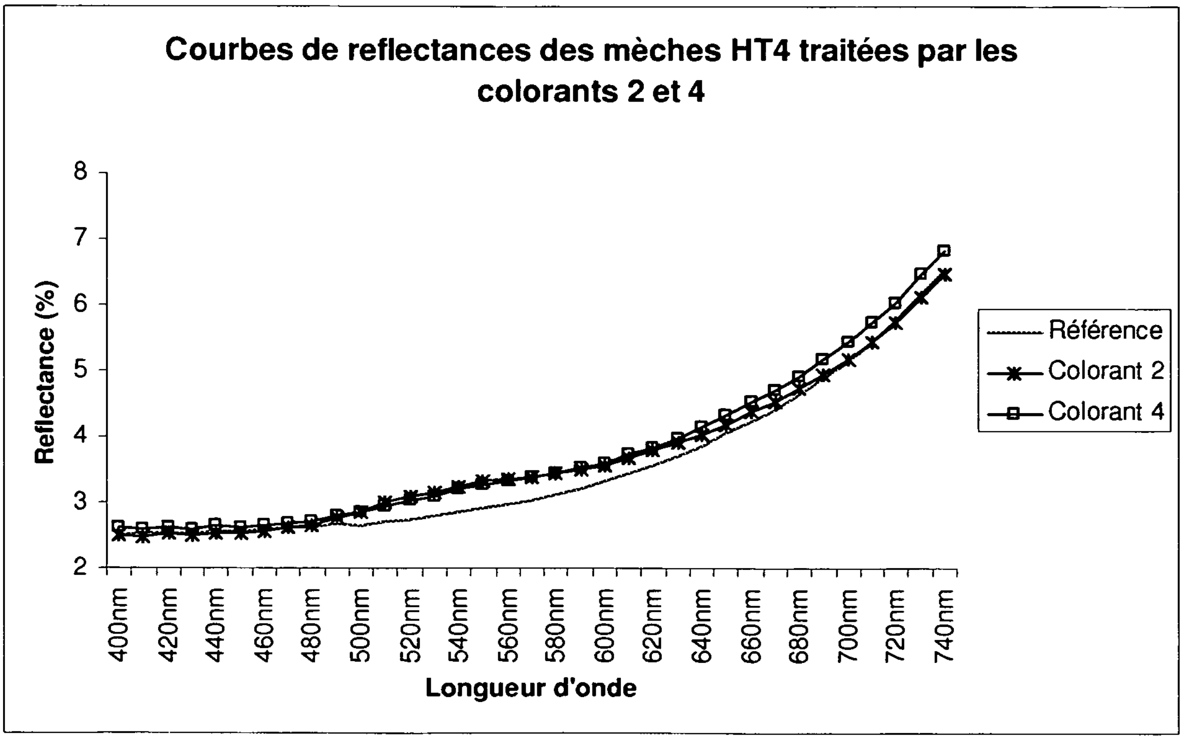


On constate tout d'abord que la réflectance d'une mèche de cheveux traitée avec une composition selon l'invention est supérieure à celle des cheveux non traités. Tout particulièrement la reflectance des mèches traitées avec le colorant [1] est nettement supérieure à celle de la mèche de référence dans la plage de longueur d'ondes supérieure à 560nm. Pour les colorants [2] et [4], la reflectance est supérieure sur une plage de longueur d'onde plus vaste, dès 500nm. Les mèches traitées par ces trois composés apparaissent donc plus claires.

*Résultats dans le système L*a*b* :*

La couleur des mèches été évaluée dans le système L*a*b* au moyen d'un spectrocolorimètre CM 3600D MINOLTA ®, (Illuminant D65).

Dans ce système L* a* b*, L* représente la luminosité, a* indique l'axe de couleur vert/rouge et b* l'axe de couleur bleu/jaune. Plus la valeur de L est élevée, plus la couleur est claire ou peu intense. Inversement, plus la valeur de L est faible, plus la couleur est foncée ou très intense. Plus la valeur de a* est élevée plus la nuance est rouge et plus la valeur de b* est élevée plus la nuance est jaune.

La variation de la coloration entre les mèches de cheveux HT4, colorées et lavées est mesurée par (ΔE) selon l'équation suivante :

$$\Delta E = \sqrt{(L^* - L_o^*)^2 + (a^* - a_o^*)^2 + (b^* - b_o^*)^2}$$

Dans cette équation, L*, a* et b* représentent les valeurs mesurées avant coloration et $L_0$*, $a_0$* et $b_0$* représentent les valeurs mesurées avant coloration (ou shampooing).

Plus la valeur de ΔE est importante, plus la différence de couleur entre les mèches HT4 et les mèches colorées est importante.

| Composé | L*(D65) | a*(D65) | b*(D65) | dE*ab(D65) |
|---|---|---|---|---|
| Référence HT4 | 20 | 2,69 | 3,02 | ------ |
| Composé 1 | 20,48 | 5,63 | 3,21 | 2,98 |
| Composé 2 | 21,14 | 1,6 | 4,96 | 2,49 |

| | L*(D65) | a*(D65) | b*(D65) | dE*ab(D65) |
|---|---|---|---|---|
| Référence | 60,45 | 1,49 | 16,08 | ------ |
| Composé 1 | 39,51 | 41,71 | 6,64 | 46,32 |
| Composé 2 | 60,53 | 7,75 | 65,3 | 49,62 |
| Composé 4 | 59,29 | 7,43 | 54,55 | 38,95 |

Les valeurs rapportées dans les tableaux ci-dessus montrent l'effet observable de la coloration obtenue même sur les cheveux foncés (HT4) et une coloration très intense sur cheveux blancs.

**Revendications**

**1.** Colorant fluorescent de formule (I) ou (II):

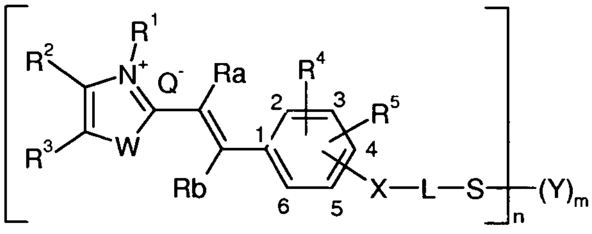

**(I)**

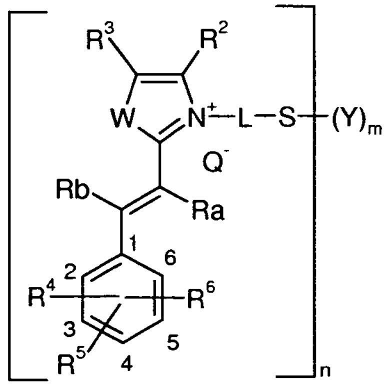

**(II)**

ses sels d'acide organique ou minéral, isomères optiques, isomères géométriques, et les solvates tels que hydrates ; **formules (I)** ou **(II)** dans lesquelles :

- ➢ **n** représente 1 ou 2 ;
- ➢ **m** représente 0 ou 1 ;
- ➢ **Ra** et **Rb,** identiques ou différents, représentent un atome d'hydrogène ou un groupement alkyle en $C_1$-$C_4$;
- ➢ $R^1$ représente un atome d'hydrogène ou un groupement ($C_1$-$C_6$)alkyle, éventuellement substitué par un groupement choisi parmi halogène, alcoxy en $C_1$-$C_4$, (di)(alkyl)amino en $C_1$-$C_4$, phényle, tolyle et méthoxyphényle ;
- ➢ ou alors $\mathbf{R^1}$ et **Ra** forment ensemble une chaîne ($C_3$-$C_6$)alkylène ou une chaîne ($C_3$-$C_7$)alkénylène ;
- ➢ $\mathbf{R^2}$ et $\mathbf{R^3}$**,** identiques ou différents, représentent un atome d'hydrogène, d'halogène, un groupement ($C_1$-$C_6$) alkyle éventuellement substitué, ($C_1$-$C_6$)alkoxy, ($C_1$-$C_6$)alkylthio; ou alors $\mathbf{R^2}$ et $\mathbf{R^3}$ forment ensemble avec les atomes de carbone qui les portent un cycle benzo éventuellement substitué ;
- ➢ $\mathbf{R^4}$**,** $\mathbf{R^5}$ et $\mathbf{R^6}$ identiques ou différents, représentent :

  - • un atome d'hydrogène ;
  - • un radical alkyle en $C_1$-$C_6$, éventuellement substitué par un ou plusieurs radicaux choisis parmi les radicaux hydroxy, alcoxy en $C_1$-$C_2$, (poly)-hydroxyalcoxy en $C_2$-$C_4$, acylamino, amino substitué par deux radicaux alkyle, identiques ou différents, en $C_1$-$C_4$;
  - • un atome d'halogène ;
  - • un groupement hydroxy ;
  - • un radical alcoxy en $C_1$-$C_2$ ;
  - • un radical alkylthio en $C_1$-$C_2$;
  - • un radical amino ;
  - • un radical hétérocycloalkyle à 5 ou 6 chaînons pouvant être substitués par 1 à 3 groupements identiques ou différents choisis parmi hydroxy, amino, (di)alkyamino, et hydroxyalkyle en $C_1$-$C_4$ ;
  - • un radical amino substitué par un ou deux radicaux alkyle, identiques ou différents, en $C_1$-$C_6$ éventuellement porteurs d'au moins :

    i) un groupement hydroxy,
    ii) un groupement amino éventuellement substitué par un ou deux radicaux alkyle en $C_1$-$C_3$, lesdits radicaux alkyle pouvant former avec l'atome d'azote auquel ils sont rattachés, un hétérocycle comprenant de 5 à 7 chaînons, saturé ou insaturé éventuellement substitué comprenant éventuellement au moins un autre hétéroatome différent ou non de l'azote ;

  - • -N(R)-C(O)-R' dans lequel le radical R est un atome d'hydrogène, un radical alkyle en $C_1$-$C_4$ éventuellement porteur d'au moins un groupement hydroxy et le radical R' est un radical alkyle en $C_1$-$C_2$ ;
  - • un radical carboxylique sous forme acide ou salifiée ;
  - • un groupement polyhalogénoalkyle comprenant de 1 à 6 atomes de carbones et de 1 à 6 atomes d'halogène, identiques ou différents ;

- ➢ ou alors deux radicaux contigus $R^4$ avec $R^5$ et/ou $R^5$ avec $R^6$ forment ensemble avec les atomes de carbone

qui les portent un cycle benzo ou un hétérocycle fusionné éventuellement substitué au groupe phényle ;
➢ ou alors $R^4$, $R^5$ et $R^6$ forment ensemble avec le groupement phényle de la formule (II) un motif tricyclique de type julolidine :

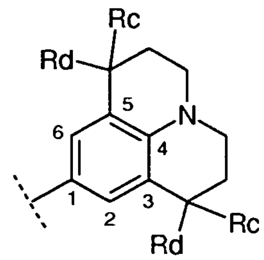

avec Rc et Rd représentant un atome d'hydrogène ou une groupement alkyle en $C_1$-$C_4$ ;
➢ L représente une chaîne hydrocarbonée bivalente en $C_1$-$C_{20}$, éventuellement substituée, éventuellement interrompue et/ou éventuellement terminée à l'une ou l'autre de ses extrémités i) par un ou plusieurs groupes divalents ou leur combinaisons choisis parmi : -N(R)-; $-N^+(R)(R°)-$, $An^-$; -O-, -S-, -CO-, $-SO_2-$ avec R, R°, identiques ou différents, choisi parmi un hydrogène, un radical alkyle en $C_1$-$C_4$, hydroxyalkyle, aminoalkyle et $An^-$ représentant un contre-ion anionique ou ii) par un hétérocycle cationique ou hétéroaryle cationique $Hét^+$, $An^-$, avec $An^-$ représentant un contr-ion anionique et $Het^+$ représentant un hétérocycle comprenant de 5 à 10 chaînons, saturé ou non, ou un hétéroaryle comprenant de 5 à 10 chaînons ;
➢ **X** représente un atome d'oxygène, de soufre ou un groupement NR avec R représentant un atome d'hydrogène ou un groupement ($C_1$-$C_6$)alkyle ;
➢ **Y** représente : i) un atome d'hydrogène ; ii) un métal alcalin ; iii) un métal alcalinoterreux ; iv) un groupement ammonium : $N^+R^\alpha R^\beta R^\gamma R^\delta$, $An''^-$ ou un groupement phosphonium : $P^+R^\alpha R^\beta R^\gamma R^\delta$, $An''^-$ avec $R^\alpha$, $R^\beta$, $R^\gamma$ et $R^\delta$, identiques ou différents, représentant un atome d'hydrogène ou un groupement ($C_1$-$C_4$)alkyle et , $An''^-$ représentant un contre-ion anionique ; ou v) un groupement protecteur de fonction thiol ;
➢ **W** représente un atome d'oxygène, de soufre ou un groupement NR' avec R' représente un atome d'hydrogène ou un groupe ($C_1$-$C_4$)alkyle ;
➢ **Q⁻** représente un contre-ion anionique ;

étant entendu que lorsque n vaut 2 alors m vaut zéro, et lorsque n vaut 1 alors m vaut 1.

2. Colorant fluorescent de formule **(I)** ou **(II)** selon la revendication précédente dans lequel m et n valent 1, et Y représente un atome d'hydrogène, ou un métal alcalin.

3. Colorant fluorescent de formule **(I)** ou **(II)** selon la revendication 1 dans lequel m et n valent 1, et Y représente un groupement protecteur.

4. Colorant fluorescent de formule **(I)** ou **(II)** selon la revendication précédente dans lequel Y représente un groupement protecteur choisi parmi les radicaux suivants :

■ ($C_1$-$C_4$)alkylcarbonyle ;
■ ($C_1$-$C_4$)alkylthiocarbonyle ;
■ ($C_1$-$C_4$)alcoxycarbonyle ;
■ ($C_1$-$C_4$)alcoxythiocarbonyle ;
■ ($C_1$-$C_4$)alkylthio-thiocarbonyle ;
■ (di) ($C_1$-$C_4$) (alkyl)aminocarbonyle ;
■ (di) ($C_1$-$C_4$) (alkyl)aminothiocarbonyle;
■ arylcarbonyle ;
■ aryloxycarbonyle ;
■ aryl($C_1$-$C_4$)alkcoxycarbonyle ;
■ (di) ($C_1$-$C_4$) (alkyl)aminocarbonyle ;
■ ($C_1$-$C_4$)(alkyl)arylaminocarbonyle ;
■ carboxy ;
■ $SO_3^-$ ; $M^+$ avec $M^+$ représentant un métal alcalin ou alors $An^-$ ou $An'^-$ de la formule (I) ou (II) et $M^+$ sont absents ;

■ aryle éventuellement substitué ;
■ hétéroaryle éventuellement substitué ;
■ hétérocycloalkyle éventuellement substitué, éventuellement cationique,
■ le groupement suivant :

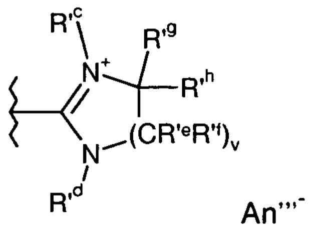

dans lequel $R'^c$, $R'^d$, $R'^e$, $R'^f$, $R'^g$ et $R'^h$, identiques ou différents représentent un atome d'hydrogène ou un groupement $(C_1\text{-}C_4)$ alkyle, ou alors deux groupement $R'^g$ avec $R'^h$, et/ou $R'^e$ avec $R'^f$ forment un groupement oxo ou thioxo, ou alors $R'^g$ avec $R'^e$ forment ensemble un cycloalkyle ; et v représente un entier compris inclusivement entre 1 et 3 ; préférentiellement $R'^c$ à $R'^h$ représentent un atome d'hydrogène ; et $An'''^-$ représente un contre-ion;
■ isothiouronium ;
■ $-C(NR'^cR'^d)=N+R'^eR'^f$; $An'''^-$ avec $R'^c$, $R'^d$, $R'^e$ et $R'^f$, identiques ou différents représentent un atome d'hydrogène ou un groupement $(C_1\text{-}C_4)$alkyle et $An'''^-$ est tel que défini précédemment ;
■ isothiourée ;
■ $-C(NR'^cR'^d)=NR'^e$; avec $R'^c$, $R'^d$ et $R'^e$ sont tels que définis précédemment ;
■ (di)aryl$(C_1\text{-}C_4)$alkyle éventuellement substitué ;
■ (di)hétéroary)$(C_1\text{-}C_4)$akyle éventuellement substitué;
■ $CR^1R^2R^3$ avec $R^1$, $R^2$ et $R^3$ identiques ou différents, représentant un atome d'halogène ou un groupe choisi parmi :

- $(C_1\text{-}C_4)$alkyle ;
- $(C_1\text{-}C_4)$alcoxy;
- aryle éventuellement substitué ;
- hétéroaryle éventuellement substitué ;
- $P(Z^1)R'^1R'^2R'^3$ avec $R'^1$, et $R'^2$ identiques ou différents représentent un groupement hydroxy, $(C_1\text{-}C_4)$ alcoxy ou alkyle, $R'^3$ représente un groupement hydroxy ou $(C_1\text{-}C_4)$alcoxy, et $Z^1$ représente un atome d'oxygène ou de soufre ;

■ cyclique stériquement encombré ; et
■ alcoxyalkyle éventuellement substitué.

**5.** Colorant fluorescent de formule **(I)** ou **(II)** selon l'une quelconque des revendications précédentes dans lequel Y représente un métal alcalin ou un groupement protecteur choisi parmi :

➢ $(C_1\text{-}C_4)$alkylcarbonyle ;
➢ arylcarbonyle ;
➢ $(C_1\text{-}C_4)$alkoxycarbonyle ;
➢ aryloxycarbonyle ;
➢ aryl$(C_1\text{-}C_4)$alkoxycarbonyle ;
➢ (di) $(C_1\text{-}C_4)$ (alkyl)aminocarbonyle ;
➢ $(C_1\text{-}C_4)$(alkyl)arylaminocarbonyle
➢ aryle éventuellement substitué ;
➢ hétéroaryle monocyclique cationique à 5 ou 6 chaînons éventuellement substitué par un ou plusieurs, identiques ou différents, groupes $(C_1\text{-}C_4)$alkyle ;
➢ hétéroaryle bicyclique cationique à 8 à 11 chaînons éventuellement substitué par un ou plusieurs, identiques ou différents, groupes $(C_1\text{-}C_4)$alkyle ;
➢ hétérocycle cationique de formule suivante :

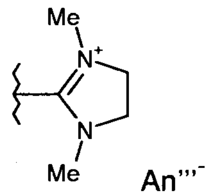

- ➢ isothiouronium ;
- ➢ $-C(NH_2)=N^+H_2$; An'''-; avec An'''- représentant un contre-ion anionique;
- ➢ isothiourée ;
- ➢ $-C(NH_2)=NH$ ; et
- ➢ $SO_3$ ; $M^+$ avec $M^+$ représentant un métal alcalin ou alors $An^-$ ou An'- de la formule (I) ou (II) et $M^+$ sont absents.

**6.** Colorant fluorescent disulfure de formule (I) ou (II) selon l'une quelconque des revendications précédentes dans lequel n=2 et m=0.

**7.** Colorant fluorescent selon une quelconque des revendications précédentes de formule **($I_a$)**, **($II_a$)**, **($I_b$)** et **($II_b$)** :

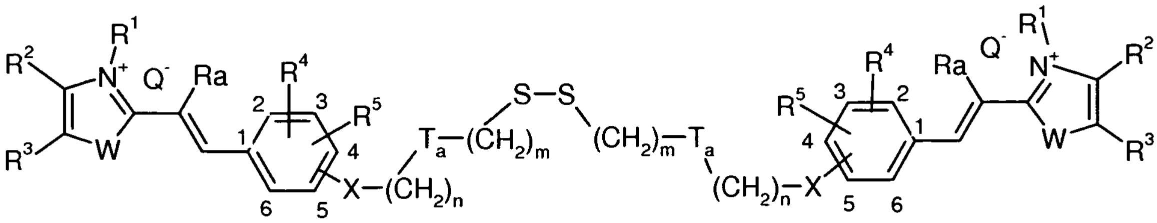

**($I_a$)**

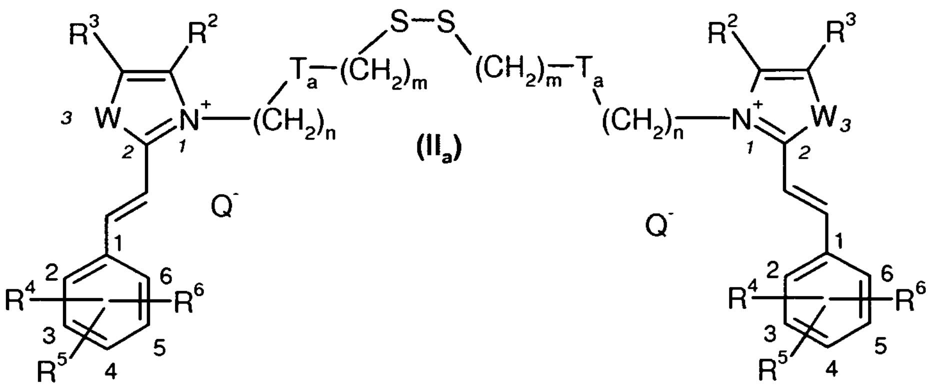

**($II_a$)**

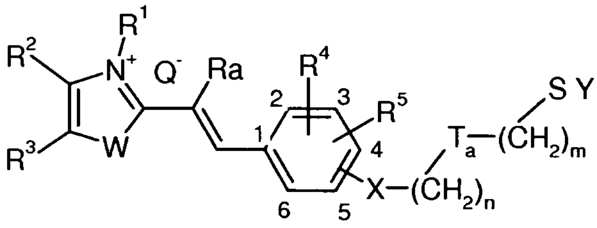

**($I_b$)**

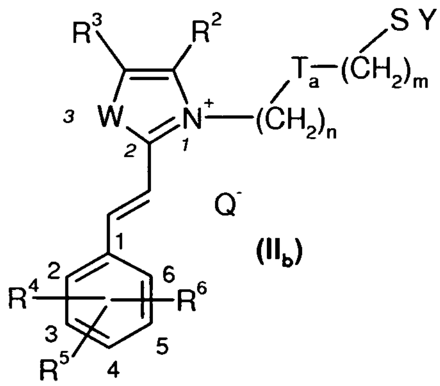

**($II_b$)**

formules **($I_a$)**, **($II_a$)**, ($I_b$) et **($II_b$)** dans lesquelles :

- **Ra, $R^1$** à **$R^6$ B, Y** et **Q-** sont tels que dans les revendications 1 à 5 ;
- **n** et **m,** identiques ou différents, représentent un entier compris inclusivement entre 1 et 6 avec la somme de n+m qui est comprise inclusivement entre 2 et 4 ;
- **X** représente un radical choisi parmi : -G-, -G'-C(G)- et -C(G)-G'-, avec G et G', identiques ou différents, représentant un atome d'oxygène, de soufre, ou NR, avec R représentant un atome d'hydrogène ou un groupement ($C_1$-$C_6$)alkyle ;
- **$T_a$** représente une liaison covalente $\sigma$, un groupement -N($R'_a$)-; -$N^+$($R'_a$)($R'_b$)-, $An^-$; -C(O)-N($R'_a$)- ou -N($R'_a$)-C(O)-, ou un hétéroaryle cationique divalent comprenant de 5 à 7 chainons, avec $R'_a$, $R'_b$, identiques ou différents, représentant un atome d'hydrogène, un radical ($C_1$-$C_4$)alkyle, $An^-$ représentant un contre-ion anionique ; particulièrement $T_a$ représente -C(O)-N($R'_a$)- et -N($R'_a$)-C(O)-.

**8.** Colorant fluorescent de formule (I) ou (II) selon une quelconque des revendications précédentes choisi parmi les colorants suivants :

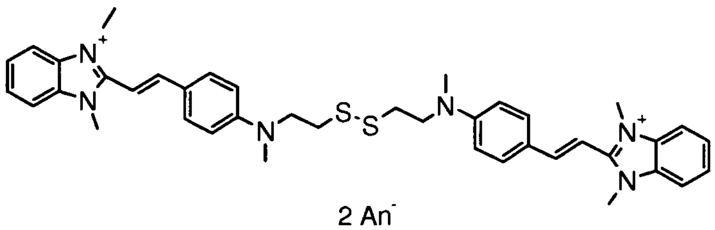

2 $An^-$

**1**

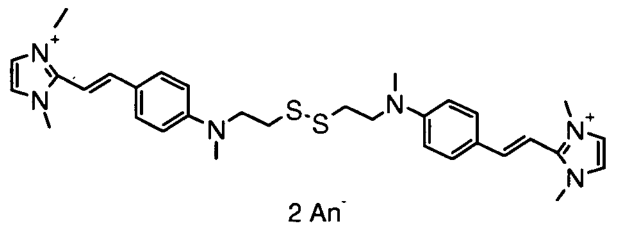

2 $An^-$

**2**

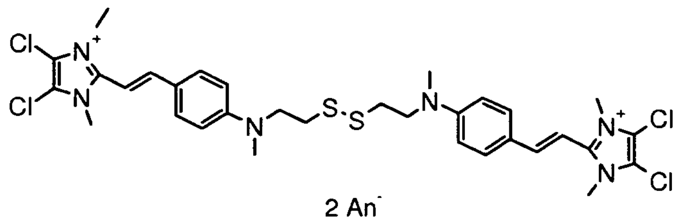

2 An⁻

**3**

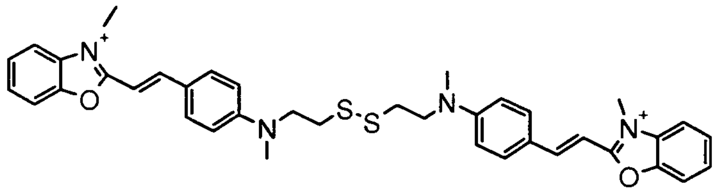

2 An⁻

**4**

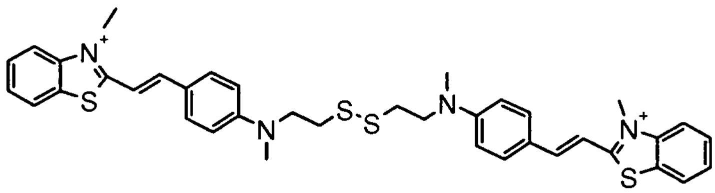

2 An⁻

**5**

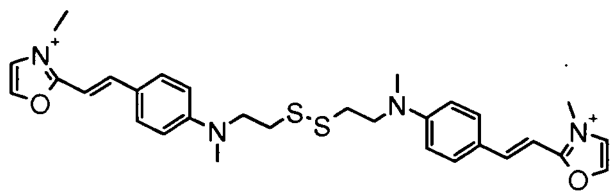

2 An⁻

**6**

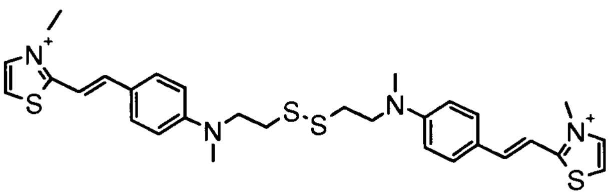

2 An⁻

**7**

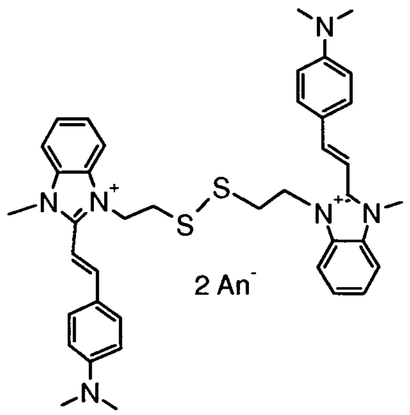

**8**

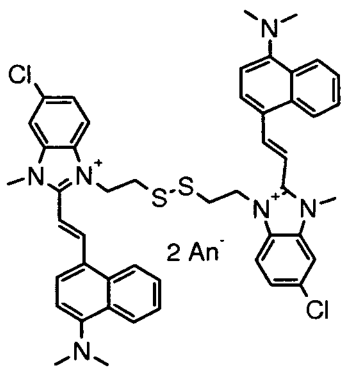

**9**

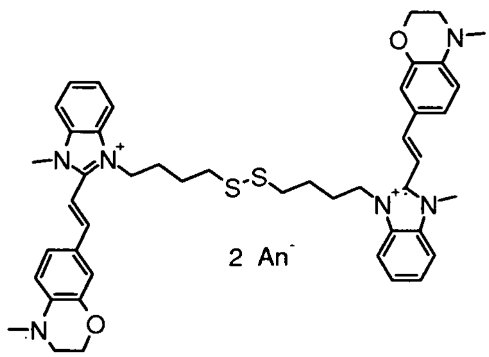

**10**

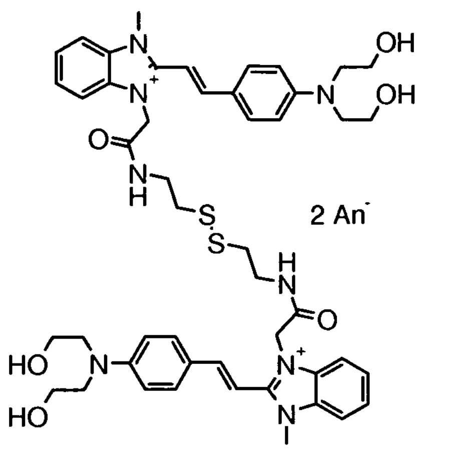

**11**

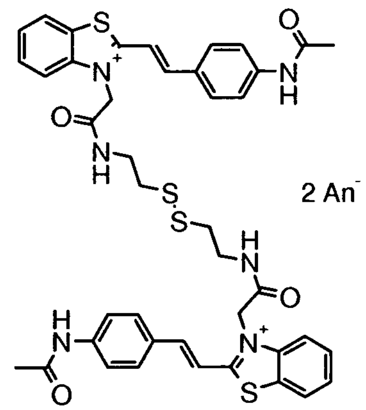

**12**

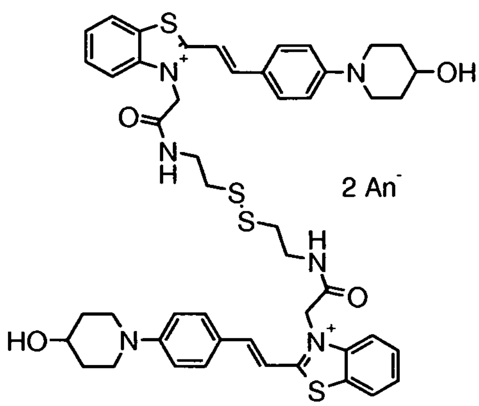

**13**

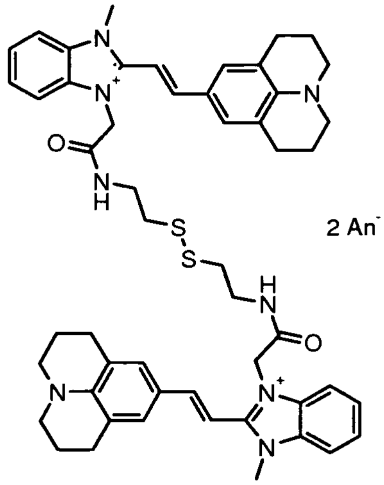

**14**

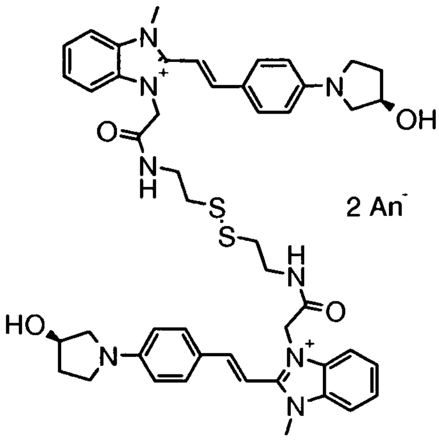

**15**

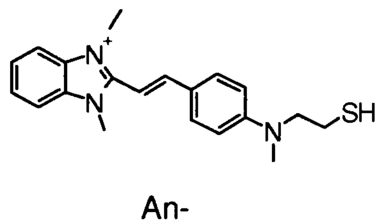

**16**

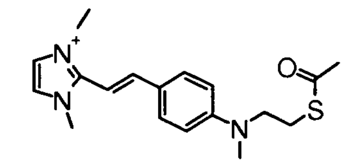

An$^-$

**17**

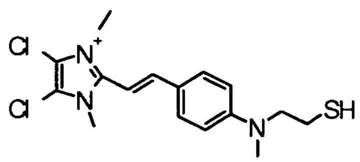

An$^-$

**18**

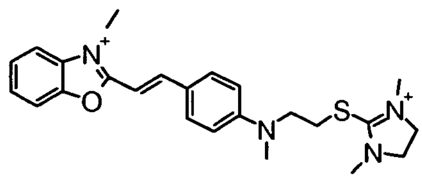

An$^-$

**19**

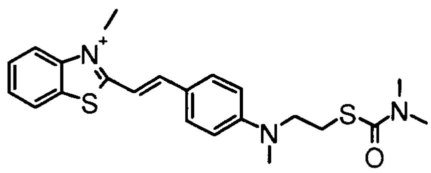

2 An$^-$

**20**

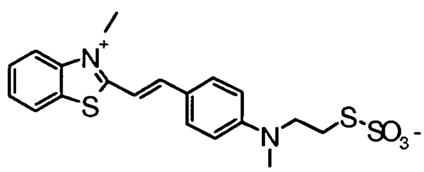

**21**

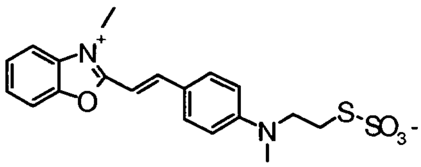

22

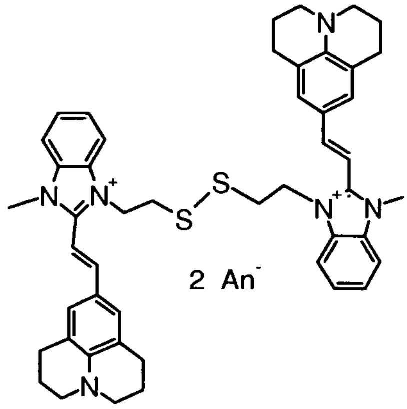

23

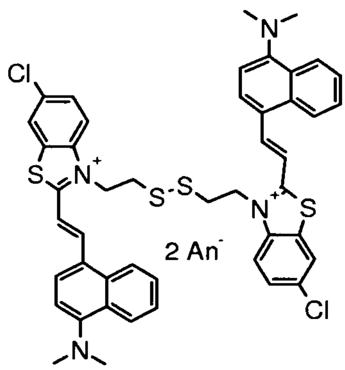

24

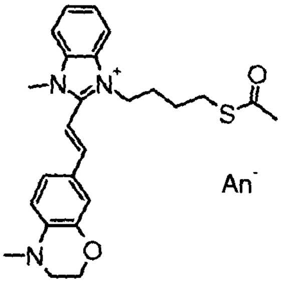

25

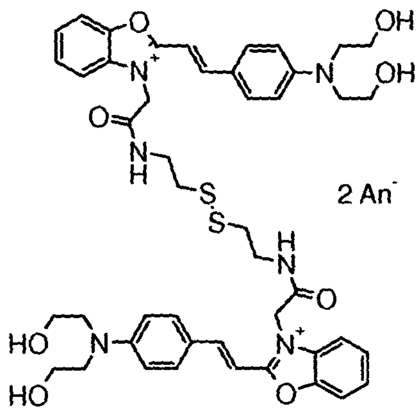

2 $An^-$

**26**

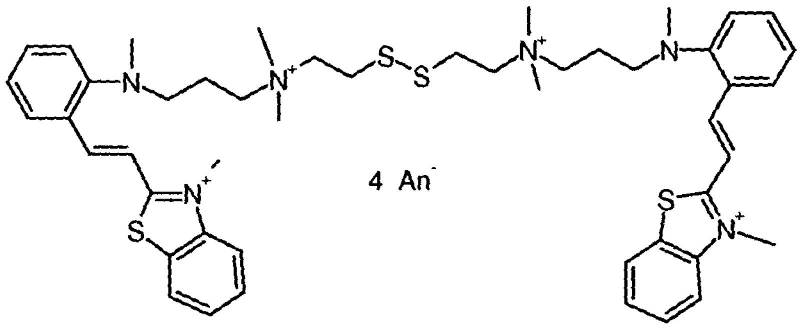

4 $An^-$

**27**

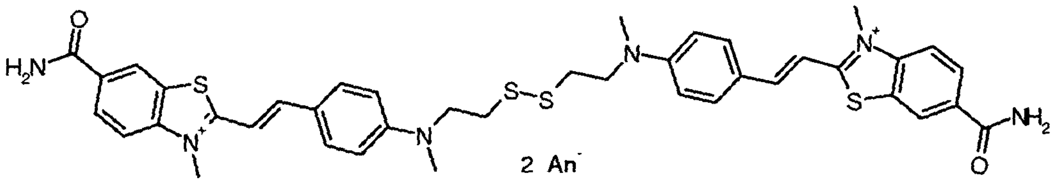

2 $An^-$

**28**

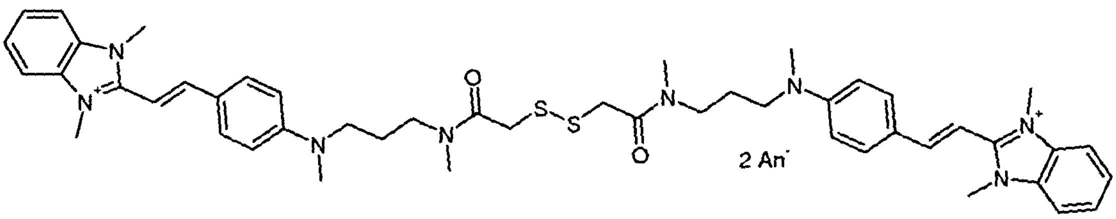

2 $An^-$

**29**

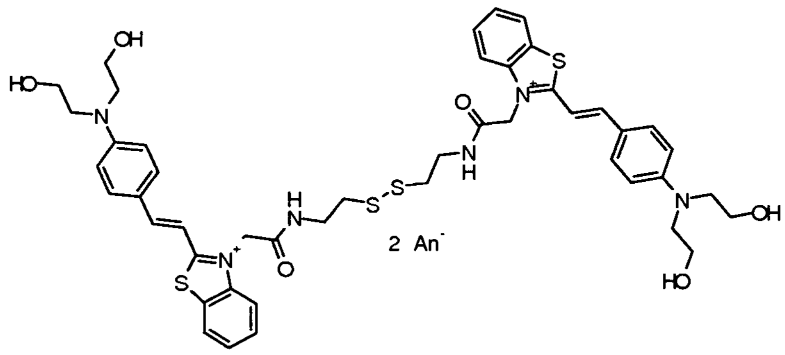

**30**

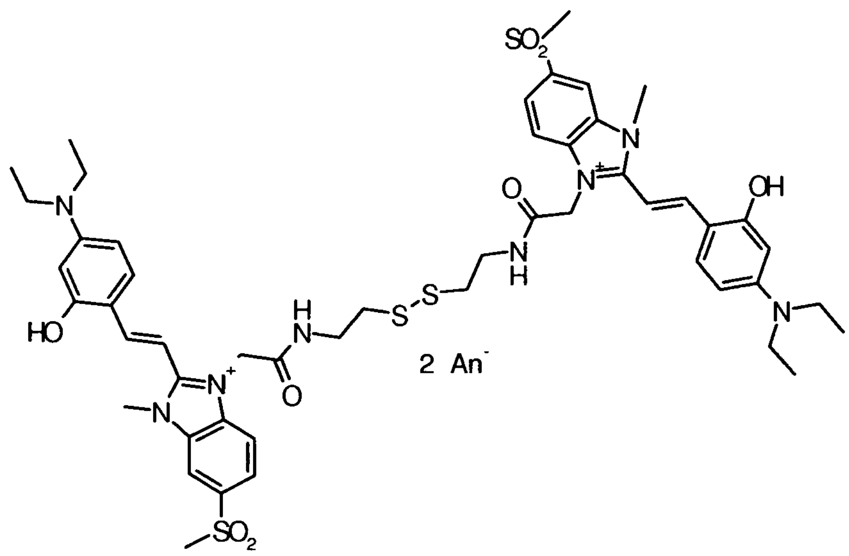

**31**

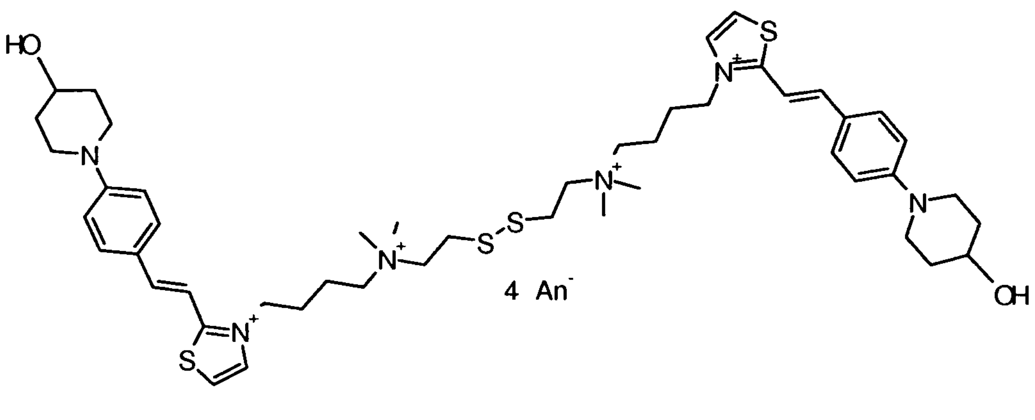

**32**

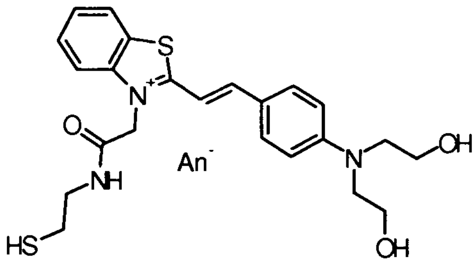

**33**

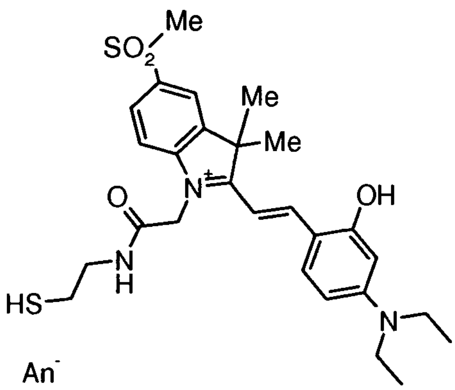

**34**

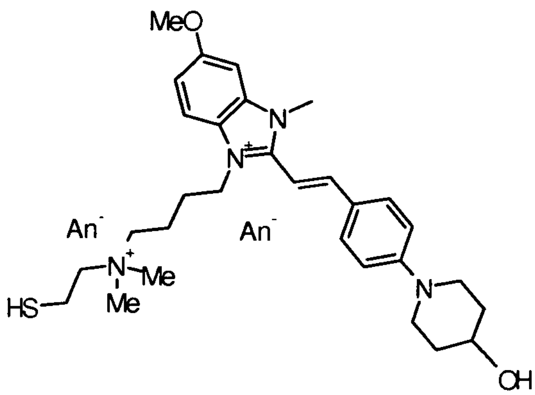

**35**

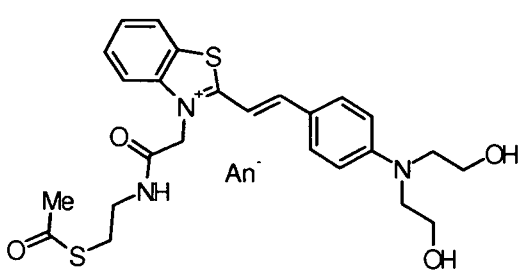

**36**

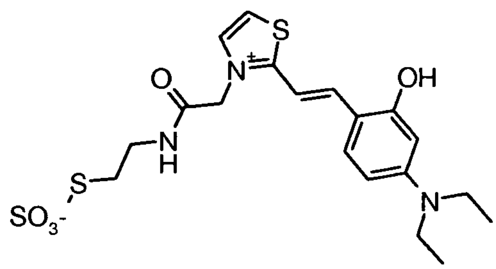

37

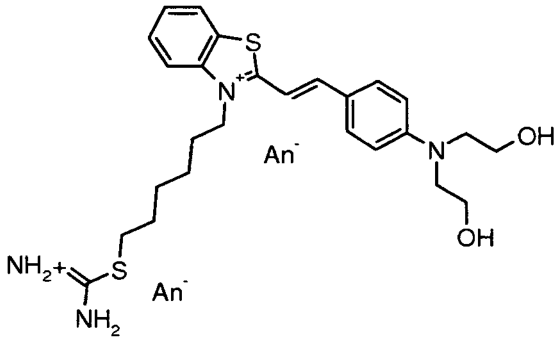

38

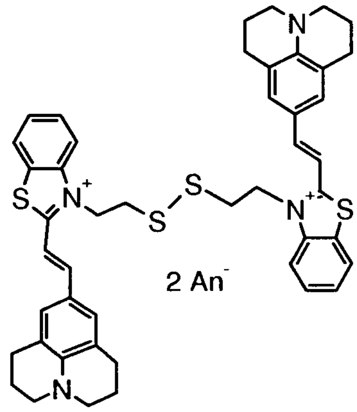

39

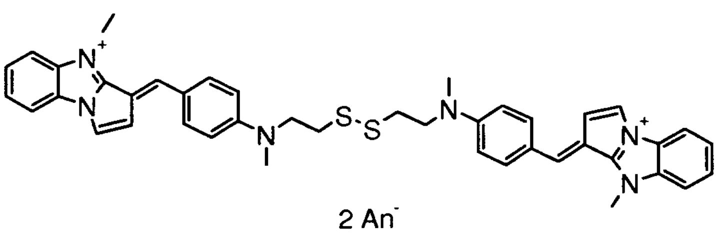

40

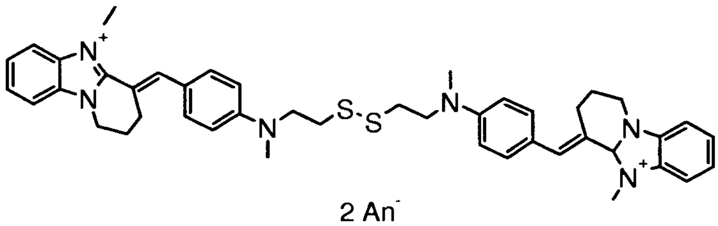

2 $An^-$

**41**

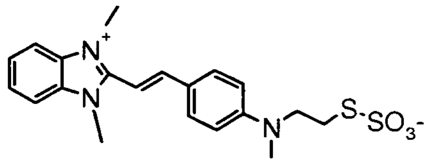

**42**

avec $An^-$, identiques ou différents, représentant un contre-ion anionique.

9. Composition tinctoriale comprenant dans un milieu cosmétique approprié, un colorant fluorescent de formule **(I)** ou **(II)** tel que défini dans une quelconque des revendications 1 à 8.

10. Procédé de coloration de matières kératiniques, dans lequel on applique sur les matières une composition tinctoriale approprié selon la revendication 9 comprenant au moins un colorant fluorescent de formule **(I)** ou **(II)**.

11. Procédé de coloration de matières kératiniques selon la revendication **10 caractérisé en ce que** les matières kératiniques sont des fibres kératiniques foncées possédant une hauteur de ton inférieure ou égale à 6.

12. Procédé selon l'une quelconque des revendications 10 ou 11 comprenant une étape supplémentaire consistant à appliquer sur les fibres kératiniques un agent oxydant.

13. Dispositif à plusieurs compartiments dans lequel un premier compartiment contient une composition tinctoriale comprenant un colorant fluorescent de formule **(I)** ou **(II)** tel que défini aux revendications 1 à 8 et un deuxième compartiment contient un agent réducteur.

14. Utilisation des colorants fluorescentes de formule **(I)** ou **(II)** tels que définis aux revendications 1 à 8 pour la teinture de fibres kératiniques humaines foncées particulièrement possédant une hauteur de ton inférieure à 6.

15. Utilisation selon la revendication précédente pour l'éclaircissement des fibres kératiniques foncées particulièrement possédant une hauteur de ton inférieure à 6.

**Claims**

1. Fluorescent dye of formula **(I)** or **(II):**

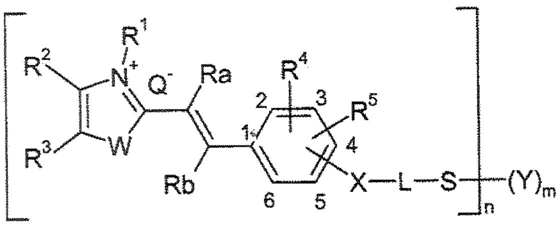

(I)

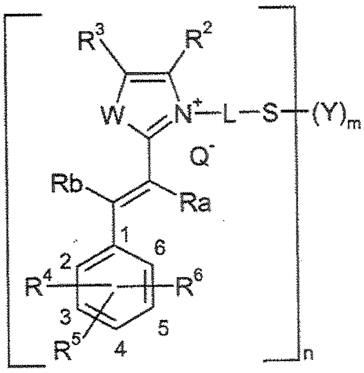

(II)

the organic or mineral acid salts, optical isomers and geometric isomers thereof, and the solvates such as hydrates:

in which formulae **(I)** and **(II)**:

- ➢ **n** represents 1 or 2;
- ➢ **m** represents 0 or 1;
- ➢ **Ra** and **Rb**, which may be identical or different, represent a hydrogen atom or a $C_1$-$C_4$ alkyl group;
- ➢ $\mathbf{R^1}$ represents a hydrogen atom or a ($C_1$-$C_6$)alkyl group, optionally substituted with a group chosen from halogen, $C_1$-$C_4$ alkoxy, $C_1$-$C_4$ (di) (alkyl) amino, phenyl, tolyl and methoxyphenyl;
- ➢ or else $\mathbf{R^1}$ and **Ra** together form a ($C_3$-$C_6$)alkylene chain or a ($C_3$-$C_7$)alkenylene;
- ➢ $\mathbf{R^2}$ and $\mathbf{R^3}$, which may be identical or different, represent a hydrogen or halogen atom, an optionally substituted ($C_1$-$C_6$) alkyl group, a ($C_1$-$C_6$)alkoxy group or a ($C_1$-$C_6$)alkylthio group; or else $\mathbf{R^2}$ and $\mathbf{R^3}$ form, together with the carbon atoms which bear them, an optionally substituted benzo rings

$R^4$, $\mathbf{R^5}$ and $\mathbf{R^6}$, which may be identical or different, represent:

- • a hydrogen atom;
- • a $C_1$-$C_6$ alkyl radical optionally substituted with one or more radicals chosen from the radicals hydroxyl, $C_1$-$C_2$ alkoxy, $C_2$-$C_4$ (poly)hydroxyalkoxy, acylamino and amino substituted with two $C_1$-$C_4$ alkyl radicals, which may be identical or different;
- • a halogen atom such as chlorine, fluorine or bromine;
- • a hydroxyl group;
- • a $C_1$-$C_2$ alkoxy radical;
- • a $C_1$-$C_2$ alkylthio radical;
- • an amino radicals;
- • a 5- or 6-membered heterocycloalkyl radical which may be substituted with 1 to 3 groups, which may be identical or different, chosen from hydroxyl, amino, (di)alkylamino and $C_1$-$C_4$ hydroxyalkyl;
- • an amino radical substituted with one or two $C_1$-$C_6$ alkyl radicals, which may be identical or different, optionally bearing at least:

  i) a hydroxyl group,

  ii) an amino group optionally substituted with one or two $C_1$-$C_3$ alkyl radicals, it being possible for said alkyl radicals to form, with the nitrogen atom to which they are attached, a saturated or unsaturated, optionally substituted heterocycle comprising from 5 to 7 members, optionally comprising at least one other heteroatom which may or may not be different from nitrogen,

- • -N(R)-C(O)-R' in which the R radical is a hydrogen atom or a $C_1$-$C_4$ alkyl radical optionally bearing at least one hydroxy group, and the R' radical is a $C_1$-$C_2$ alkyl radical;
- • a carboxylic radical in acid or salified form;
- • a polyhaloalkyl group comprising from 1 to 6 carbon atoms and from 1 to 6 halogen atoms, which may be identical or different;

➢ or else two contiguous radicals $R^4$ with $R^5$ and/or $R^5$ with $R^6$ form, together with the carbon atoms which bear them, a benzo ring or a heterocycle, optionally substituted, fused to the phenyl group;

➢ or else $R^4$, $R^5$ and $R^6$ form, together with the phenyl group of formula (II), a tricyclic unit of julolidine type:

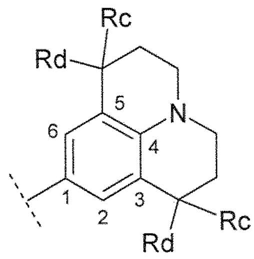

with Rc and Rd representing a hydrogen atom or a $C_1$-$C_4$ alkyl group;

➢ **L** represents a $C_1$-$C_{20}$ divalent hydrocarbon-based chain, which is optionally substituted, optionally interrupted and/or optionally terminated at one or the other of its ends i) with one or more divalent groups or combinations thereof chosen from: -N(R)-; $-N^+(R)(R^o)-$, $An^-$, -O-, -S-, -CO- and $-SO_2-$ with R and $R^o$, with may be identical or different, chosen from a hydrogen, and a $C_1$-$C_4$ alkyl, hydroxyalkyl or aminoalkyl radical, and $An^-$ representing an anionic counterion, or ii) with a cationic heterocycle or cationic heteroaryl $Het^+$, $An^-$, with $An^-$ as defined above and $Het^+$ representing a saturated or unsaturated heterocycle comprising from 5 to 10 members, or a heteroaryl comprising from 5 to 10 members;

➢ **X** represents an oxygen or sulfur atom or an NR group with R representing a hydrogen atom or a ($C_1$-$C_6$) alkyl group;

➢ **Y** represents: i) a hydrogen atom; ii) an alkali metal; iii) an alkaline earth metal; iv) an ammonium group: $N^+R^{\alpha}R^{\beta}R^{\gamma}R^{\delta}$ , $An''^-$ or a phosphonium group: $P^+R^{\alpha}R^{\beta}R^{\gamma}R^{\delta}$ , $An''^-$ with $R^{\alpha}$, $R^{\beta}$, $R^{\gamma}$ and $R^{\delta}$, which may be identical or different, representing a hydrogen atom or a ($C_1$-$C_4$)alkyl group and $An''^-$ an anionic counterion; or v) a thiol-function-protecting group;

➢ **W** represents an oxygen or sulfur atom or an NR' group with **R'** representing a hydrogen atom or a ($C_1$-$C_4$)alkyl group;

➢ **$Q^-$** represents an anionic counterion;

it being understood that, when n is 2, then m is zero, and when n is 1, then m is 1.

2. Fluorescent dye of formula **(I)** or **(II)** according to the preceding claim, in which m and n are 1 and Y represents a hydrogen atom or an alkali metal.

3. Fluorescent dye of formula **(I)** or **(II)** according to Claim 1, in which Y represents a protecting group.

4. Fluorescent dye of formula **(I)** or **(II)** according to the preceding Claim, in which Y represents a protecting group chosen from the following radicals:

• ($C_1$-$C_4$) alkylcarbonyl;
• ($C_1$-$C_4$) alkylthiocarbonyl;
• ($C_1$-$C_4$) alkoxycarbonyl;
• ($C_1$-$C_4$) alkoxythiocarbonyl;
• ($C_1$-$C_4$) alkylthiothiocarbonyl;
• (di) ($C_1$-$C_4$) (alkyl) aminocarbonyl;
• (di) ($C_1$-$C_4$) (alkyl) aminothiocarbonyl;
• arylcarbonyl;
• aryloxycarbonyl;
• aryl ($C_1$-$C_4$) alkoxycarbonyl;
• (di) ($C_1$-$C_4$) (alkyl) aminocarbonyl;
• ($C_1$-$C_4$) (alkyl) arylaminocarbonyl;
• carboxyl;
• $SO_3^-$; $M^+$ with $M^+$ representing an alkali metal or else $An^-$ or $An'^-$ of formula (I) or (II) and $M^+$ are absent;
• optionally substituted aryl;
• optionally substituted heteroaryl;
• optionally cationic, optionally substituted heterocycloalkyl,
• the following group:

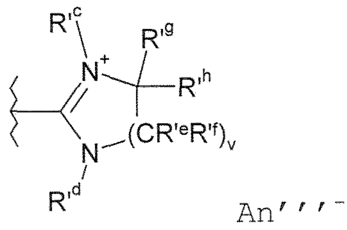

in which R'$^c$, R'$^d$, R'$^e$, R'$^f$, R'$^g$ and R'$^h$, which may be identical or different, represent a hydrogen atom or a ($C_1$-$C_4$) alkyl group, or else two groups R'$^g$ with R'$^h$, and/or R'$^e$ with R'$^f$ form an oxo or thioxo group, or else R'$^g$ with R'$^e$ together form a cycloalkyl; and v represents an integer between 1 and 3 inclusive; preferably R'$^c$ to R'$^h$ represent a hydrogen atom; and An'''$^-$ represents an anionic counterion;

■ isothiouronium;

■ -C(NR'$^c$R'$^d$)=N$^+$R'$^e$R'$^f$; An'''$^-$ with R'$^c$, R'$^d$ R'$^c$ and R'$^f$, which may be identical or different, representing a hydrogen atom or a ($C_1$-$C_4$)alkyl group and An'''$^-$ being as defined above;

■ isothiourea;

■ -C(NR'$^c$=R'$^d$)=NR'$^e$; An'$^-$ with R'$^c$, R'$^d$ and R'$^e$ as defined above;

■ optionally substituted (di) aryl ($C_1$-$C_4$) alkyl;

■ optionally substituted (di) heteroaryl ($C_1$-$C_4$) alkyl;

■ -CR$^1$R$^2$R$^3$ with R$^1$, R$^2$ and R$^3$, which may be identical or different, representing a halogen atom or a group chosen from:

- ($C_1$-$C_4$)alkyl;
- ($C_1$-$C_4$) alkoxy;
- optionally substituted aryl;
- optionally substituted heteroaryl;
- P(Z$^1$)R'$^1$R'$^2$R'$^3$ with R'$^1$ and R'$^2$, which may be identical or different, representing a hydroxyl, ($C_1$-$C_4$) alkoxy or alkyl group, R'$^3$ representing a hydroxyl or ($C_1$-$C_4$)alkoxy group, and Z$^1$ representing an oxygen or sulfur atom;

■ a sterically hindered cyclic group; and

■ optionally substituted alkoxyalkyl.

**5.** Fluorescent dye of formula **(I)** or **(II)** according to any one of the preceding claims, in which Y represents an alkali metal or a protecting group chosen from:

➢ ($C_1$-$C_4$) alkylcarbonyl;

➢ arylcarbanyl;

➢ ($C_1$-$C_4$) alkoxycarbonyl;

➢ aryloxycarbonyl;

➢ aryl ($C_1$-$C_4$) alkoxycarbonyl;

➢ (die) ($C_1$-$C_4$) (alkyl) aminocarbonyl;

➢ ($C_1$-$C_4$) (alkyl) arylaminocarbonyl;

➢ optionally substituted aryl;

➢ 5- or 6-membered cationic monocyclic heteroaryl optionally substituted with one or more ($C_1$-$C_4$)alkyl groups which may be identical or different;

➢ 8- to 11-membered cationic bicyclic heteroaryl optionally substituted with one or more ($C_1$-$C_4$)alkyl groups which may be identical or different;

➢ cationic heterocycle of the following formula:

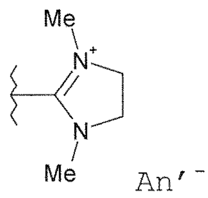

- ➢ isothiouronium;
- ➢ -C $(NH_2)$ =$N^+H_2$; An'''; with An'''- representing an anionic counterio,
- ➢ isothiourea;
- ➢ -C $(NH_2)$ =NH; and
- ➢ $SO_3^-$; $M^+$ with $M^+$ representing an alkali metal or else $An^-$ or An'- of formula (I) or (II) and $M^+$ are absent.

**6.** Disulfide fluorescent dye of formula (I) or (II) according to any one of the preceding claims, in which n=2 and m=0.

**7.** Fluorescent dye according to any one of the preceding claims, of formula **($I_a$)**, **($II_a$)**, **($I_b$)** and **($II_b$) :**

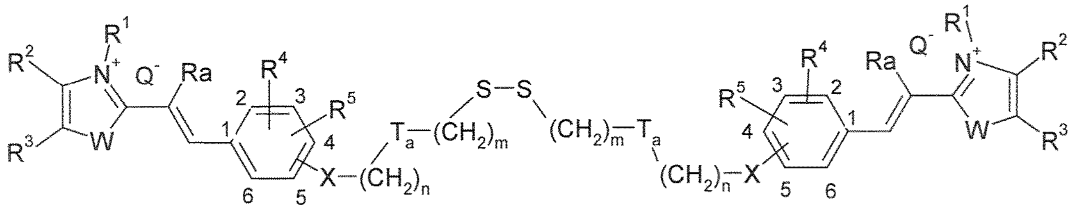

**($I_a$)**

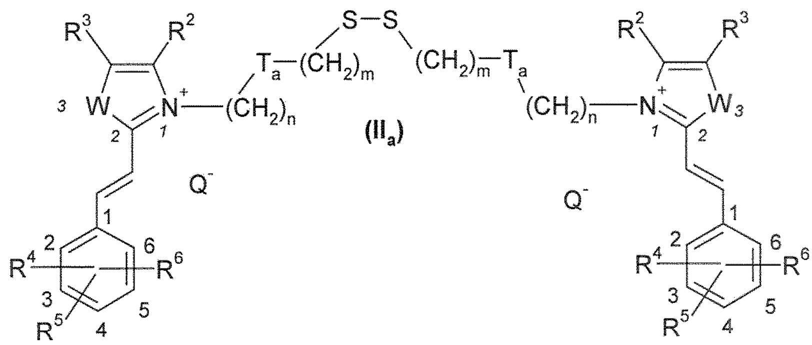

**($II_a$)**

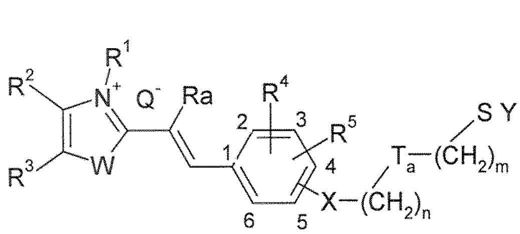

**($I_b$)**

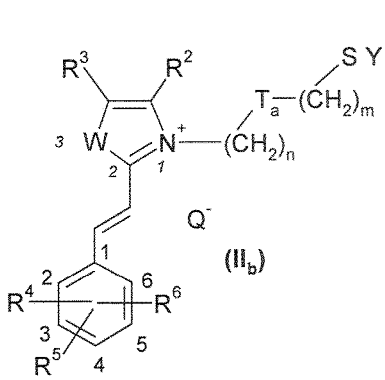

**($II_b$)**

in which formulae **($I_a$)** , **($II_a$)**, **($I_b$)** and **($II_b$) :**

> **Ra, R¹** to **R⁶, B, Y** and **Q⁻** are as in claims 1 to 5;
> **n** and **m,** which may be identical or different, represent an integer between 1 and 6 inclusive, with the sum of n+m being between 2 and 4 inclusive;
> **X** represents a radical chosen from: -G-, -G'-C(G)- and -C(G)-G', with G and G', which may be identical or different, representing an oxygen or sulfur atom, or NR, with R representing a hydrogen atom or a $(C_1-C_6)$alkyl group;
> $\mathbf{T_a}$ represents a σ covalent bond, a group $-N(R'_a)-$; $-N^+(R'_a)(R'_b)-$, $An^-$; $-C(O)-N(R'_a)-$ or $-N(R'_a)-C(O)-$, or a divalent cationic heteroaryl comprising from 5 to 7 members, with $R'_a$, $R'_b$, which may be identical or different, representing a hydrogen atom or a $(C_1-C_4)$alkyl radical, $An^-$ representing an anionic counterion; in particular, $T_a$ represents $-C(O)-N(R'_a)-$ and $-N(R'_a)-C(O)-$.

with $An^-$, which may be identical or different, representing an anionic counterion.

**8.** Fluorescent dye of formula (I) or (II) according to any one of the preceding claims, chosen from the following dyes:

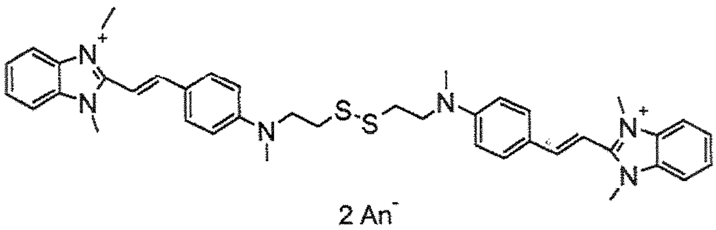

2 $An^-$

**1**

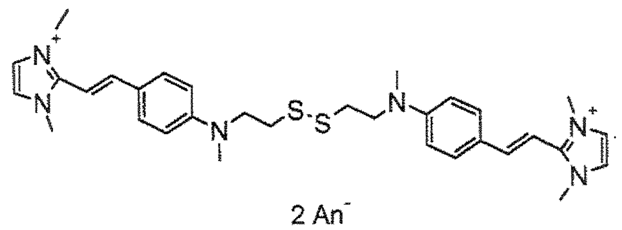

2 $An^-$

**2**

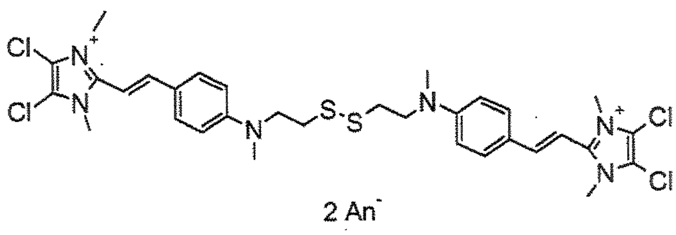

2 $An^-$

**3**

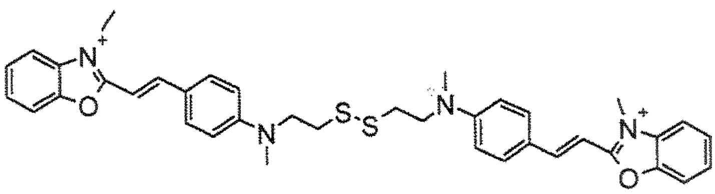

2 $An^-$

**4**

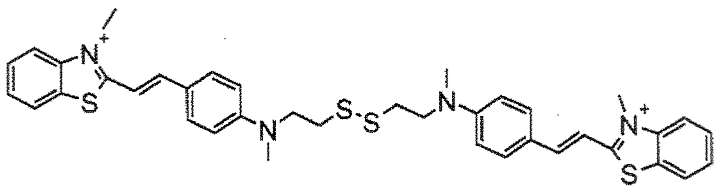

2 An$^-$

**5**

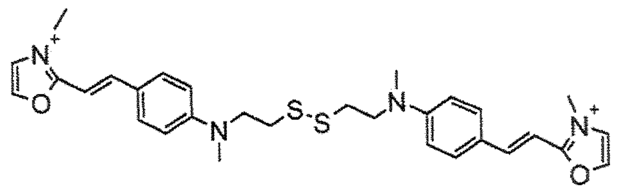

2 An$^-$

**6**

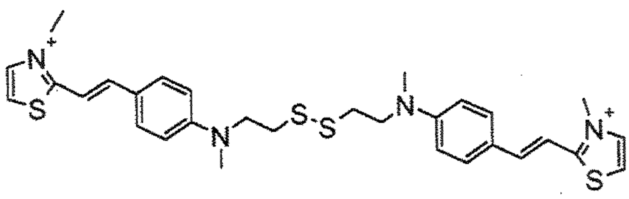

2 An$^-$

**7**

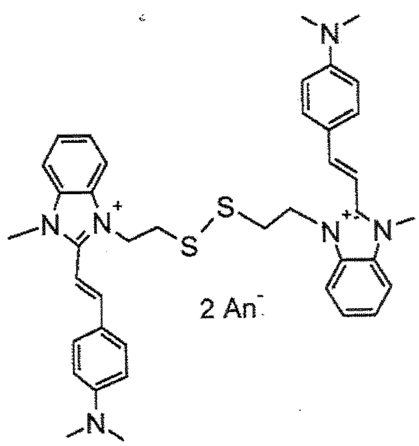

2 An$^-$

**8**

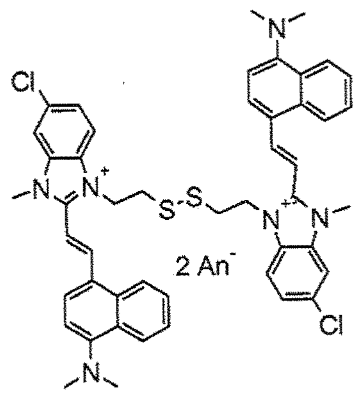

9

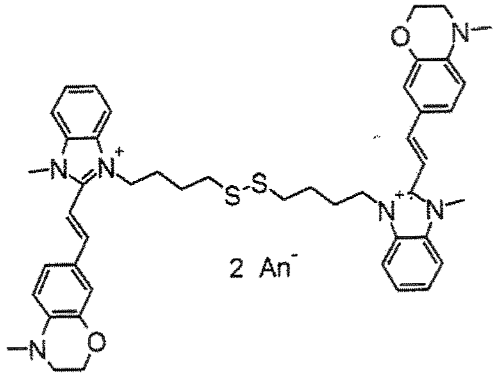

10

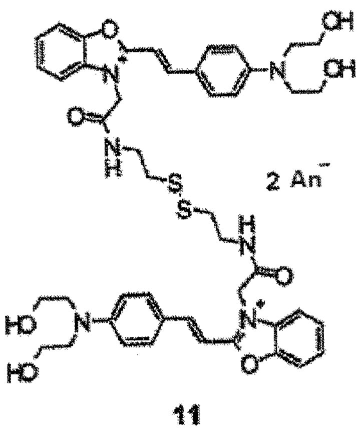

11

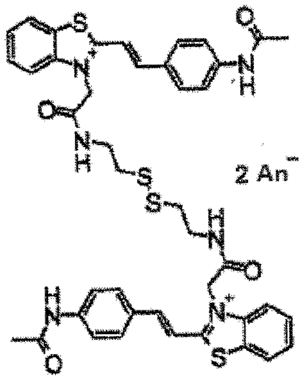

12

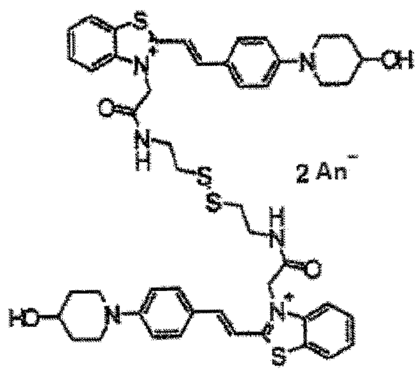

13

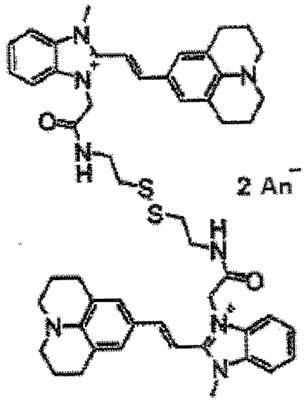

14

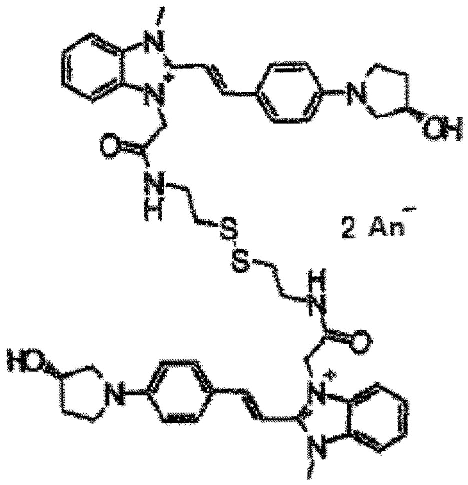

2 $An^-$

**15**

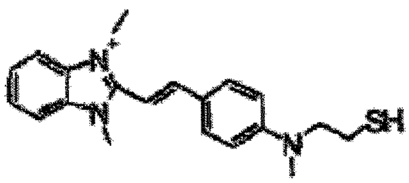

An-

**16**

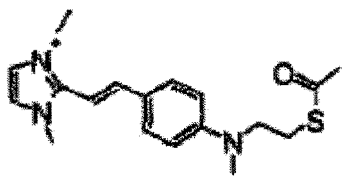

$An^-$

**17**

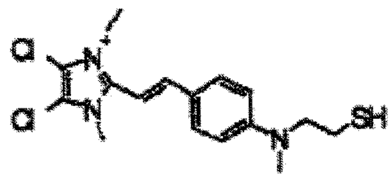

$An^-$

**18**

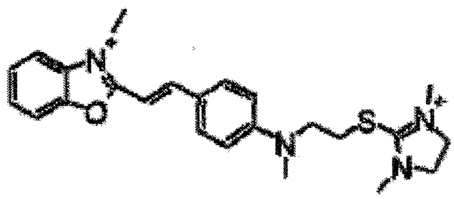

An⁻

**19**

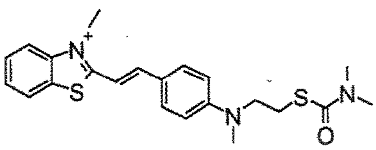

2 An⁻

**20**

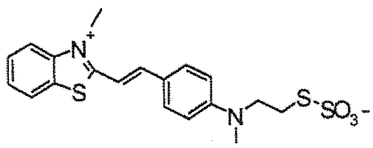

**21**

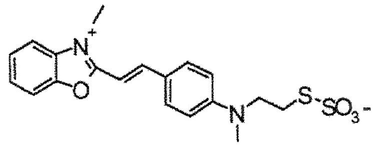

**22**

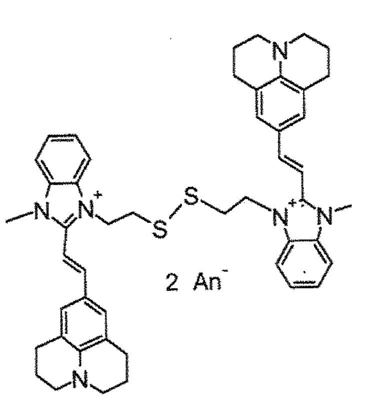

2 An⁻

**23**

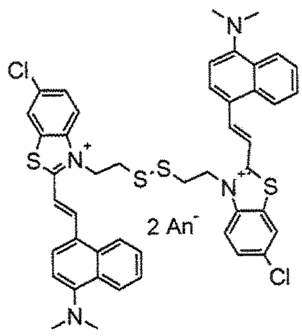

24

68

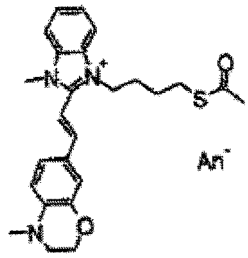

25

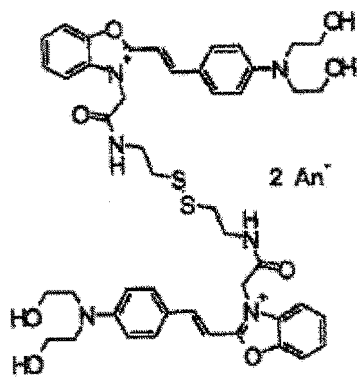

26

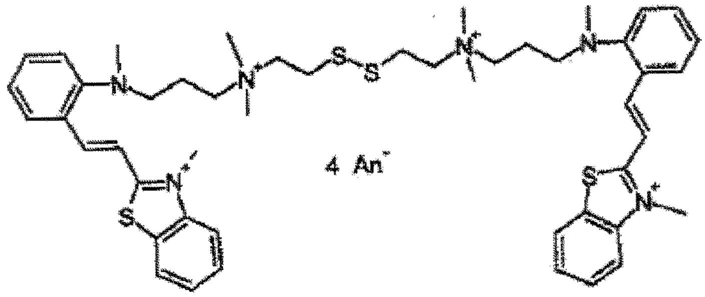

27

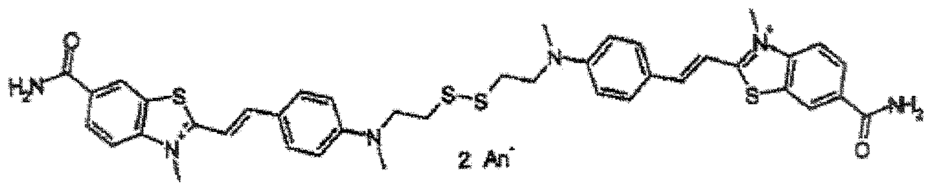

28

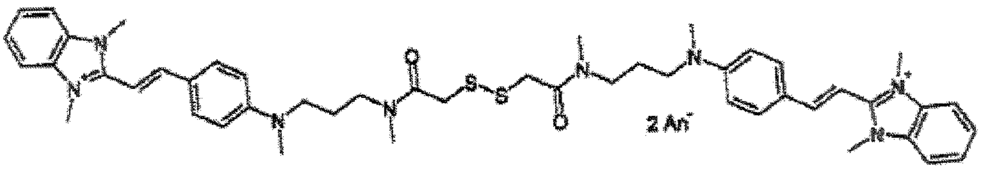

29

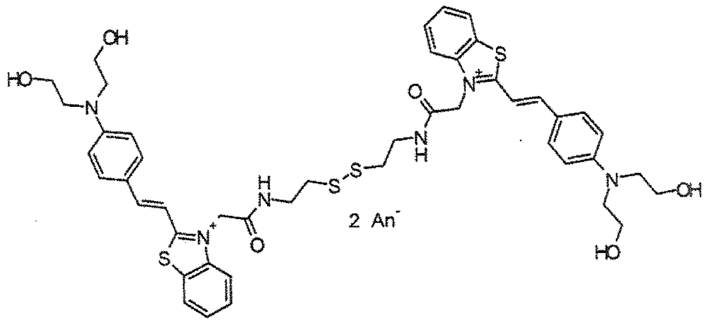

30

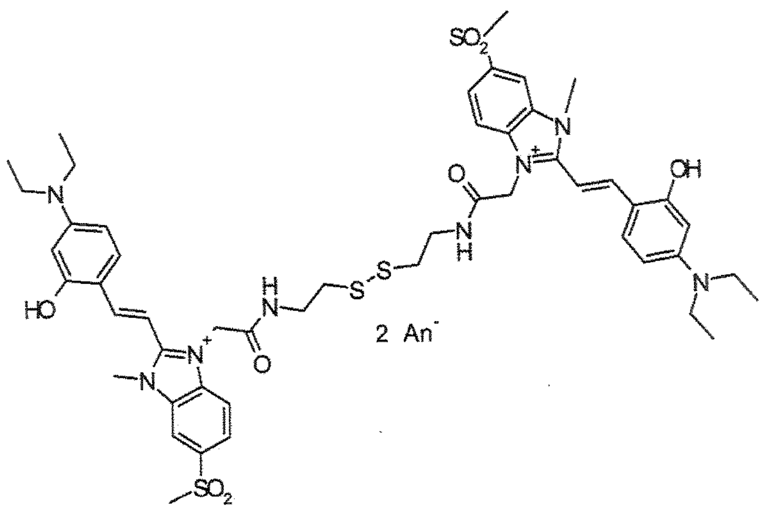

31

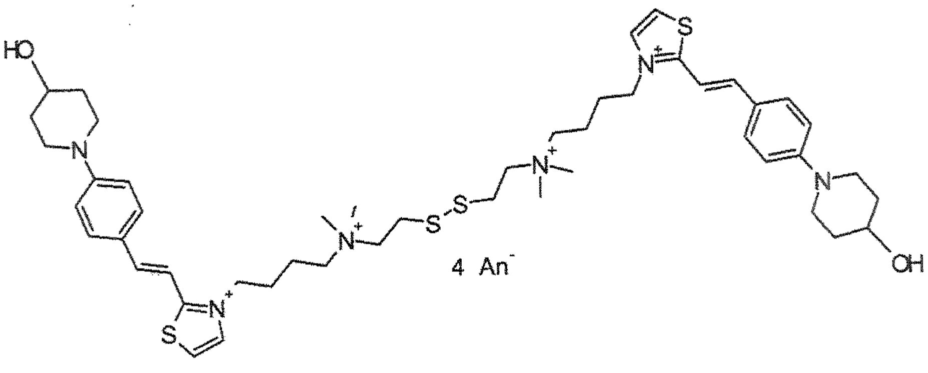

32

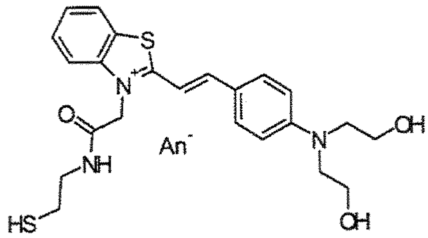

33

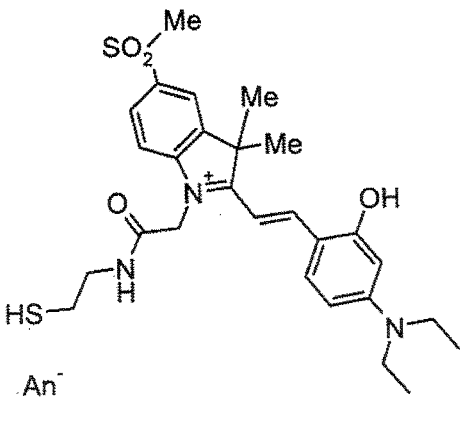

34

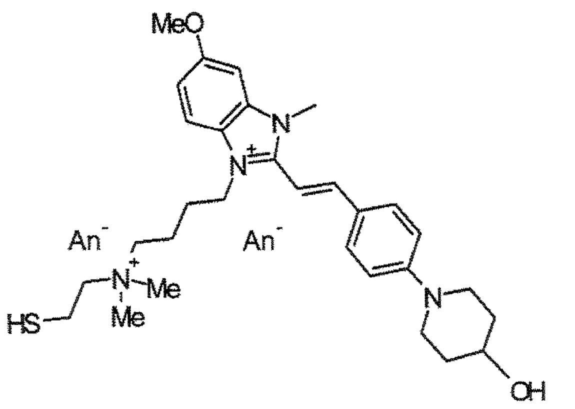

**35**

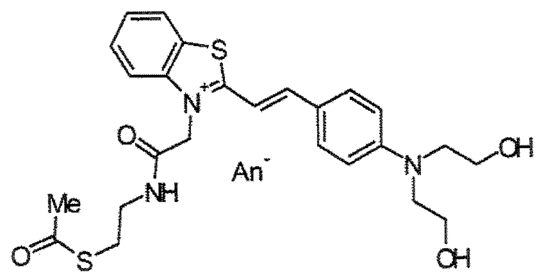

**36**

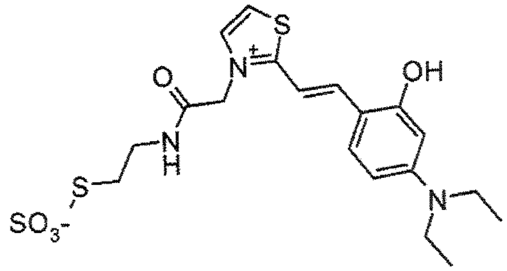

**37**

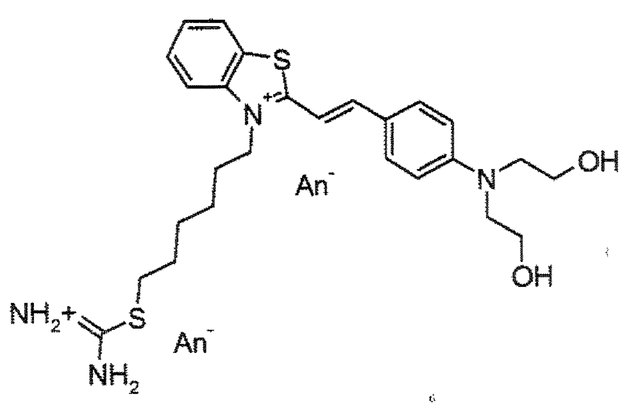

**38**

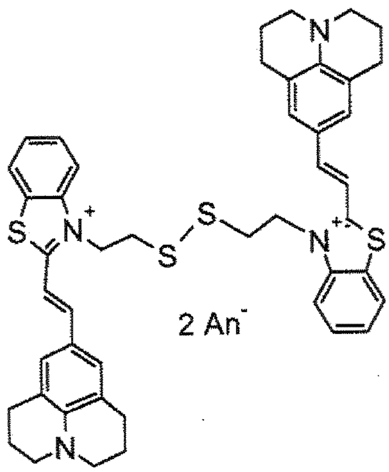

2 $An^-$

**39**

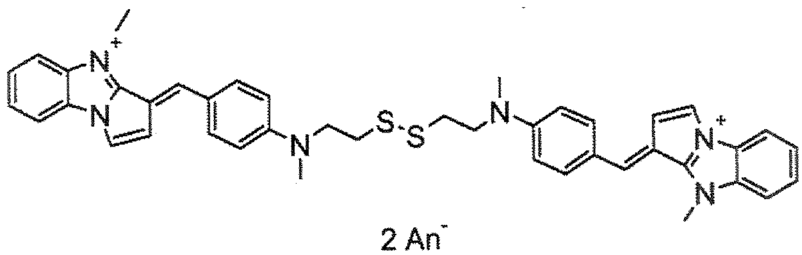

2 $An^-$

**40**

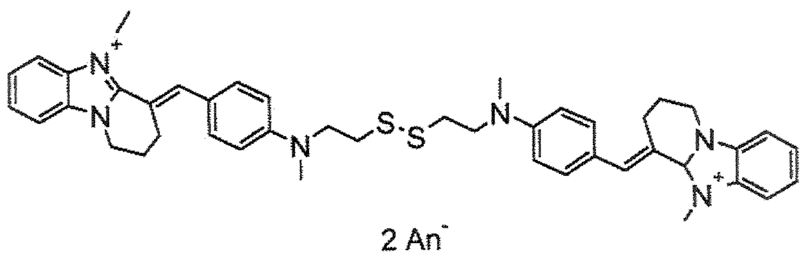

2 $An^-$

**41**

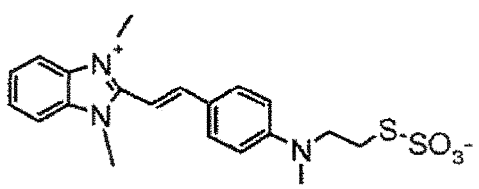

**42**

with $An^-$, which may be identical or different, representing an anionic counterion.

**9.** Dye composition comprising, in a suitable cosmetic medium, a fluorescent dye of formula **(I)** or **(II)** as defined in any one of claims 1 to 8.

**10.** Process for dyeing keratin materials, in which a suitable dye composition according to Claim 9 comprising at least one fluorescent dye of formula **(I)** or **(II)** is applied to the materials.

**11.** Process for dyeing keratin materials according to Claim 10, **characterized in that** the keratin materials are dark keratin fibers having a tone height of less than or equal to 6.

**12.** Process according to either one of Claims 10 and 11, comprising an additional step consisting in applying an oxidizing agent to the keratin fibers.

**13.** Multicompartment device in which a first compartment contains a dye composition comprising a fluorescent dye of formula **(I)** or **(II)** as defined in Claims 1 to 8, and a second compartment contains a reducing agent.

**14.** Use of the fluorescent dyes of formula **(I)** or **(II)** as defined in Claims 1 to 8, for dyeing dark human keratin fibers; which particularly have a tone height of less than 6.

**15.** Use according to the preceding claim, for lightening dark keratin fibers have a tone height of less than 6.

**Patentansprüche**

**1.** Fluoreszenzfarbstoff der Formel (I) oder (II):

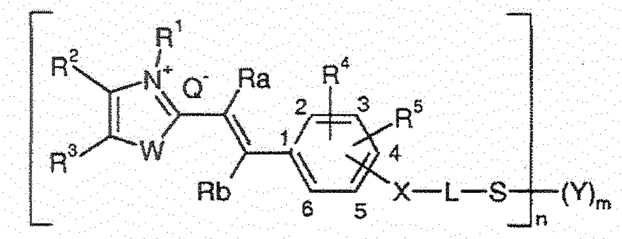

(I)

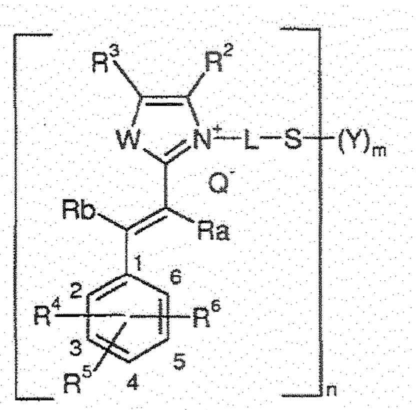

(II)

Salze davon mit einer organischen oder anorganischen Säure, optische Isomere davon, geometrische Isomere davon und Solvate wie Hydrate;
wobei in den Formeln **(I)** oder **(II):**

- ➢ **n** für 1 oder 2 steht;
- ➢ **m** für 0 oder 1 steht;
- ➢ **Ra** und **Rb** gleich oder verschieden sind und für ein Wasserstoffatom oder eine $C_1$-$C_4$-Alkylgruppe stehen;
- ➢ **$R^1$** für ein Wasserstoffatom oder eine ($C_1$-$C_6$)-Alkylgruppe, die gegebenenfalls durch eine unter Halogen, $C_1$-$C_4$-Alkoxy, $C_1$-$C_4$-(Di)(alkyl)-amino, Phenyl, Tolyl und Methoxyphenyl ausgewählte Gruppe substituiert ist, steht;
- ➢ oder auch **$R^1$** und **Ra** zusammen eine ($C_3$-$C_6$)-Alkylenkette oder eine ($C_3$-$C_7$) -Alkenylenkette bilden;
- ➢ **$R^2$** und **$R^3$** gleich oder verschieden sind und für ein Wasserstoff- oder Halogenatom oder eine gegebenenfalls subsituierte ($C_1$-$C_6$) -Alkylgruppe, eine ($C_1$-$C_6$)-Alkoxygruppe oder eine ($C_1$-$C_6$) -Alkylthiogruppe stehen oder auch **$R^2$** und **$R^3$** zusammen mit den Kohlenstoffatomen, an die sie gebunden sind, einen gegebenenfalls substituierten Benzoring bilden;
- ➢ **$R^4$, $R^5$** und **$R^6$** gleich oder verschieden sind und für:
  - • ein Wasserstoffatom;
  - • einen $C_1$-$C_6$-Alkylrest, der gegebenenfalls durch einen oder mehrere unter Hydroxy-, $C_1$-$C_2$-Alkoxy-,

$C_2$-$C_4$-(Poly)hydroxyalkoxy-, Acylamino-, durch zwei gleiche oder verschiedene $C_1$-$C_4$-Alkylreste substituieren Aminoresten ausgewählte Reste substituiert ist;
• ein Halogenatom;
• eine Hydroxygruppe;
• einen $C_1$-$C_2$-Alkoxyrest;
• einen $C_1$-$C_2$-Alkylthiorest;
• einen Aminorest;
• einen 5- oder 6-gliedrigen Heterocycloalkylrest, der durch 1 bis 3 gleiche oder verschiedene unter Hydroxy, Amino, (Di)alkyl-amino und $C_1$-$C_4$-Hydroxyalkyl ausgewählt Substitutenten substituiert sein kann;
• einen Aminorest, der durch einen oder zwei gleiche oder verschiedene $C_1$-$C_6$-Alkylreste, die gegebenenfalls mindestens:

i) eine Hydroxygruppe,
ii) eine Aminogruppe, die gegebenenfalls durch einen oder zwei $C_1$-$C_3$-Alkylreste substituiert ist, wobei die Alkylgruppen mit dem Stickstoffatom, an das sie gebunden sind, einen gegebenenfalls substituierten gesättigten oder ungesättigten, 5- bis 7-gliedrigen Heterocyclus, der gegebenenfalls ein weiteres, von Stickstoff verschiedenes oder damit identische Heteroatom enthalten kann, bilden können,
tragen, substituiert sein kann;

• -N (R) -C (O) -R' worin der Rest R für ein Wasserstoffatom oder einen $C_1$-$C_4$-Alkylrest, der gegebenenfalls mindestens eine Hydroxygruppe trägt, steht und der Rest R' für einen $C_1$-$C_2$-Alkylrest steht;
• einen Carboxylrest in Säureform oder versalzter Form;
• eine Polyhalogenalkylgruppe mit 1 bis 6 Kohlenstoffatomen und 1 bis 6 gleichen oder verschiedenen Halogenatomen

stehen;
➢ oder auch zwei benachbarte Reste $R^4$ mit $R^5$ und/oder $R^5$ mit $R^6$ zusammen mit den Kohlenstoffatomen, an die sie gebunden sind, einen Benzoring oder einen an die Phenylgruppe anellierten, gegebenenfalls substituierten Heterocyclus bilden;
➢ oder auch $R^4$, $R^5$ und $R^6$ zusammen mit der Phenylgruppe der Formel (II) eine tricyclische Einheit vom Julolidin-Typ bilden:

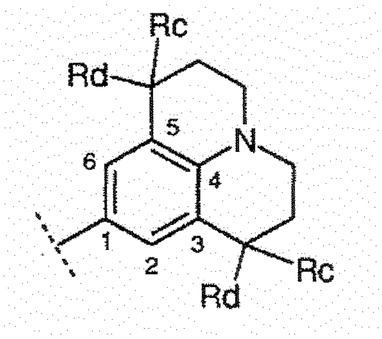

wobei Rc und Rd für ein Wasserstoffatom oder eine $C_1$-$C_4$-Alkylgruppe stehen;
➢ **L** für eine gegebenenfalls substituierte zweiwertige $C_1$-$C_{20}$-Kohlenwasserstoffkette steht, die gegebenenfalls i) durch eine oder mehrere zweiwertige Gruppen oder Kombinationen davon, die unter -N(R)-; $-N^+ (R) (R^o)$ -, $An^-$; -O-; -S-, -CO- und $-SO_2$- ausgewählt sind, wobei R und R° gleich oder verschieden sind und unter Wasserstoff und einem ($C_1$-$C_4$)-Alkyl-, Hydroxyalkyl- und Aminoalkylrest ausgewählt sind und $An^-$ für ein anionisches Gegenion steht, oder ii) durch einen kationischen Heterocyclus oder ein kationisches Heteroaryl $Het^+$, $An^-$, wobei $An^-$ für ein anionisches Gegenion steht und $Het^+$ für einen gesättigten oder ungesättigten 5- bis 10-gliedrigen Heterocyclus oder ein 5- bis 10-gliedriges Heteroaryl steht, unterbrochen und/oder gegebenenfalls an einem oder beiden seiner Enden durch diese Gruppen terminiert ist;
➢ **X** für ein Sauerstoff- oder Schwefelatom oder eine NR-Gruppe steht, wobei R für ein Wasserstoffatom oder eine ($C_1$-$C_6$)-Alkylgruppe steht;
➢ **Y** für i) ein Wasserstoffatom; ii) ein Alkalimetall; iii) ein Erdalkalimetall; iv) eine Ammoniumgruppe : $N^+R^\alpha R^\beta R^\gamma R^\delta$, An''- oder eine Phosponiumgruppe: $P^+R^\alpha R^\beta R^\gamma R^\delta$, An''-, wobei $R^\alpha$, $R^\beta$, $R^\gamma$ und $R^\delta$ gleich oder verschieden sind und für ein Wasserstoffatom oder eine ($C_1$-$C_4$) -Alkylgruppe stehen und An''- für ein anionisches

Gegenion steht; oder v) eine Schutzgruppe für eine Thiolfunktion steht;

➢ **W** für ein Sauerstoff- oder Schwefelatom oder eine NR'-Gruppe steht, wobei R' für ein Wasserstoffatom oder eine $(C_1\text{-}C_4)$-Alkylgruppe steht;

➢ $\mathbf{Q}^-$ für ein anionisches Gegenion steht;

mit der Maßgabe, dass dann, wenn n gleich 2 ist, m gleich null ist, und dann, wenn n gleich 1 ist, m gleich 1 ist.

**2.** Fluoreszenzfarbstoff der Formel **(I)** oder **(II)** nach dem vorhergehenden Anspruch, worin m und n gleich 1 sind und Y für ein Wasserstoffatom oder ein Alkalimetall steht.

**3.** Fluoreszenzfarbstoff der Formel **(I)** oder **(II)** nach Anspruch 1, worin m und n gleich 1 sind und Y für eine Schutzgruppe steht.

**4.** Fluoreszenzfarbstoff der Formel **(I)** oder **(II)** nach dem vorhergehenden Anspruch, worin Y für eine unter den folgenden Resten **ausgewählte** Schutzgruppe steht:

■ $(C_1\text{-}C_4)$ -Alkylcarbonyl;
■ $(C_1\text{-}C_4)$ -Alkylthiocarbonyl;
■ $(C_1\text{-}C_4)$ -Alkoxycarbonyl;
■ $(C_1\text{-}C_4)$ -Alkoxythiocarbonyl;
■ $(C_1\text{-}C_4)$ -Alkylthiothiocarbonyl;
■ (Di) - $(C_1\text{-}C_4)$ (alkyl) aminocarbonyl;
■ (Di) - $(C_1\text{-}C_4)$ - (alkyl) aminothiocarbonyl;
■ Arylcarbonyl;
■ Aryloxycarbonyl;
■ Aryl- $(C_1\text{-}C_4)$ -alkoxycarbonyl;
■ (Di) - $(C_1\text{-}C_4)$ - (alkyl) aminocarbonyl;
■ $(C_1\text{-}C_4)$ - (Alkyl) arylaminocarbonyl;
■ Carboxyl;
■ $SO_3^-$; $M^+$, wobei $M^+$ für ein Alkalimetall steht, oder auch $An^-$ oder $An'^-$ der Formel (I) oder (II) steht und $M^+$ fehlen;
■ gegebenenfalls substituiertem Aryl;
■ gegebenenfalls substituiertem Heteroaryl;
■ gegebenenfalls kationischem, gegebenenfalls substituiertem Heterocycloalkyl,
■ der folgenden Gruppe:

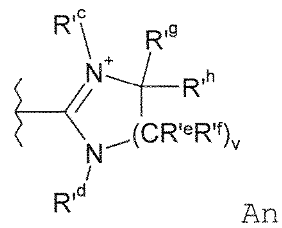

$An'''^-$

worin $R'^c$, $R'^d$, $R'^e$, $R'^f$, $R'^g$ und $R'^h$ gleich oder verschieden sein können und für ein Wasserstoffatom oder eine $(C_1\text{-}C_4)$ -Alkylgruppe stehen oder auch zwei Gruppen $R'^g$ mit $R'^h$ und/oder $R'^e$ mit $R'^f$ eine Oxo- oder Thioxogruppe bilden oder auch $R'^g$ mit $R'^e$ zusammen ein Cycloalkyl bilden und v für eine ganze Zahl zwischen 1 und 3 inklusive steht; $R'^c$ bis $R'^h$ vorzugsweise für ein Wasserstoffatom stehen und $An'''^-$ für ein Gegenion steht;
■ Isothiouronium;
■ $-C(NR'^c R'^d) =N^+-R'^eR'^f$; $An'''^-$, wobei $R'^c$, $R'^d$, $R'^e$ und $R'^f$ gleich oder verschieden sind und für ein Wasserstoffatom oder eine $(C_1\text{-}C_4)$ -Alkylgruppe stehen und $An'''^-$ die oben angegebene Bedeutung besitzt;
■ Isothioharnstoff;
■ $-C(NR'^cR'^d)=NR'^e$, wobei $R'^c$, $R'^d$ und $R'^e$ die oben angegebene Bedeutung besitzen;
■ gegebenenfalls substituiertem (Di) aryl-$(C_1\text{-}C_4)$-alkyl;
■ gegebenenfalls substituiertem (Di)heteroaryl-$(C_1\text{-}C_4)$ -alkyl;
■ $CR^1R^2R^3$, wobei, $R^1$, $R^2$ und $R^3$ gleiche oder verschieden sind und für ein Halogenatom oder eine unter:

- $(C_1-C_4)$-Alkyl;
- $(C_1-C_4)$ -Alkoxy;
- gegebenenfalls substituiertem Aryl;
- gegebenenfalls substituiertem Heteroaryl;
- P $(Z^1)$ $R'^1R'^2$ $R'^3$, wobei $R'^1$ und $R'^2$ gleich oder verschieden sind und für eine Hydroxy-, $(C_1-C_4)$-Alkoxy- or Alkylgruppe stehen, $R'^3$ für eine Hydroxy- oder $(C_1-C_4)$-Alkoxygruppe steht und $Z^1$ für ein Sauerstoff- oder Schwefelatom steht;

ausgewählte Gruppe stehen;

■ einer sterisch gehinderten acyclischen Gruppe und

■ gegebenenfalls substituiertem Alkoxyalkyl.

**5.** Fluoreszenzfarbstoff der Formel **(I)** oder **(II)** nach einem der vorhergehenden Ansprüche, worin Y für ein Alkalimetall oder eine unter:

➢ $(C_1-C_4$ -Alkylcarbonyl;
➢ Carbonyl;
➢ $(C_1-C_4)$ -Alkoxycarbonyl;
➢ Oxycarbonyl;
➢ Aryl- $(C_1-C_4)$ -alkoxycarbonyl;
➢ (Di) - $(C_1-C_4)$ - (alkyl) aminocarbonyl;
➢ $(C_1-C_4)$ - (Alkyl) arylaminocarbonyl;
➢ gegebenenfalls substituiertem Aryl;
➢ einem 5- oder 6-gliedrigem kationischen monocyclischen Heterocyclus, der gegebenenfalls durch eine oder mehrere gleiche oder verschiedene $(C_1-C_4)$ -Alkylgruppen substituiert ist;
➢ einem kationischen Heterocyclus der folgenden Formel:

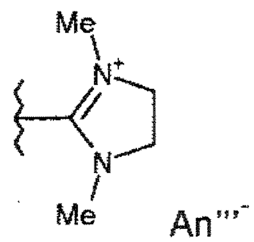

➢ Isothiouronium;
➢ -C $(NH_2)$ $=N^+H_2$; An'''-; wobei An'''- für ein anionisches Gegenion steht;
➢ Isothioharnstoff;
➢ $-C(NH_2)=NH$ und
➢ $SO_3$-; $M^+$, wobei $M^+$ für ein Alkalimetall steht, oder auch $An^-$ oder $An'^-$ der Formel (I) oder (II) steht und $M^+$ fehlen;

ausgewählte Schutzgruppe steht.

**6.** Fluoreszenzfarbstoff der Formel **(I)** oder **(II)** nach einem der vorhergehenden Ansprüche, worin n=2 und m=0.

**7.** Fluoreszenzfarbstoff nach einem der vorhergehenden Ansprüche der Formel **$(I_a)$, $(II_a)$, $(I_b)$** und **$(II_b)$:**

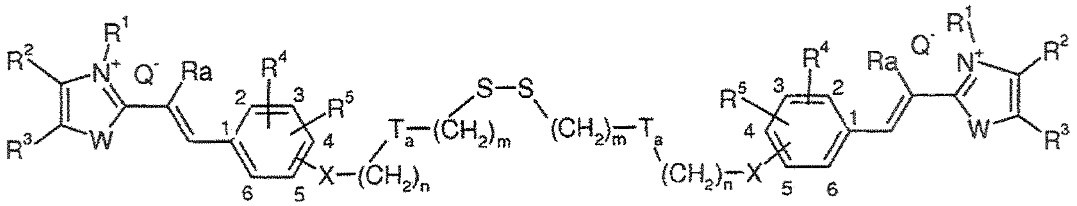

$(I_a)$

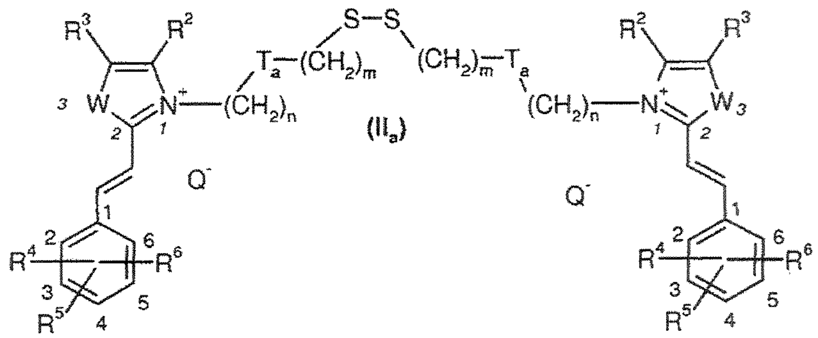

$(II_a)$

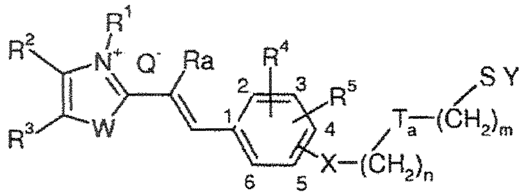

$(I_b)$

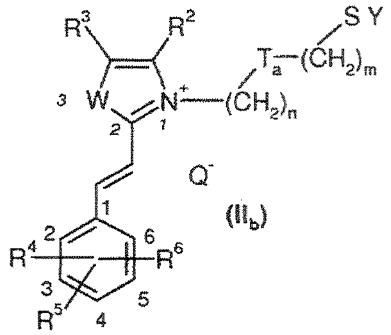

$(II_b)$

wobei in den Formen **$(I_a)$, (via), $(I_b)$** und **$(II_b)$:**

- ➢ **Ra, $R^1$** bis **$R^6$, B, Y** und **$Q^-$** die in den Ansprüchen 1 bis 5 angegebene Bedeutung besitzen;
- ➢ **n** und **m** gleich oder verschieden sind und für eine ganze Zahl zwischen 1 und 6 inklusive stehen, wobei die Summe von n+m zwischen 2 und 4 inklusive liegt;
- ➢ **X** für einen unter -G-, -G'-C(G)- und -C(G)-G'- ausgewähltes Rest steht, wobei G und G' gleich oder verschieden sind und für ein Sauerstoff- oder Schwefelatom oder NR stehen, wobei R für ein Wasserstoffatom oder eine $(C_1-C_6)$-Alkylgruppe steht;
- ➢ **$T_a$** für eine kovalente σ-Bindung, eine -N(R'$_a$)-; -N$^+$(R'$_a$) (R'$_b$)-, An$^-$ ; -C(O)-N(R'$_a$) - oder -N(R'$_a$)-C(O)-Gruppe oder ein 5- bis 7-gliedriges zweiwertiges kationisches Heteroaryl steht, wobei R'$_a$ und R'$_b$ gleich oder verschieden sind und für ein Wasserstoffatom oder einen $(C_1-C_4)$-Alkylrest stehen und An$^-$ für ein anionischer Gegenion steht; $T_a$ insbesondere für -C(O)-N(R'$_a$)- und -N(R'$_a$)-C(O) sieht.

**8.** Fluoreszenzfarbstoff der Formel **(I)** oder **(II)** nach einem der vorhergehenden Ansprüche, ausgewählte unter den folgenden Farbstoffen:

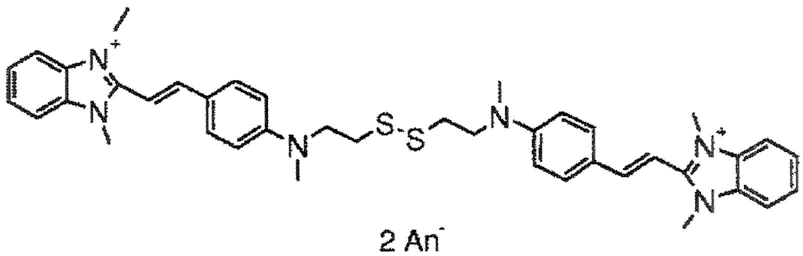

2 An$^-$

**1**

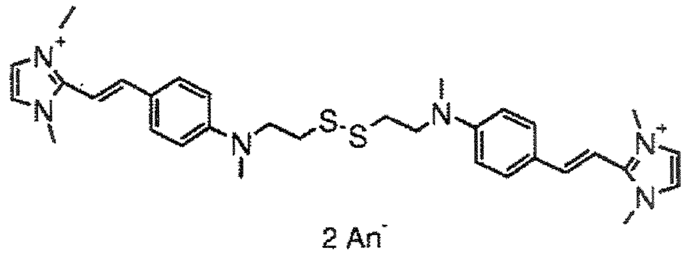

2 An$^-$

**2**

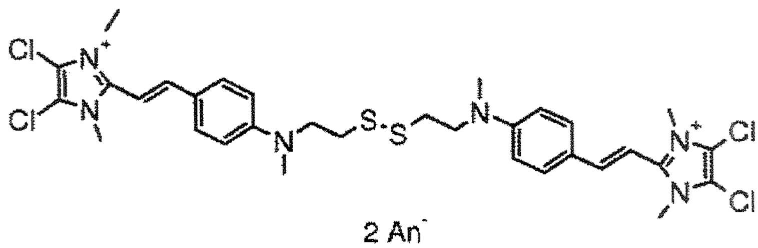

2 An$^-$

**3**

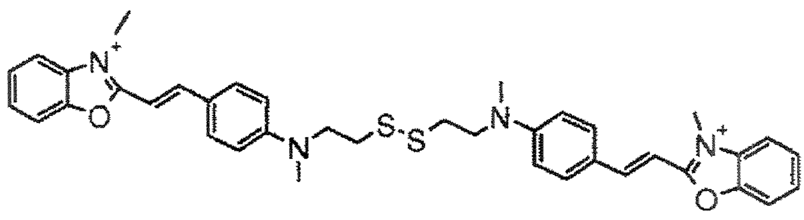

2 An$^-$

**4**

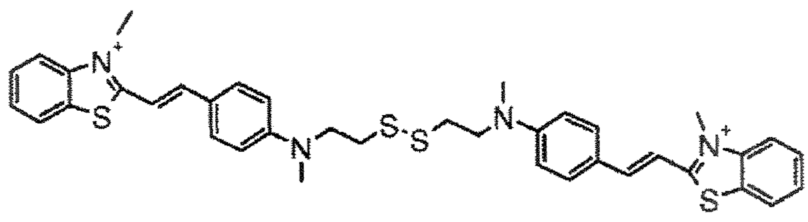

2 An$^-$

**5**

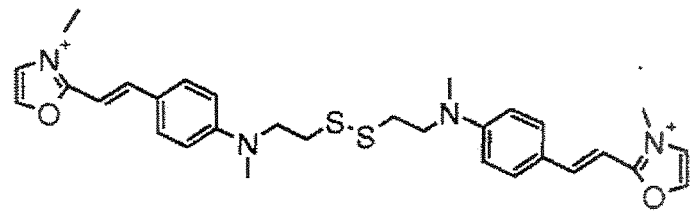

2 $An^-$

**6**

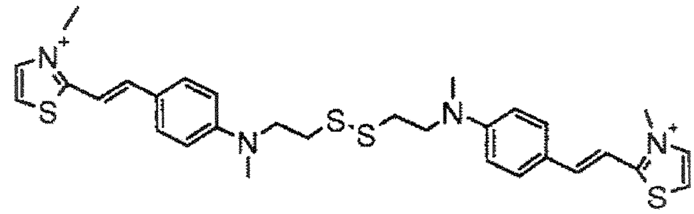

2 $An^-$

**7**

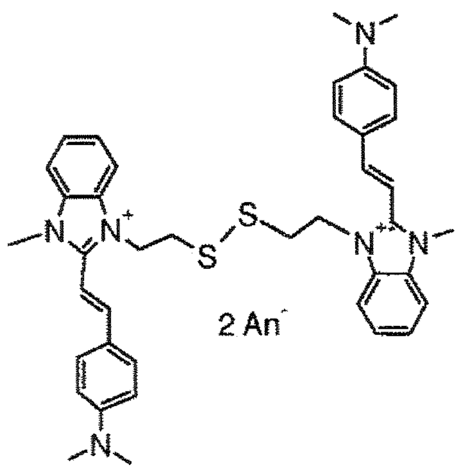

2 $An^-$

**8**

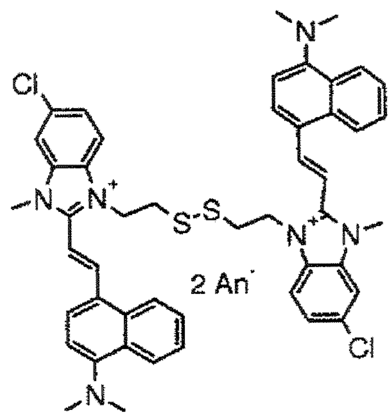

9

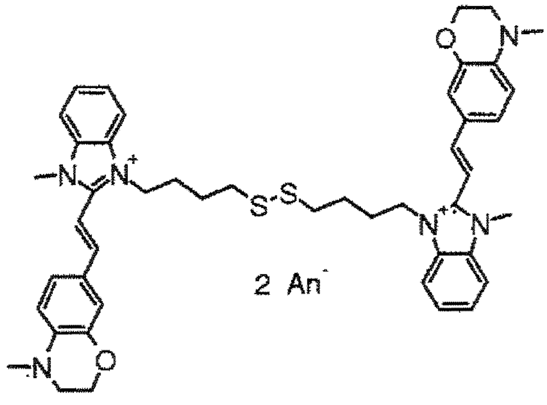

10

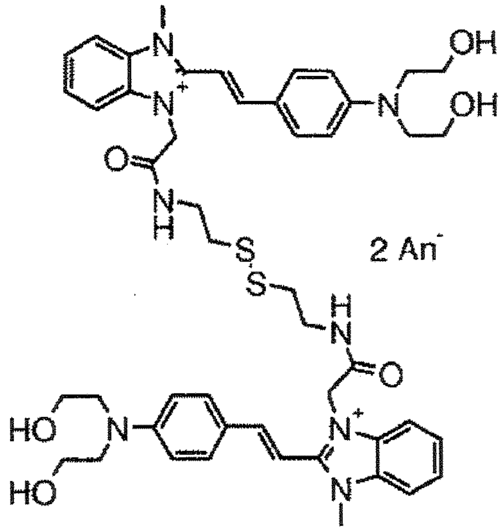

11

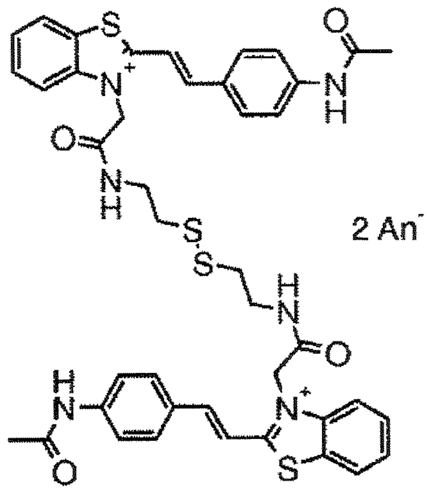

**12**

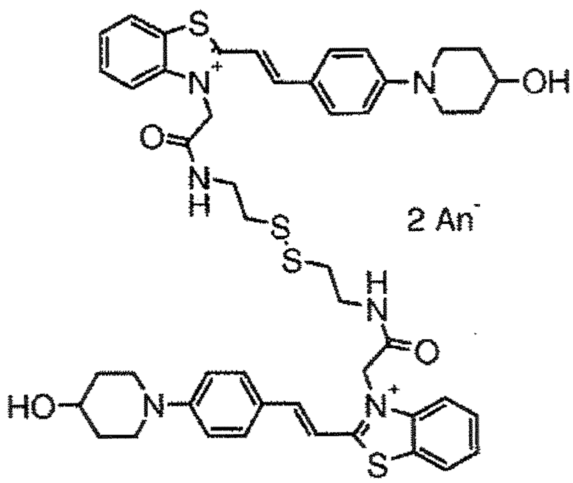

**13**

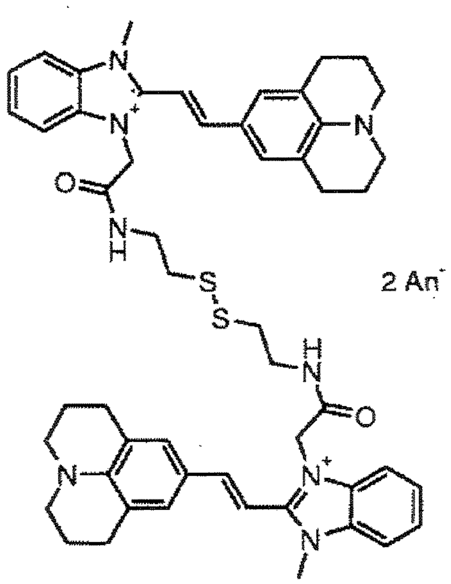

2 An-

**14**

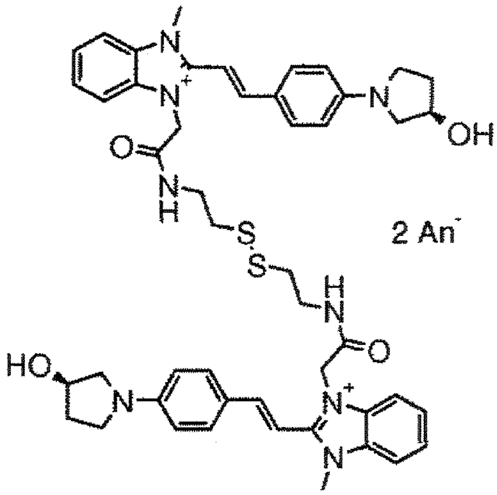

2 An-

**15**

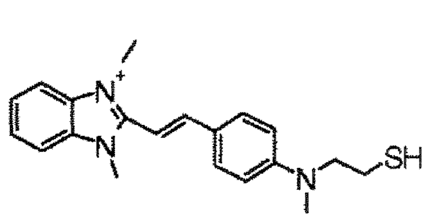

An-

**16**

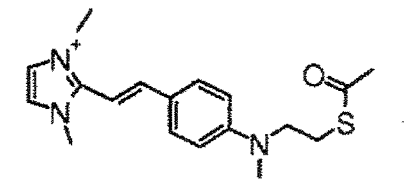

$An^-$

**17**

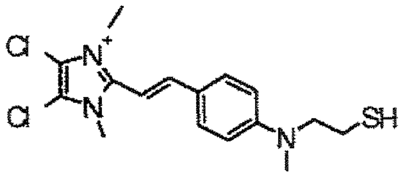

$An^-$

**18**

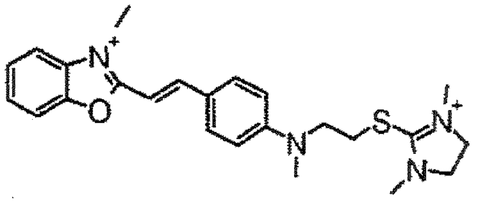

$An^-$

**19**

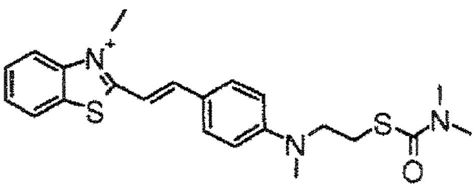

2 $An^-$

**20**

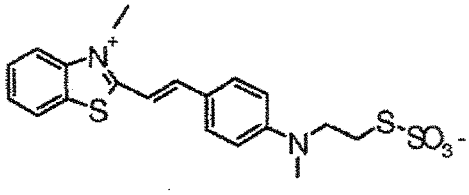

**21**

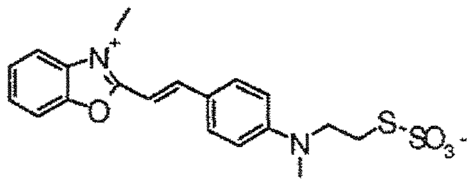

22

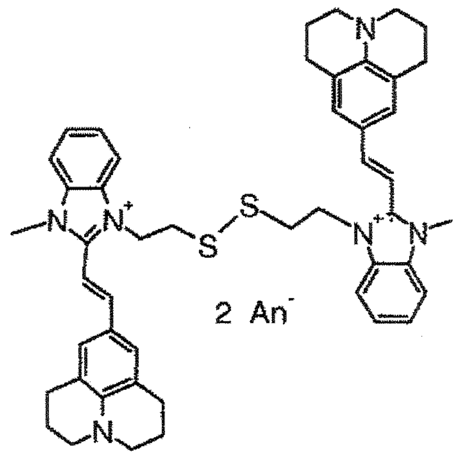

23

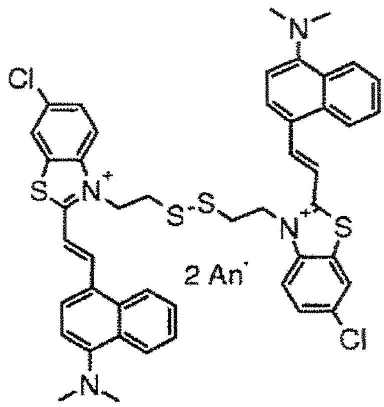

24

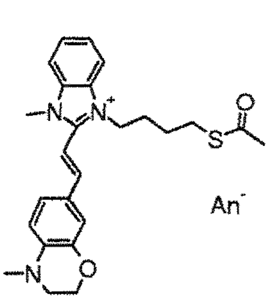

25

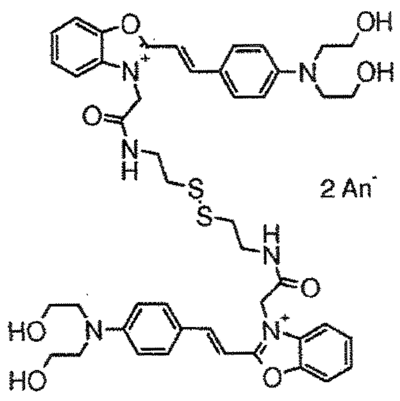

26

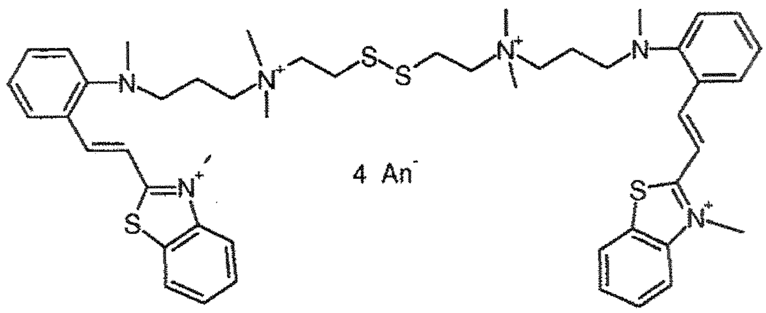

27

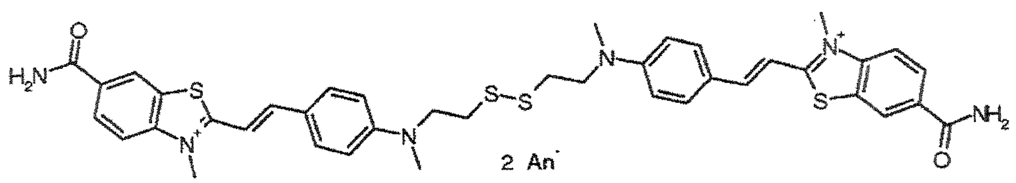

28

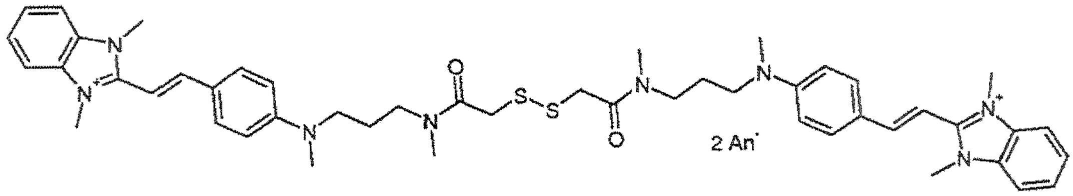

29

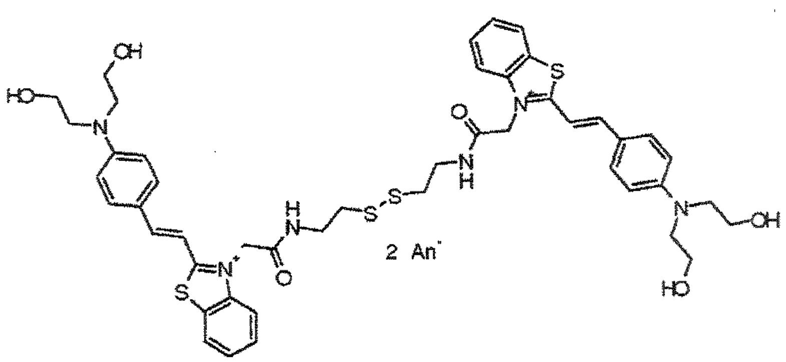

**30**

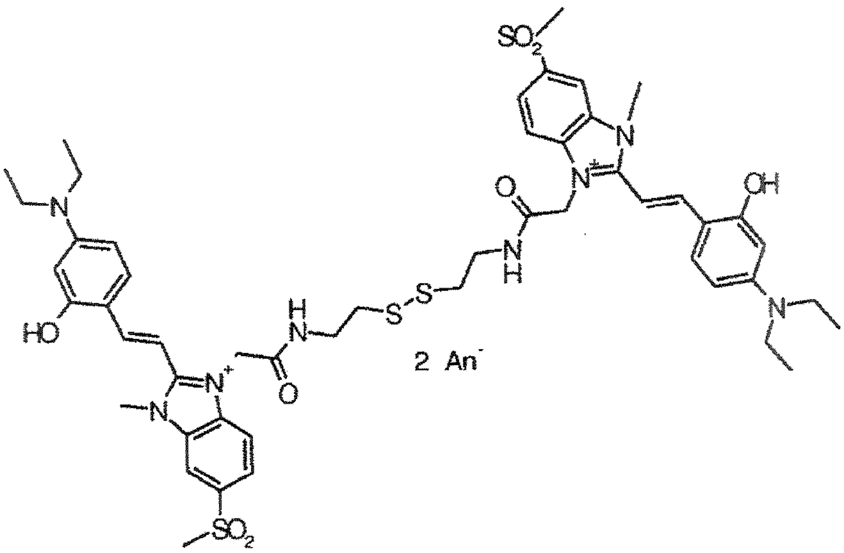

**31**

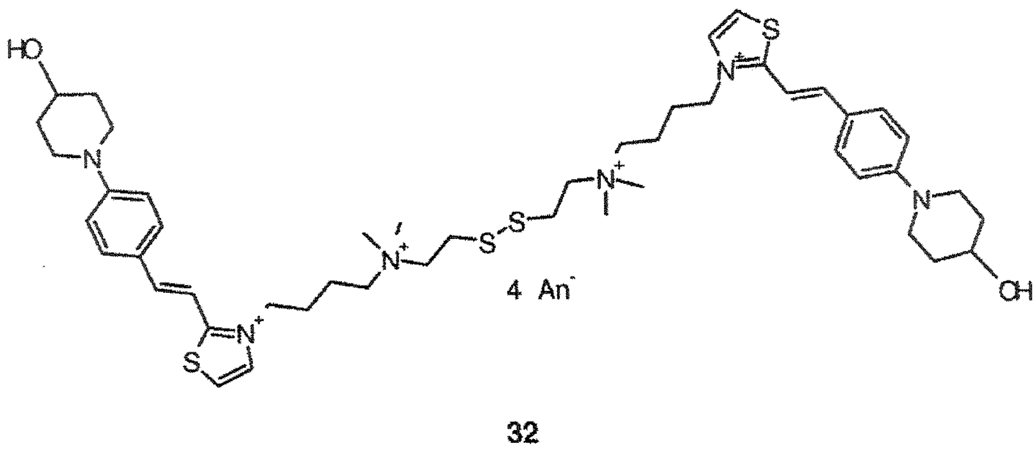

**32**

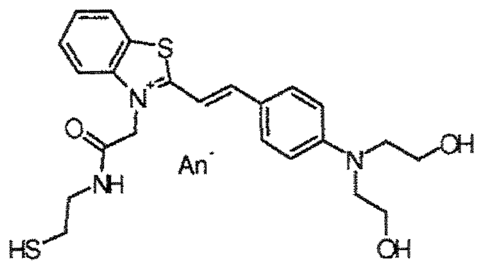

**33**

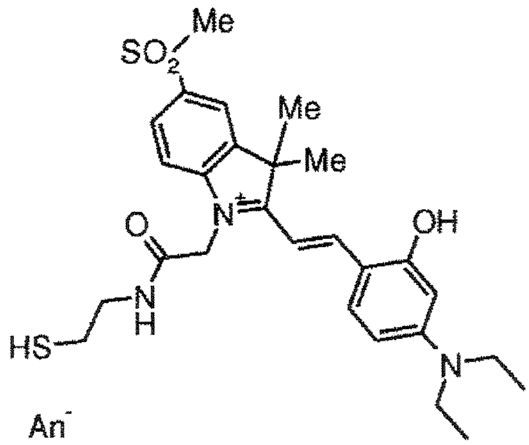

**34**

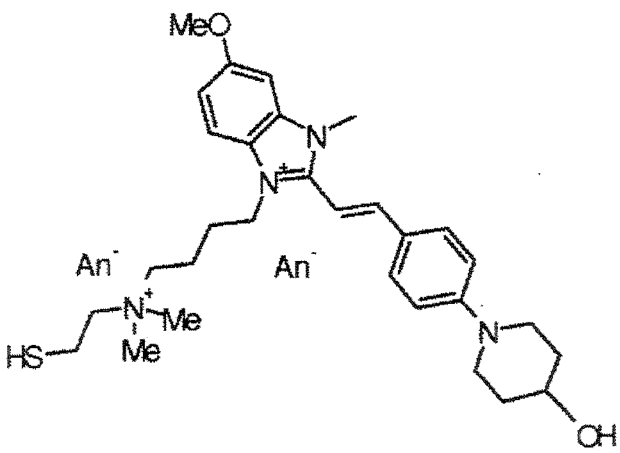

**35**

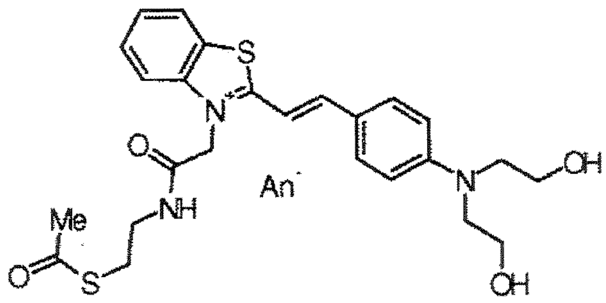

**36**

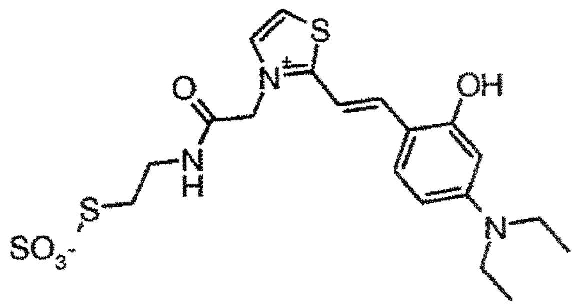

**37**

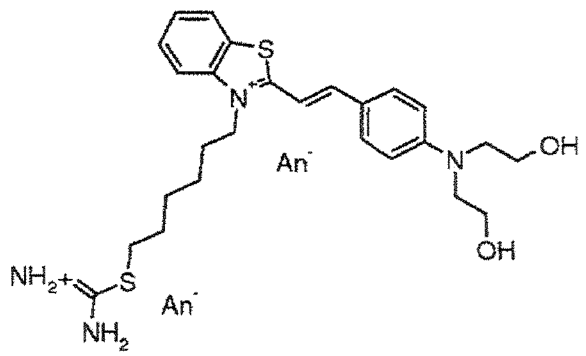

**38**

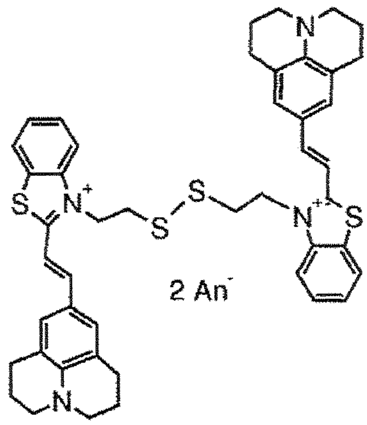

**39**

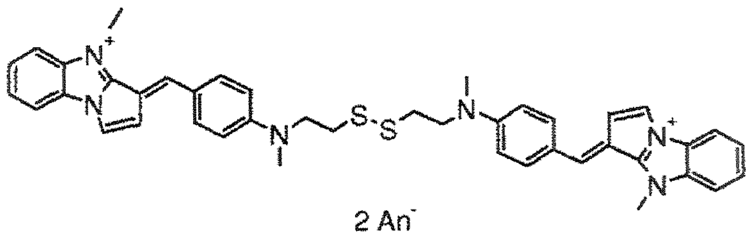

**40**

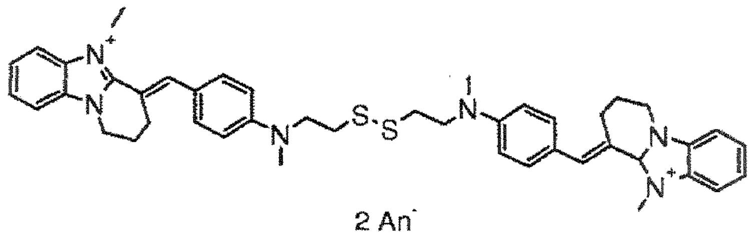

**41**

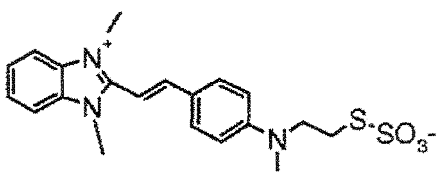

**42**

wobei $An^-$ gleich oder verschieden sein kann und für ein anionisches Gegenion steht.

**9.** Färbezusammensetzung, die in einem geeigneten kosmetischen Medium einen Fluoreszenzfarbstoff der Formel **(I)** oder **(II)** gemäß einem der Ansprüche 1 bis 8 umfasst.

**10.** Verfahren zum Färben von Keratznmaterialien, bei dem man auf die Materialien eine geeignete Färbezusammensetzung nach Anspruch 9, die mindestens einen Fluoreszenzfarbstoff der Formel **(I)** oder **(II)** umfasst, aufbringt.

**11.** Verfahren zum Färben von Keratinmaterialien nach Anspruch 10, **dadurch** gekenntzeichnet, dass es sich bei den Keratinmaterialien um dunkle Keratinfasern mit einer Tonhöhe kleiner gleich 6 handelt.

**12.** Verfahren nach einem der Ansprüche 10 oder 11 mit einem zusätzlichen Schritt, der darin besteht, dass man auf die Keratinfasern ein Oxidationsmittel aufbringt.

**13.** Vorrichtung mit mehreren Kompartimenten, wobei ein erstes Kompartiment eine Färbezusammensetzung, die einen Fluoreszenzfarbstoff der Formel **(I)** oder **(II)** gemäß einem der Ansprüche 1 bis 8 umfasst, enthält und ein zweites Kompartiment ein Reduktionsmittel enthält.

**14.** Verwendung der Fluoreszenzfarbstoffe der Formel **(I)** oder **(II)** gemäß einem der Ansprüche 1 bis 8 zum Färben von dunklen menschlichen Keratinfasern, insbesondere mit einer Tonhöhe kleiner 6.

**15.** Verwendung nach dem vorhergehenden Anspruch zur Aufhellung von dunklen Keratinfasern, insbesondere mit einer Tonhöhe kleiner 6.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 1166753 A
- EP 1166757 A
- WO 03028685 A
- WO 2004091473 A
- WO 2005097051 A
- EP 1647580 A
- WO 2006134043 A
- WO 2006136617 A
- WO 2007039527 A
- FR 2586913


### Littérature non-brevet citée dans la description


- **CHARLES ZVIAK.** Science des traitements capillaires. 1988, 215-278
- Protective Groups in Organic Synthesis. Protecting Groups. Thieme, 2005, 193-217
- Protecting Groups. Thieme, 2005
- « Thiols and organic Sulfides », « Thiocyanates and Isothiocyanates, organic ». Ullmann's Encyclopedia. Wiley-VCH, 2005
- Reactions, Mechanisms and Structures. **J. MARCH.** Advanced Organic Chemistry. John Willey & Sons, 1995
- **T. W. GREENE.** Protective Groups in Organic Synthesis
- Reactions, Mechanisms and Structures. **J. MARCH.** Advanced Organic Chemistry. John Willey & Sons, 1992
- Protective Groups in Organic Synthesis. Protecting Groups. Thieme, 2005
- Reactions, Mechanisms and Structures. Advanced Organic Chemistry. John Willey & Sons, 1992